(19) Europäisches Patentamt / European Patent Office / Office européen des brevets

(11) **EP 3 612 568 B1**

(12) **EUROPEAN PATENT SPECIFICATION**

(45) Date of publication and mention
of the grant of the patent:
**03.11.2021   Bulletin 2021/44**

(51) Int Cl.:
*C07K 16/30* (2006.01)          *C07K 14/47* (2006.01)

(21) Application number: **18719282.8**

(86) International application number:
**PCT/GB2018/050997**

(22) Date of filing: **17.04.2018**

(87) International publication number:
**WO 2018/193231 (25.10.2018 Gazette 2018/43)**

(54) **CELL**

ZELLE

CELLULE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **18.04.2017   GB 201706123**
**06.12.2017   GB 201720343**

(43) Date of publication of application:
**26.02.2020   Bulletin 2020/09**

(73) Proprietor: **Autolus Limited**
**London W12 7RZ (GB)**

(72) Inventors:
• **CORDOBA, Shaun**
**London W12 7RZ (GB)**
• **KOKALAKI, Evangelina**
**London W12 7RZ (GB)**
• **PULÉ, Martin**
**London W12 7RZ (GB)**
• **THOMAS, Simon**
**London W12 7RZ (GB)**
• **ONUOHA, Shimobi**
**London W12 7RZ (GB)**

(74) Representative: **D Young & Co LLP**
**120 Holborn**
**London EC1N 2DY (GB)**

(56) References cited:
WO-A1-2013/185552     WO-A1-2016/102965
WO-A1-2016/210293     WO-A2-2015/092024
WO-A2-2016/073755

**Description**

FIELD OF THE INVENTION

The present invention relates to a cell expressing a chimeric antigen receptor (CAR).

BACKGROUND TO THE INVENTION

*Chimeric antigen receptors (CARs)*

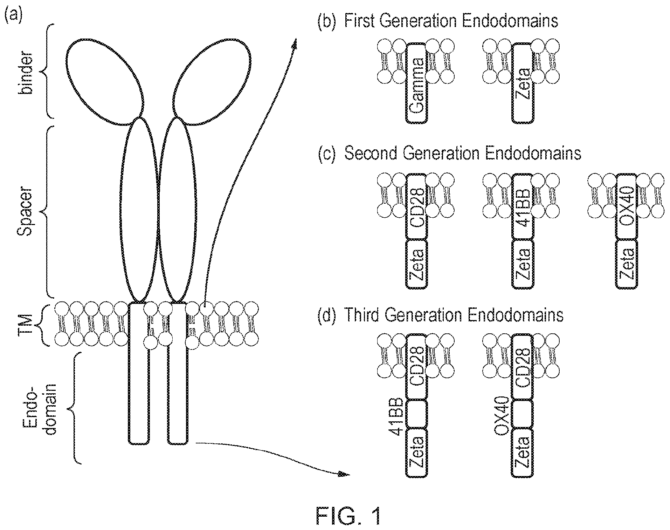

A number of immunotherapeutic agents have been described for use in cancer treatment, including therapeutic monoclonal antibodies (mAbs), bi-specific T-cell engagers and chimeric antigen receptors (CARs).
Chimeric antigen receptors are proteins which graft the specificity of a monoclonal antibody (mAb) to the effector function of a T-cell. Their usual form is that of a type I transmembrane domain protein with an antigen recognizing amino terminus (binder), and a transmembrane domain connected to a compound endodomain which transmits T-cell survival and activation signals, as shown in Figure 1(a). The endodomain may comprise one or more activating endodomains, such as 4-1BB or OX40, which transmit survival signals. Even more potent generation CARs have been described which have endodomains capable of transmitting activation, proliferation and survival signals, as shown in Figures 1 (b)-(d).
The most common form of these molecules are fusions of single-chain variable fragments (scFv) derived from monoclonal antibodies, which recognize a target antigen, fused via a trans-membrane domain to a signalling endodomain. Such molecules result in activation of the T-cell in response to recognition by the scFv of its target. When T cells express such a CAR, they recognize and kill target cells that express the target antigen. Several CARs have been developed against tumour-associated antigens, and adoptive transfer approaches using such CAR-expressing T cells are currently in clinical trial for the treatment of various cancers.
Various CARs have been tested *in vitro* and *in vivo* trials, as summarised in Table 1 below.

Table 1

| Target antigen | Associated malignancy |
|---|---|
| α-Folate receptor | Ovarian cancer |
| CAIX | Renal cell carcinoma |
| CD19 | B-cell malignancies |
| CD20 | Lymphomas and B-cell malignancies |
| CD22 | B-cell malignancies |
| CD30 | Lymphomas |
| CD33 | AML |
| CD44v7/8 | Cervical carcinoma |
| CEA | Breast and colorectal cancer |
| EGP-2 | Multiple malignancies |
| EGP-40 | Colorectal cancer |
| erb-B2 | Colorectal, breast and prostate cancer |
| erb-B 2,3,4 | Breast and others |
| FBP | Ovarian cancer |
| Fetal acetylcholine receptor | Rhabdomyosarcoma |
| GD2 | Neuroblastoma |
| GD3 | Melanoma |
| Her2/neu | Medulloblastoma, osteosarcoma, Glioblastoma, lung malignancy |
| IL-13R-a2 | Glioma, glioblastoma, medullablastoma |
| KDR | Tumor neovasculature |

(continued)

| Target antigen | Associated malignancy |
|---|---|
| k-light chain | B-cell malignancies |
| LeY | Carcinomas, epithelial-derived tumours |
| L1 cell adhesion molecule | Neuroblastoma |
| MAGE-A 1 | Melanoma |
| Mesothelin | Various tumours |
| Murine CMV infected cells | Murine CMV |
| MUC1 | Breast, Ovary |
| NKG2D ligands | Various tumours |
| Oncofetal antigen (h5T4) | Various tumours |
| PSCA | Prostate carcinoma |
| PSMA | Prostate/tumour vasculature |
| ROR1 | Various tumours |
| TAA targeted by mAb IgE | Various tumours |
| TAG-72 | Adenocarcinomas |
| VEGF-R2 | Tumor neovasculature |

However, it has been observed that adoptively transferred T-cells sometimes show limited persistence, expansion and/ or proliferation *in vivo,* due to for example, insufficient signalling, lack of IL2 or differentiation.

Additionally, adoptively transferred T-cells may succumb to inhibitory stimuli within the tumour microenvironment. For example, they may become exhausted, undergo activation induced cell death consequent to over activation, or may cause on-target off-tumour effects. These disadvantages may contribute to a reduced proliferation, expansion and persistence of the CAR T-cell even in the more potent generation CAR shown in Figures 1 (b) to 1 (d).

Other examples of adoptively transferred T-cells showing limited persistence, expansion and/ or proliferation may be due to low expression profile or low density of the target antigen on the tumour cell or the specific size of the target antigen. In this respect, large target antigens can inhibit close synapse binding, thereby decreasing the sensitivity of the CAR.

CAR T-cell persistence and activity can be enhanced by administration of cytokines, or by the CAR T-cells producing cytokines constitutively. However, these approaches have limitations: systemic administration of cytokines can be toxic; and constitutive production of cytokines may lead to uncontrolled proliferation and transformation.

There is therefore a need for alternative CAR T-cell approaches, which facilitate proliferation, expansion and persistence of T cells, which are not associated with the disadvantages mentioned above.

SUMMARY OF ASPECTS OF THE INVENTION

The present inventors have developed a new CAR system which has a boosted capacity to induce T cell signalling, leading to enhanced T-cell activation, proliferation and persistence. The CAR system includes an endodomain which increases the phosphorylation of key molecules involved in T cell activation signalling pathway. By tipping the balance of the system towards phosphorylation, the threshold necessary for T cell activation to occur is decreased.

Thus, in a first aspect of the invention, the present inventors provide a cell which co-expresses a first chimeric antigen receptor (CAR) and a second CAR, each CAR comprising an antigen-binding domain and an endodomain, wherein the endodomain of first CAR comprises the tyrosine kinase domain of a Src family kinase or the intracellular domain of CD4 or CD8 coreceptor; and the endodomain of the second CAR is an activating endodomain comprising one or more immunoreceptor tyrosine-based activation motif(s) (ITAM(s)).

The first CAR and the second CAR of the cell may bind to different antigens.

The first CAR and the second CAR of the cell may bind to the same antigen.

The first CAR and the second CAR of the cell may bind to different epitopes on the same antigen.

The first CAR and the second CAR of the cell may bind to the same epitope on the same antigen.

The endodomain of the first CAR of the cell may comprise the tyrosine kinase domain of a Src family kinase (SRK).

The endodomain of the first CAR of the cell may comprise the tyrosine kinase domain of a SRK selected from Fyn, Src, Lck, and/or mutated Lck (Y505F).

The endodomain of the first CAR of the cell may comprise the tyrosine kinase domain of Fyn.

The endodomain of the first CAR of the cell may comprise the intracellular domain of CD4 or CD8 coreceptor.

The activating endodomain of the second CAR of the cell may comprise CD3-ζ.

The activating endodomain of the second CAR of the cell may comprise CD3-ζ and a co-stimulatory domain. The co-stimulatory domain may, for example, be or comprise one or more of 4-1BB, CD28 and/or OX40.

The or each antigen may be expressed at a low density on the target cell.

The or each antigen may have a relatively long or bulky extracellular domain.

The antigen-binding domain of the first CAR and/or second CAR of the cell may bind to the antigen CD22. CD22 has a very long extracellular portion containing seven immunoglobulin domains. CD22 antigens are expressed during early stages in B-cell maturation. It is these cells that develop into B-cell acute leukaemia.

In a second aspect, the present invention provides a nucleic acid construct comprising a first nucleic acid sequence encoding a first CAR as defined in the first aspect of the invention and a second nucleic acid sequence encoding a second CAR as defined in the first aspect of the invention.

The nucleic acid construct may have the following structure:

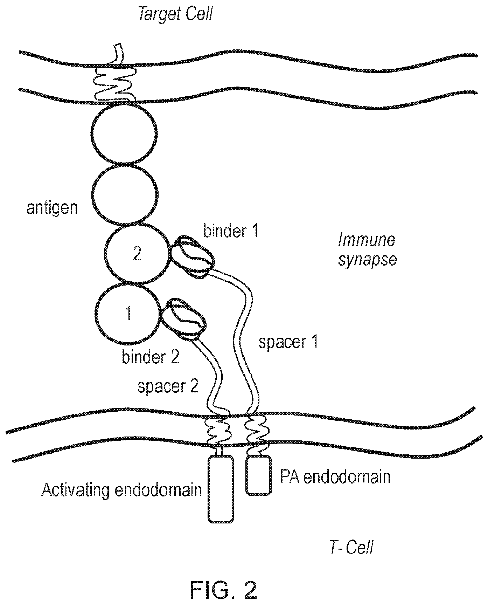

AgB1-spacer1-TM1-endo1-coexpr-AbB2-spacer2-TM2-endo2 in which

AgB1 is a nucleic acid sequence encoding an antigen-binding domain of the first CAR;
spacer 1 is a nucleic acid sequence encoding a spacer of the first CAR;
TM1 is a nucleic acid sequence encoding a transmembrane domain of the first CAR;
endo1 is a nucleic acid sequence encoding the endodomain of the first CAR;
coexpr is a nucleic acid sequence enabling co-expression of both CARs
AgB2 is a nucleic acid sequence encoding an antigen-binding domain of the second CAR;
spacer 2 is a nucleic acid sequence encoding a spacer of the second CAR;
TM2 is a nucleic acid sequence encoding a transmembrane domain of the second CAR;
Endo2 is a nucleic acid sequence encoding the activating endodomain of the second CAR;

which nucleic acid sequence, when expressed in a cell, encodes a polypeptide which is cleaved at the cleavage site such that the first and second CARs are co-expressed at the cell surface.

The sequence enabling co-expression of the two CARs may encode a self-cleaving peptide or a sequence which allows alternative means of co-expressing two CARs such as an internal ribosome entry sequence (IRES) or a second promoter.

The spacers of the first and second CARs may be different. The spacers may be tailored to the antigen or epitope recognised by the CAR so that an appropriate distance is provide for the T-cell:target cell synapse for T cell activation to occur.

Alternative codons may be used in regions of sequence encoding the same or similar amino acid sequences, such as the spacer and/or transmembrane domain of the first and second CARs. This may avoid homologous recombination.

In a third aspect, the present invention provides kit which comprises

(i) a first nucleic acid sequence encoding the first chimeric antigen receptor (CAR) as defined in the first aspect of the invention, which nucleic acid sequence has the following structure:

AgB1-spacer1-TM1-endo1
in which

AgB1 is a nucleic acid sequence encoding the antigen-binding domain of the first CAR;
spacer1 is a nucleic acid sequence encoding the spacer of the first CAR;
TM1 is a nucleic acid sequence encoding the transmembrane domain of the first CAR; and
endo1 is a nucleic acid sequence encoding the endodomain of the first CAR; and

(ii) a second nucleic acid sequence encoding the second chimeric antigen receptor (CAR) as defined in the first aspect of the invention, which nucleic acid sequence has the following structure:

Ag B2-spacer2-TM2-endo2
in which

AgB2 is a nucleic acid sequence encoding the antigen-binding domain of the second CAR;

spacer2 is a nucleic acid sequence encoding the spacer of the second CAR; and
TM2 is a nucleic acid sequence encoding the transmembrane domain of the second CAR; and
endo2 is a nucleic acid sequence encoding the activating endodomain of the second CAR.

In a fourth aspect, the present invention provides a kit comprising: a first vector which comprises the first nucleic acid sequence as defined in the third aspect of the invention; and a second vector which comprises the second nucleic acid sequence as defined in the third aspect of the invention.

The vectors may, for example, be integrating viral vectors or transposon vectors.

In a fifth aspect, the present invention provides a vector comprising a nucleic acid construct according to the second aspect of the invention. The vector may, for example, be a retroviral vector or a lentiviral vector or a transposon vector.

In a sixth aspect, the present invention provides a method for making a cell according to the first aspect of the invention, which comprises the step of introducing a nucleic acid construct according to the second aspect of the invention; a first nucleic acid sequence and second nucleic acid sequence as defined in the third aspect of the invention; and/or a first vector and a second vector according as defined in the fourth aspect of the invention, or a vector according to the fifth aspect of the invention, into a cell.

The cell may, for example, be from a sample isolated from a patient, a related or unrelated haematopoietic transplant donor, a completely unconnected donor, from cord blood, differentiated from an embryonic cell line, differentiated from an inducible progenitor cell line, or derived from a transformed cell line.

In a seventh aspect, the present invention provides a pharmaceutical composition comprising a plurality of cells according to the first aspect of the invention.

Described herein is a method for treating and/or preventing a disease, which comprises the step of administering a pharmaceutical composition according to the seventh aspect of the invention to a subject.

The method described herein may comprise the following steps:

(i) isolation of a cell-containing sample from a subject;
(ii) transduction or transfection of the cells with: a nucleic acid construct of the second aspect of the invention; a first nucleic acid sequence and second nucleic acid sequence as defined in the third aspect of the invention; and/or a first vector and a second vector as defined in the fourth aspect of the invention, or a vector according to the fifth aspect of the invention; and
(iii) administering the cells from (ii) to the subject.

The disease may be cancer. The cancer may be a B cell malignancy.

In an eighth aspect, the present invention relates to a pharmaceutical composition according to the seventh aspect of the invention for use in treating a disease.

Described herein is the use of a cell according to the first aspect of the invention in the manufacture of a medicament for treating and/or preventing a disease.

The cell of the first aspect of the invention is capable of killing a target cell. The target cell may express one or more antigens recognised by the first and/or second CAR of the cell of the present invention. The target cell may express a high level of one or more inhibitory receptors, such as PDL1. Where a target cell expresses a high level of an inhibitory receptor such as PDL1, recognition by a T-cell would recruit inhibitory phosphatases such as PD1 to the T-cell:target-cell synapse. In these circumstances it is beneficial to increase the amount of kinase (i.e. amplify phosphorylation) in order to allow T-cell activation to occur.

The present inventors have developed a new CAR system which involves two CARs, one with an activating endodomain and one with a phosphorylation amplifying endodomain. When the two CARs recognise their target antigen, the phosphorylation amplifying endodomain causes the equilibrium between phosphorylation:dephosphorylation to tip in favour of phosphorylation, amplifying cell signalling via the activating endodomain. This provides a system for increasing T-cell activation and therefore T-cell proliferation, persistence and engraftment which does not rely on cytokine administration or production.

DESCRIPTION OF THE FIGURES

**Figure 1:** (a) Schematic diagram illustrating a classical CAR. (b) to (d): Different generations and permutations of CAR endodomains: (b) initial designs transmitted ITAM signals alone through Fcε R1-γ or CD3-ζ endodomain, while later designs transmitted additional (c) one or (d) two activating endodomains, such as 4-1BB, in the same compound.

**Figure 2:** Schematic diagram of a first and second chimeric antigen receptor (CAR) of the invention. The first CAR has an endodomain comprising a phosphorylation amplifying (Pa) endodomain. The second CAR has an endodomain comprising an activating endodomain. The antigen-binding domains of the two chimeric antigen receptors bind different epitopes on the same antigen. For example, binder 1 of the first CAR binds to a first epitope, and binder 2 of the second CAR binds to a second epitope of the same antigen. The spacer may be different, for example, spacer 1 on the first CAR may be longer than spacer 2 of the second CAR. Note: Although only one chain is shown, the CARs in this system may be homodimers.

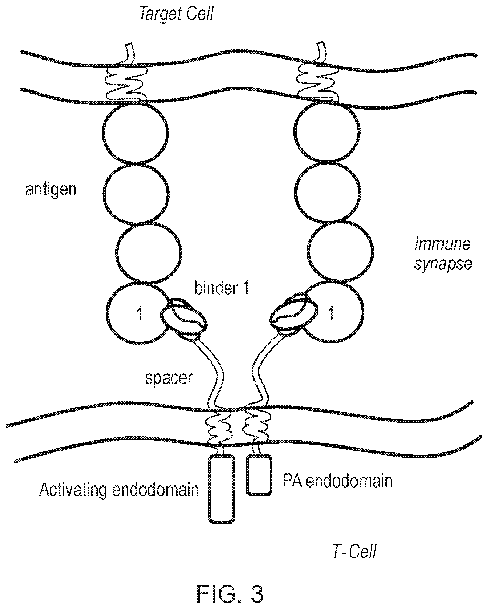

**Figure 3:** Schematic diagram of an alternative first and second chimeric antigen receptor (CAR) of the invention. The first and second CARs have a similar structure to the ones shown in Figure 2 in terms of endodomains. The difference is that the antigen-binding domains of the two chimeric antigen receptors bind the same epitopes on the antigen. For example binder 1 of the first CAR and the second CAR both bind to epitope 1 of an antigen, and they may comprise identical antigen-binding portions. Similarly, the spacer on the first and second CAR may be the same. Note: Although only one chain is shown, the CARs in this system may be homodimers.

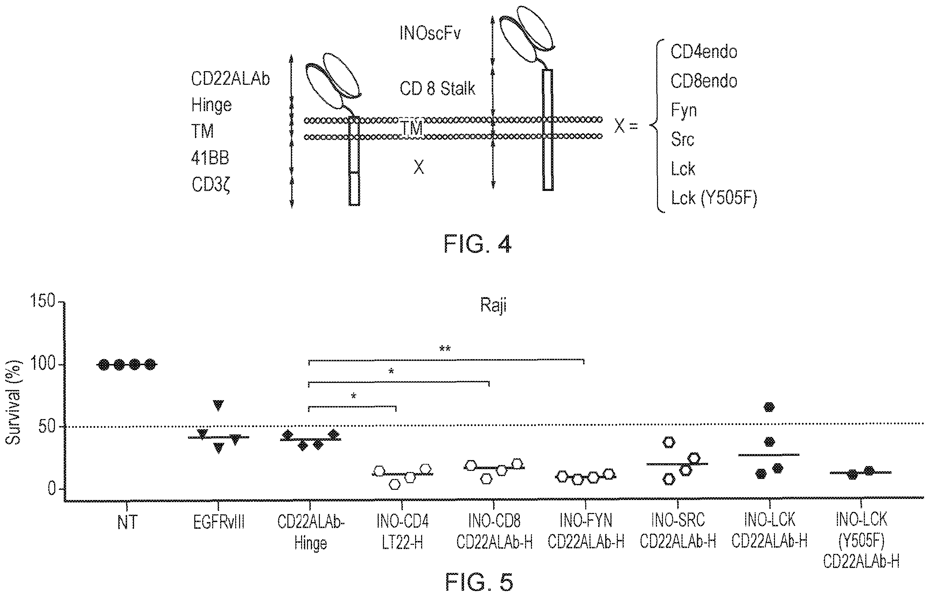

**Figure 4:** Schematic diagram of a CAR T cell, which comprises a first CAR and a second CAR, each of which binds to different epitopes on the same antigen. The first CAR comprises an inotuzumab scFv antigen binding domain which is binds to CD22, a CD8 Stalk transmembrane domain and any one or more of the following phosphorylation amplifying endodomains (X) comprising either the tyrosine kinase domain of Fyn, Src, Lck or mutated Lck (Y505F) or the intracellular domain of CD4 or CD8 coreceptors. The second CAR comprises a CD22ALab antigen binding domain also capable of binding to an epitope on a CD22 antigen, a Hinge transmembrane domain and activating endodomain comprising CD3-$\zeta$ and 4-1BB.

**Figure 5:** Cytotoxicity (72h) of CAR T cell constructs for Raji target cells. To measure cytotoxic capacity of the panel of constructs comprising a phosphorylating amplifying endodomain, each CAR was challenged against the Raji cell line. 72 hours after the T cells and Raji cells were co-cultured, the absolute number of Raji target cells was calculated, and the number in the CAR-T cell containing sample normalised according to the target number in the non-transduced (NT) condition. The normalised data are expressed as a percentage of cell survival. The construct having a first CAR comprising a phosphorylating amplifying endodomain comprising the tyrosine kinase domain of a Fyn shows the lowest overall percentage of cell survival compared to the other phosphorylating amplification endodomains.

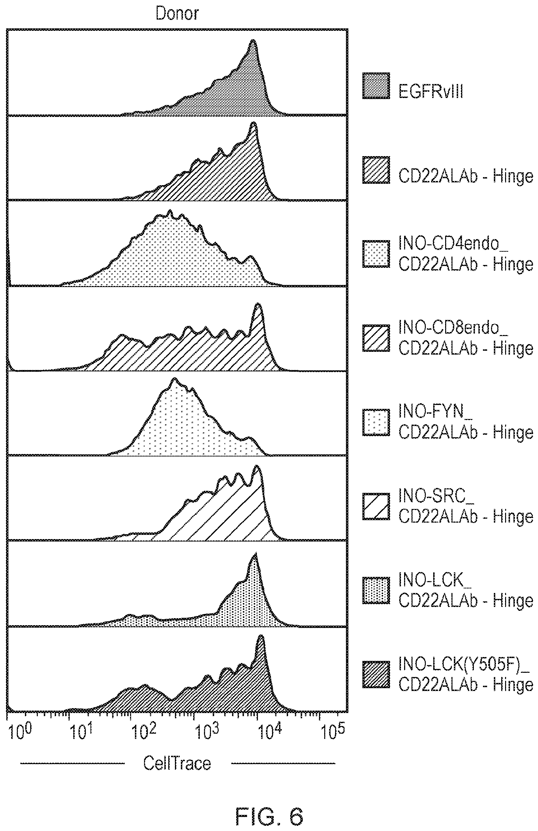

**Figure 6:** T-cell proliferation (day 7) following co-culture with Raji cells. CD56-depleted CAR expressing T cells were analysed by flow cytometry to measure the dilution of the Cell Trace Violet (CTV) which occurs as the T-cells divide. The T cells labelled with CTV are excited with a 405 nm (violet) laser. Proliferation of the CAR T cells comprising a phosphorylating amplifying endodomain is shown to be significantly improved for the donor tested compared to the constructs lacking a phosphorylating amplifying endodomain.

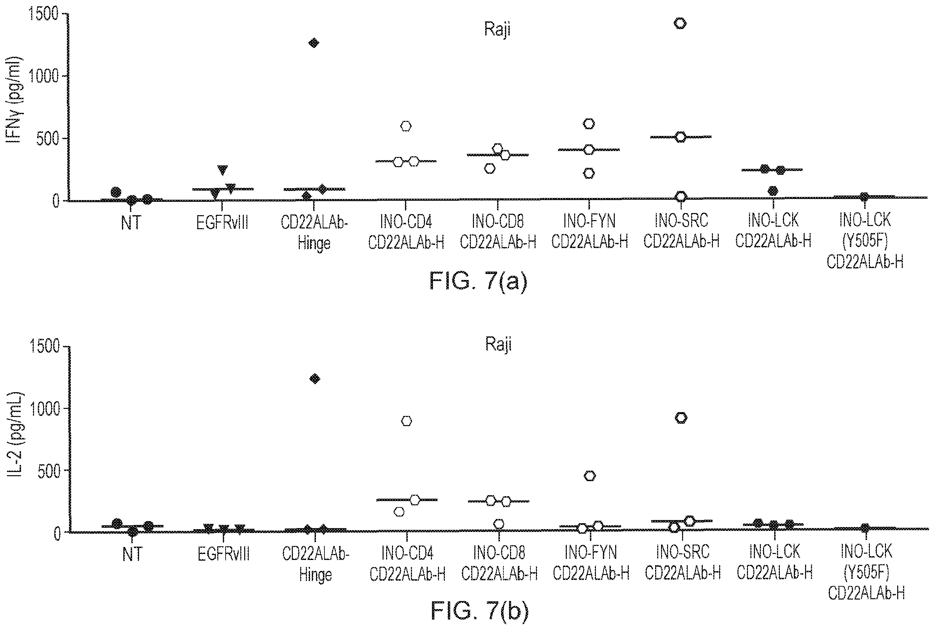

**Figure 7(a):** IFN-$\gamma$ cytokine production (72h) after co-culture with Raji cells. T-cells expressing the panel of CAR constructs having different phosphorylating amplifying endodomains were compared for IFN-$\gamma$ secretion after 72h co-culture with Raji target cells.

**Figure 7(b):** IL-2 cytokine production (72h) after co-culture with Raji cells. T-cells expresiing the panel of CAR constructs having different phosphorylating amplifying endodomains were compared for IL-2 secretion after 72h co-culture with Raji target cells.

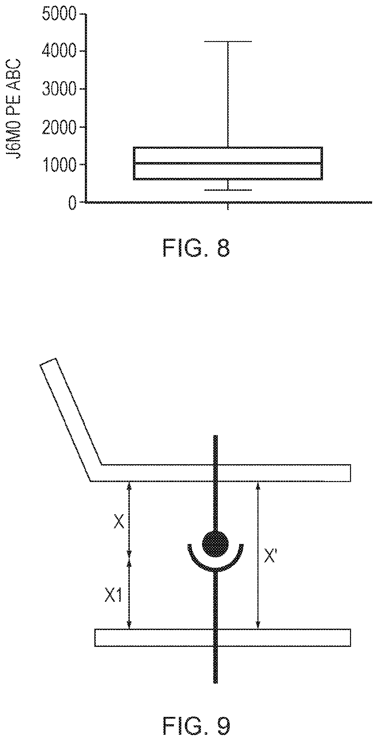

**Figure 8** - Expression data of BCMA on primary myeloma cells
Myeloma cells from bone marrow samples from 39 multiple myeloma patients were isolated by a CD138+ magnetic bead selection. These cells were stained with the anti-BCMA monoclonal antibody J6MO conjugated with PE (GSK). Antigen copy number was quantified using PE Quantibrite beads (Becton Dickenson) as per the manufacturer's instructions. A box and whiskers plot of antigen copy number is presented along with the range, interquartile and median values plotted. It was found that the range is 348.7-4268.4 BCMA copies per cell with a mean of 1181 and a median of 1084.9.

**Figure 9** - Schematic diagram illustrating the distance parameters at a T-cell:target cell synapse.

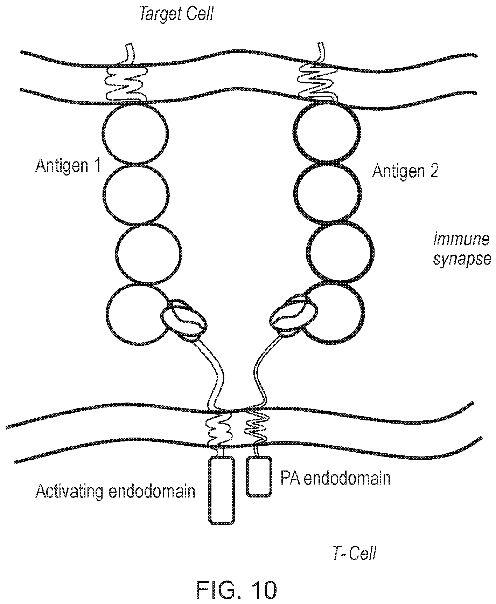

**Figure 10** - Schematic diagram of an alternative first and second chimeric antigen receptor (CAR) of the invention. The first and second CARs have a similar structure to the ones shown in Figure 3 in terms of endodomains. The difference is that the antigen-binding domains of the two chimeric antigen receptors bind to different target antigens.

**Figure 11** - Schematic diagram of an alternative first and second chimeric antigen receptor (CAR) of the invention. As for the arrangement shown in Figure 10, the antigen-binding domains of the first and second CARs bind to different target antigens. In this arrangement, one CAR comprises a phosphorylation amplifying domain together with a co-stimulatory domain.

**Figure 12** - Cytotoxicity (72h) of CAR T cells expressing various CAR systems against SupT1 target cells expressing the target antigen CD22 at an average copy number of 0, 255, 634, 1090 or 78,916 copies per cell

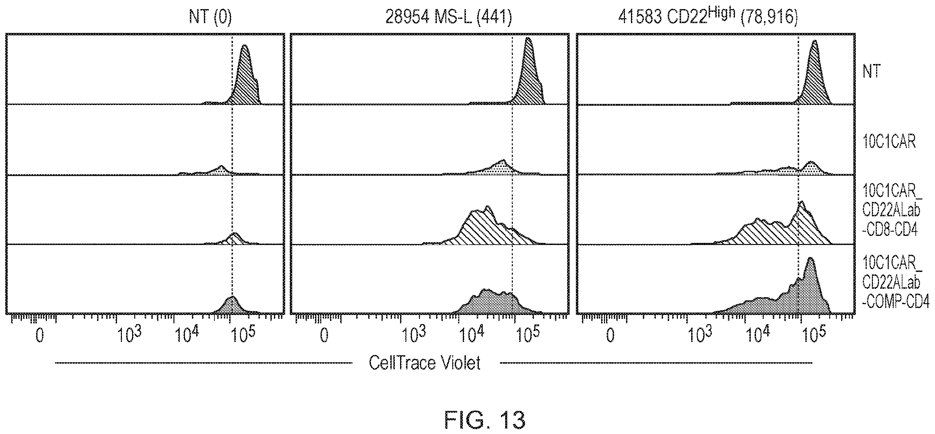

**Figure 13** - T-cell proliferation (day 7) following co-culture with SupT1 target cells expressing the target antigen CD22 at an average copy number of 441 or 78,916 copies per cell.

DETAILED DESCRIPTION

CHIMERIC ANTIGEN RECEPTORS (CARs)

CARs, which are shown schematically in Figure 1, are chimeric type I trans-membrane proteins which connect an extracellular antigen binding domain (binder) to an endodomain. The binder is typically a single-chain variable fragment (scFv) derived from a monoclonal antibody (mAb) such as inotuzumab, but it can be based on other formats which comprise an antibody-like antigen binding site. A spacer domain is usually necessary to isolate the binder from the membrane and to allow it a suitable orientation. A common spacer domain used is the Fc of IgG1. More compact spacers can suffice e.g. the stalk from CD8$\alpha$ and even just the IgG1 hinge alone, depending on the different antigens, and/or indeed the different epitopes of the same antigen. A transmembrane domain anchors the protein in the cell membrane and connects the spacer to the endodomain. In a classical, activating CAR, the endodomain comprises an intracellular signalling domain.

Early CAR designs had endodomains derived from the intracellular parts of either the $\gamma$ chain of the Fc$\varepsilon$R1 or CD3-$\zeta$. Consequently, these first generation receptors transmitted immunological signal 1, which was sufficient to trigger T-cell killing of cognate target cells but failed to fully activate the T-cell to proliferate and survive. To overcome this limitation, compound endodomains have been constructed: fusion of the intracellular part of a T-cell co-stimulatory molecule to that of CD3-$\zeta$ results in second generation receptors which can transmit an activating and co-stimulatory signal simultaneously after antigen recognition. The co-stimulatory domain commonly used is that of CD28. This supplies a potent co-stimulatory signal - namely immunological signal 2 (IL2), which triggers T-cell proliferation. Receptors described herein include TNF receptor family endodomains, such as the closely related OX40 and 4-1BB, which transmit survival signals, as shown in Figure 1(c). Even more potent third generation CARs have also been described which have endodomains capable of transmitting activation, proliferation and survival signals, as shown in Figure 1(d). Receptors in the present invention comprise activating endodomains that either include a 4-1BB or do not include a 4-1 BB domain.

CAR-encoding nucleic acids may be transferred to T cells using, for example, retroviral vectors. Lentiviral vectors may be employed. In this way, a large number of cancer-specific T cells can be generated for adoptive cell transfer. When the CAR binds the target-antigen, this results in the transmission of an activating signal to the T-cell it is expressed on. Thus, the CAR is capable of directing specificity and cytotoxicity of the T cell towards tumour cells expressing the targeted antigen.

*T-cell activation (resting state):*

T-cell receptors (TCR) and CARs cause T-cell signalling by stimulating tyrosine phosphorylation: the addition of a phosphate group to the amino acid tyrosine on a protein. Tyrosine-protein kinases transfer a phosphate group from ATP to a tyrosine residue in a protein. These enzymes can be divided into two main groups: receptor tyrosine kinases (RTK), which are transmembrane proteins involved in signal transduction; and cytoplasmic / non-receptor tyrosine kinases, which act as regulatory proteins, playing key roles in cell differentiation, motility, proliferation, and survival.

In the resting state, the molecules involved in T cell-signalling are repeatedly colliding by means of diffusion. For example, the TCR/CD3 complex is constantly being phosphorylated by a Src-family kinase (SFK), a tyrosine kinase, either free or non-covalently bound to the cytoplasmic tails of CD4 or CD8 co-receptors.

In turn, the complex is continuously dephosphorylated by tyrosine phosphatases, such as CD45 and CD148. The continuous phosphorylation and dephosphorylation happens in a random manner, and in the absence of ligand binding, this equilibrium favours dephosphorylation and the overall phosphorylation of TCR is low such that T-cell activation does not proceed.

*T cell activation (activated state):*

Antigen recognition by the TCR is the first step in T-cell activation. At the start of the signalling cascade, the immunoreceptor tyrosine-based activation motifs (ITAMs) of CD3 are phosphorylated by a SFK (Lck) and then bound by another kinase called ZAP70. After ZAP70 binds to CD3, co-receptors CD4 or CD8 become associated with the TCR/CD3 complex and bind to the major compatibility complex (MHC). CD8 co-receptor association with the complex stabilises the TCR-MHC peptide (MHCp) interaction and the recruited/free Lck continues the phosphorylation of CD3 elements, ZAP70, as well as many other downstream targets.

The TCR-MHCp and CAR-target cell antigen complex spans a short length. This forms small zones of close contact, from which the inhibitory CD45 and CD148 phosphatase molecules with ectodomains too large to fit are excluded. CD45 steric exclusion extends the phosphorylation half-lives of TCR/MHCp complexes or CAR-target cell antigen complexes, which are trapped within the close-contact zone. In the absence of CD45, Lck is favoured, which phosphorylates ITAMs. Such prolonged phosphorylation allows more time for ZAP-70 recruitment, its activation by phosphorylation, and subsequent phosphorylation of adaptor proteins. This extended and increased level of phosphorylation tips the balance of the resting T cell phosphorylation/ dephosphorylation equilibrium state towards phosphorylation, meeting the T cell activation threshold required for activation to proceed.

A central mechanism of CAR T-cell activation is therefore the equilibrium shift of phosphorylation/ dephosphorylation events that occur upon target cell antigen recognition.

The present inventors have devised a CAR system which effectively lowers the T cell activation threshold (of phosphorylation events required for T cell activation to proceed) by manipulating the equilibrium towards phosphorylation. This is done by including a phosphorylation amplification endodomain into the CAR system.

In one aspect, the present invention relates to a cell which co-expresses a first CAR and a second CAR, wherein the first CAR comprises an endodomain comprising the tyrosine kinase domain of a Src family kinase or the intracellular domain of CD4 or CD8 coreceptor, and wherein the second CAR comprises an activating endodomain comprising one or more immunoreceptor tyrosine-based activation motif(s) (ITAM(s)).

ENDODOMAIN

The endodomain of the first CAR of the cell of the present invention is an intracellular domain which can either directly or indirectly amplify the phosphorylation of an ITAM. For example, the endodomain may be able to directly or indirectly amplify the phosphorylation of one or more ITAM(s) present in the activating endodomain of the second CAR.

An example of direct amplification of phosphorylation is phosphorylation by a kinase. The tyrosine kinase domain of a SRK protein can directly phosphorylate the tyrosine residues on ITAMs.

An example of indirect amplification of phosphorylation is via recruitment of a kinase. For example, the intracellular domains of CD4 and CD8 coreceptors indirectly indirectly amplify phosphorylation of ITAMs by clustering with the CAR T cell antigen complex, recruiting further SRK proteins, which then directly phosphorylate the ITAMs. These coreceptors also further stabilise the CAR T cell antigen complex, which optimizes signalling through the CAR T cell receptor complex required for T cell activation.

The endodomain of the first CAR may comprise all or part of the tyrosine kinase domain of an SRK protein.

Alternatively or additionally, the endodomain of the first CAR may comprise all or part of the intracellular domain of a CD4 coreceptor or the CD8 coreceptor.

SRC FAMILY KINASE (SRK)

SRK member proteins contain a 14-carbon myristic acid moiety attached to an SH4 domain, a SH3 domain followed by an SH2 domain, an SH2-kinase linker, a tyrosine kinase domain (also known as a catalytic domain or a SH1 domain), and a C-terminal regulatory segment. The tyrosine kinase domain of a SRK protein is an example of an endodomain in the context of the present invention.

SRK is a family of non-receptor tyrosine kinases that includes nine members: Src, Yes, Fyn and Frg, forming the SrcA subfamily, Lck, Hck, Blk and Lyn in the SrcB subfamily and Frk in its own subfamily. The SrcA and SrcB subfamilies are specific to vertebrates; however, Src homologs exist in organisms as diverse as unicellular choanoflagellates.

SRKs interact with many cellular cytosolic, nuclear and membrane proteins, modifying these proteins by phosphorylation of tyrosine residues. The SH3, SH2 and kinase domains of SFK display large sequence and structural similarity. They also have in common a myristoylated and/or palmitoylated membrane anchoring region in the N-terminus, including positively charged residues (Arg and/or Lys), known as the SH4 domain. The activity of SFKs is largely controlled by the equilibrium between phosphorylation and dephosphorylation at a C-terminal inhibitory tyrosine and an activating tyrosine in the catalytic domain.

The endodomain of the first CAR of the cell may comprise the tyrosine kinase domain of a SRK protein. Sequences of such domains are disclosed as SEQ ID NOs: 1, 3, 5, and 7.

Alternatively, the endodomain of the first CAR of the cell may comprise the full-length sequence of a SRK protein. Sequences of such domains are disclosed below as SEQ ID NO: 2, 4, 6 and 8.

The endodomain of the first CAR may comprise a variant of one of the sequence shows as SEQ ID No. 1 to 8 having at least 80, 85, 90, 95, 98 or 99% sequence identity, provided that the variant sequence retains the capacity to amplify phosphorylation. The variant should retain tyrosine kinase activity.

FYN

Fyn (UniProt ID: P06241) is a 59 kda member of the SRK family, typically associated with T cell and neuronal signalling in development and normal cell physiology. It encodes a membrane-associated non-receptor tyrosine kinase that plays a role in many biological processes including regulation of cell growth and survival, cell adhesion, integrin-mediated signaling, cytoskeletal remodelling, cell motility, immune response and axon guidance. Fyn is phosphorylated on its C-terminal tail within the catalytic domain (also known as the tyrosine kinase domain). Fyn participates in the downstream signaling pathways that lead to T-cell differentiation and proliferation following T-cell receptor (TCR) stimulation. Fyn also participates in negative feedback regulation of TCR signaling through phosphorylation.

The inventors have discovered that Fyn is a particularly effective endodomain for use in a cell comprising a first CAR and a second CAR of the present invention. For example, the Fyn-CAR construct shown in Figure 5 has the lowest overall percentage of cell survival compared to the other phosphorylating amplification domains.

Tyrosine kinase domain Fyn (SEQ ID NO: 1)

LQLIKRLGNGQFGEVVMGTWNGNTKVAIKTLKPGTMSPESFLEEAQIMKKLKHDKLVQLYAVV

SEEPIYIVTEYMNKGSLLDFLKDGEGRALKLPNLVDMAAQVAAGMAYIERMNYIHRDLRSANILV

GNGLICKIADFGLARLIEDNEYTARQGAKFPIKWTAPERALYGRFTIKSDVWSFGILLTELVTKGR

VPYPGMNNREVLEQVERGYRMPCPQDCPISLHELMIHCWKKDPEERPTFEYLQSFLEDYF

Full-length Fyn (SEQ ID NO: 2):

GCVQCKDKEATKLTEERDGSLNQSSGYRYGTDPTPQHYPSFGVTSIPNYNNFHAAGGQGLTV

FGGVNSSSHTGTLRTRGGTGVTLFVALYDYEARTEDDLSFHKGEKFQILNSSEGDWWEARSL

TTGETGYIPSNYVAPVDSIQAEEWYFGKLGRKDAERQLLSFGNPRGTFLIRESETTKGSYSLSIR

DWDDMKGDHVKHYKIRKLDNGGYYITTRAQFETLQQLVQHYSERAAGLCCRLVVPCHKGMPR

LTDLSVKTKDVWEIPRESLQLIKRLGNGQFGEVVMGTWNGNTKVAIKTLKPGTMSPESFLEEA

QIMKKLKHDKLVQLYAVVSEEPIYIVTEYMNKGSLLDFLKDGEGRALKLPNLVDMAAQVAAGMA

YIERMNYIHRDLRSANILVGNGLICKIADFGLARLIEDNEYTARQGAKFPIKWTAPERALYGRFTI

KSDVWSFGILLTELVTKGRVPYPGMNNREVLEQVERGYRMPCPQDCPISLHELMIHCWKKDPE

ERPTFEYLQSFLEDYFTATEPQYQPGENL

SRC

Src (UniProt ID: P12931) is a 59 kDa member of the SRK family. This proto-oncogene also known as c-Src, is a non-receptor tyrosine kinase protein. Src is activated following engagement of different classes of cellular receptors including immune response receptors, integrins and other adhesion receptors, receptor protein tyrosine kinases, G protein-coupled receptors as well as cytokine receptors. Src participates in signaling pathways that control a diverse spectrum of biological activities including gene transcription, immune response, cell adhesion, cell cycle progression, apoptosis, migration, and transformation.

Tyrosine kinase domain of Src (SEQ ID NO: 3)

LRLEVKLGQGCFGEVWMGTWNGTTRVAIKTLKPGTMSPEAFLQEAQVMKKLRHEKLVQLYAV
VSEEPIYIVTEYMSKGSLLDFLKGETGKYLRLPQLVDMAAQIASGMAYVERMNYVHRDLRAANI
LVGENLVCKVADFGLARLIEDNEYTARQGAKFPIKWTAPEAALYGRFTIKSDVWSFGILLTELTT
KGRVPYPGMVNREVLDQVERGYRMPCPPECPESLHDLMCQCWRKEPEERPTFEYLQAFLED
YF

Full-length Src (SEQ ID NO: 4):

MGSNKSKPKDASQRRRSLEPAENVHGAGGGAFPASQTPSKPASADGHRGPSAAFAPAAAEP
KLFGGFNSSDTVTSPQRAGPLAGGVTTFVALYDYESRTETDLSFKKGERLQIVNNTEGDWWLA
HSLSTGQTGYIPSNYVAPSDSIQAEEWYFGKITRRESERLLLNAENPRGTFLVRESETTKGAYC
LSVSDFDNAKGLNVKHYKIRKLDSGGFYITSRTQFNSLQQLVAYYSKHADGLCHRLTTVCPTSK
PQTQGLAKDAWEIPRESLRLEVKLGQGCFGEVWMGTWNGTTRVAIKTLKPGTMSPEAFLQEA
QVMKKLRHEKLVQLYAVVSEEPIYIVTEYMSKGSLLDFLKGETGKYLRLPQLVDMAAQIASGMA
YVERMNYVHRDLRAANILVGENLVCKVADFGLARLIEDNEYTARQGAKFPIKWTAPEAALYGRF
TIKSDVWSFGILLTELTTKGRVPYPGMVNREVLDQVERGYRMPCPPECPESLHDLMCQCWRK
EPEERPTFEYLQAFLEDYFTSTEPQYQPGENL

LCK

Lck (UniPort ID: P06239) is a 56kDa on-receptor tyrosine-protein kinase that plays an essential role in the selection and maturation of developing T-cells in the thymus and in the function of mature T-cells. It also plays a key role in T-cell antigen receptor (TCR)-linked signal transduction pathways. Lck is constitutively associated with the cytoplasmic portions of the CD4 and CD8 surface receptors, and association of the TCR with a peptide antigen-bound MHC complex facilitates the interaction of CD4 and CD8 with MHC class II and class I molecules, respectively, thereby recruiting the associated Lck protein to the vicinity of the TCR/CD3 complex. Lck then phosphorylates tyrosines residues within the immunoreceptor tyrosine-based activation motifs (ITAM) of the cytoplasmic tails of the TCR-gamma chains and CD3 subunits, initiating the TCR/CD3 signaling pathway. Once stimulated, the TCR recruits the tyrosine kinase ZAP70, that becomes phosphorylated and activated by Lck. Following this, a large number of signaling molecules are recruited, ultimately leading to lymphokine production. Lck also contributes to signaling by other receptor molecules.

Tyrosine kinase domain of Lck (SEQ ID NO: 5):

LKLVERLGAGQFGEVWMGYYNGHTKVAVKSLKQGSMSPDAFLAEANLMKQLQHQRLVRLYA
VVTQEPIYIITEYMENGSLVDFLKTPSGIKLTINKLLDMAAQIAEGMAFIEERNYIHRDLRAANILVS
DTLSCKIADFGLARLIEDNEYTAREGAKFPIKWTAPEAINYGTFTIKSDVWSFGILLTEIVTHGRIP
YPGMTNPEVIQNLERGYRMVRPDNCPEELYQLMRLCWKERPEDRPTFDYLRSVLEDFF

Full-length Lck (SEQ ID NO: 6):

MGCGCSSHPEDDWMENIDVCENCHYPIVPLDGKGTLLIRNGSEVRDPLVTYEGSNPPASPLQ
DNLVIALHSYEPSHDGDLGFEKGEQLRILEQSGEVVWKAQSLTTGQEGFIPFNFVAKANSLEPE
PWFFKNLSRKDAERQLLAPGNTHGSFLIRESESTAGSFSLSVRDFDQNQGEVVKHYKIRNLDN
GGFYISPRITFPGLHELVRHYTNASDGLCTRLSRPCQTQKPQKPWWEDEWEVPRETLKLVERL
GAGQFGEVWMGYYNGHTKVAVKSLKQGSMSPDAFLAEANLMKQLQHQRLVRLYAVVTQEPI
YIITEYMENGSLVDFLKTPSGIKLTINKLLDMAAQIAEGMAFIEERNYIHRDLRAANILVSDTLSCKI
ADFGLARLIEDNEYTAREGAKFPIKWTAPEAINYGTFTIKSDVWSFGILLTEIVTHGRIPYPGMTN
PEVIQNLERGYRMVRPDNCPEELYQLMRLCWKERPEDRPTFDYLRSVLEDFFTATEGQYQPQ
P

The Lck mutant (Y505F) provided herein is a constitutively active variant showing significantly increased cytotoxicity (see Figures 5, 6 and 7a and 7b). The Y505F mutant protects against CSK phosphorylation at the 505 tyrosine residue.

Tyrosine kinase domain of Lck_Y505F (SEQ ID NO: 7)

LKLVERLGAGQFGEVWMGYYNGHTKVAVRSLKQGSMSPDAFLAEANLMKQLQHQRLVRLYA
VVTQEPIYIITEYMENGSLVDFLKTPSGIKLTINKLLDMAAQIAEGMAFIEERNYIHRDLRAANILVS
DTLSCKIADFGLARLIEDNEYTAREGAKFPIKWTAPEAINYGTFTIKSDVWSFGILLTEIVTHGRIP
YPGMTNPEVIQNLERGYRMVRPDNCPEELYQLMRLCWKERPEDRPTFDYLRSVLEDFF

Full length Lck_Y505F (SEQ ID NO: 8):

MGCGCSSHPEDDWMENIDVCENCHYPIVPLDGKGTLLIRNGSEVRDPLVTYEGSNPPASPLQ
DNLVIALHSYEPSHDGDLGFEKGEQLRILEQSGEVVWKAQSLTTGQEGFIPFNFVAKANSLEPE
PWFFKNLSRKDAERQLLAPGNTHGSFLIRESESTAGSFSLSVRDFDQNQGEVVKHYKIRNLDN
GGFYISPRITFPGLHELVRHYTNASDGLCTRLSRPCQTQKPQKPWWEDEWEVPRETLKLVERL
GAGQFGEVWMGYYNGHTKVAVRSLKQGSMSPDAFLAEANLMKQLQHQRLVRLYAVVTQEPI
YIITEYMENGSLVDFLKTPSGIKLTINKLLDMAAQIAEGMAFIEERNYIHRDLRAANILVSDTLSCKI
ADFGLARLIEDNEYTAREGAKFPIKWTAPEAINYGTFTIKSDVWSFGILLTEIVTHGRIPYPGMTN
PEVIQNLERGYRMVRPDNCPEELYQLMRLCWKERPEDRPTFDYLRSVLEDFFTATEGQFQPQ
P

INTRACELLULAR CD4 AND CD8 CO-RECEPTORS

Membrane proteins CD4 and CD8 co-receptors are expressed on T helper (Th) cells and cytotoxic T lymphocytes (CTL). They are non-polymorphic cell surface glycoproteins expressed on subsets of thymocytes and mature peripheral T cells. Generally, CD4 is expressed on Th cells that recognise antigens in association with class II MHC molecules, and CD8 is expressed on CTLs that recognize antigens in association with class I MHC molecules. CD4 and CD8 actively participate as co-receptors during T cell signalling and enhance antigen responsiveness mediated by TCR.

It has been shown that CD4 and CD8 associated with equal amounts of Lck, both enhanced IL2 production equivalently when cross-linked with suboptimal levels of anti-TCR antibody. The cytoplasmic tail portion of CD8 and CD4 coreceptors interacts with the cytoplasmic tail of CD3-ζ.

The endodomain of the first CAR of the cell may comprise the intracellular domain (also known as the cytoplasmic tail) of a CD4 or CD8 coreceptor. Sequences of such domains are disclosed as SEQ ID NOs: 9 and 11, respectively.

Alternatively, the endodomain of the first CAR of the cell may comprise the full-length sequence of a CD4 or CD8 coreceptor, or truncations thereof. The full-length sequence of the CD4 and CD8 coreceptor are provided below as SEQ ID Nos: 10 and 12, respectively.

The endodomain of the first CAR may comprise a variant of one of the sequence shown as SEQ ID No. 9 to 12 having at least 80, 85, 90, 95, 98 or 99% sequence identity, provided that the variant sequence retains the capacity to amplify phosphorylation. The variant should retain the capacity to bind an SRK kinase such as Lck.

CD4 CORECEPTOR

CD4 (UniProt ID: P01730) is a co-receptor that assists the T cell receptor (TCR) in communicating with an antigen-presenting cell. Using its intracellular domain, CD4 amplifies the signal generated by the TCR by recruiting Lck, which is essential for activating many molecular components of the signaling cascade of an activated T cell. Various types of Th cells are thereby produced. CD4 also interacts directly with MHC class II molecules on the surface of the antigen-presenting cell using its extracellular domain. The extracellular domain adopts an immunoglobulin-like beta-sandwich with seven strands in 2 beta sheets.

During antigen presentation, both the TCR complex and CD4 are recruited to bind to different regions of the MHCII molecule ($\alpha$1/$\beta$1 and $\beta$2, respectively). Close proximity between the TCR complex and CD4 in this situation means the Lck kinase bound to the cytoplasmic tail of CD4 is able to tyrosine-phosllorylate the Immunoreceptor tyrosine activation motifs (ITAM) present on the cytoplasmic domains of CDS. Phosphorylated ITAM motifs on CD3 recruits and activates SH2 domain-containing protein tyrosine kinases (PTK) such as ZAP70 to further mediate downstream signal transduction via tyrosine phosphorylation, leading to transcription factor activation including NF-$\kappa$B and consequent T cell activation.

Cytoplasmic tail of CD4 (SEQ ID NO: 9)
CVRCRHRRRQAERMSQIKRLLSEKKTCQCPHRFQKTCSPI

Full-length CD4 (SEQ ID NO: 10):

MNRGVPFRHLLLLVLQLALLPAATQGKKVVLGKKGDTVELTCTASQKKSIQFHWKNSNQIKILGN

QGSFLTKGPSKLNDRADSRRSLWDQGNFPLIIKNLKIEDSDTYICEVEDQKEEVQLLVFGLTANS

DTHLLQGQSLTLTLESPPGSSPSVQCRSPRGKNIQGGKTLSVSQLELQDSGTWTCTVLQNQK

KVEFKIDIVVLAFQKASSIVYKKEGEQVEFSFPLAFTVEKLTGSGELWWQAERASSSKSWITFDL

KNKEVSVKRVTQDPKLQMGKKLPLHLTLPQALPQYAGSGNLTLALEAKTGKLHQEVNLVVMRA

TQLQKNLTCEVWGPTSPKLMLSLKLENKEAKVSKREKAVWVLNPEAGMWQCLLSDSGQVLLE

SNIKVLPTWSTPVQPMALIVLGGVAGLLLFIGLGIFFCVRCRHRRRQAERMSQIKRLLSEKKTCQ

CPHRFQKTCSPI

CD8 CORECEPTOR

CD8 (UniProt ID: P01732) plays a key role in signal transduction by recruiting essential signaling components to the cytoplasmic side of the TCR-CD3-$\zeta$ complex. Evidence by Wooldridge et al., (2005) J Biol Chem; 280(30):27491-501 shows that although CD8 and TCR do not bind cooperatively to pMHCI, TCR associates with CD8 on the T cell surface and the interaction stabilises the T cell receptor antigen complex.

Cytoplasmic tail of CD8 (SEQ ID NO: 11)
LYCNHRNRRRVCKCPRPVVKSGDKPSLSARYV

Full-length CD8 (SEQ ID NO: 12):

MALPVTALLLPLALLLHAARPSQFRVSPLDRTWNLGETVELKCQVLLSNPTSGCSWLFQPRGA
AASPTFLLYLSQNKPKAAEGLDTQRFSGKRLGDTFVLTLSDFRRENEGYYFCSALSNSIMYFSH
FVPVFLPAKPTTTPAPRPPTPAPTIASQPLSLRPEACRPAAGGAVHTRGLDFACDIYIWAPLAGT
CGVLLLSLVITLYCNHRNRRRVCKCPRPVVKSGDKPSLSARYV

## CO-STIMULATORY ENDODOMAIN

The first CAR may comprise one or more co-stimulatory domains in addition to the endodomain. The co-stimulatory domain(s) may be membrane proximal or membrane-distal in comparison with the endodomain.

The costimulatory endodomain may comprises one or more of the following: CD28, OX40 and 4-1BB endodomain, which are described in more detail below.

The costimulatory domain may comprise a CD28 endodomain in combination with an OX40 or 41 BB endodomain.

## ANTIGEN BINDING DOMAIN

The antigen-binding domain is the portion of the CAR which recognizes the antigen. Numerous antigen-binding domains are known in the art, including those based on the antigen-binding site of an antibody, antibody mimetics, and T-cell receptors. For example, the antigen-binding domain may comprise: a single-chain variable fragment (ScFv) derived from a monoclonal antibody; a natural ligand of the target antigen; a peptide with sufficient affinity for the target; a single domain antibody; an artificial single binder such as a Darpin (designed ankyrin repeat protein); or a single-chain derived from a T-cell receptor.

A 'target antigen' is an entity, which is specifically recognised and bound by the antigen-binding domain of a CAR. The term 'ligand' is used synonymously with 'antigen'. An 'epitope' refers to the specific binding site to which the antigen-binding domain (e.g., scFv portion of the CAR) binds.

In the context of the first aspect of the invention, the antigen-binding domain of first CAR and second CAR of the cell may recognise different antigens. Alternatively, the antigen-binding domain of the first CAR and the second CAR of the cell may recognise the same antigen. In one embodiment, the antigen binding domains of the first CAR and second CAR may recognise distinct epitopes of the same antigen. Where antigen binding domains of the first CAR and second CAR recognise distinct epitopes of the same antigen, it is possible for the first CAR and second CAR to bind the same antigen molecule which automatically brings the endodomain of the first CAR into proximity with the activating endodomain of the second CAR.

In the CAR system of the present invention, maximal activation is achieved when both the first and second CAR bind their target antigen. Where the first CAR and second CAR bind different antigens, this provides an extra level of safety, as maximal activation will only occur when two targets e.g. two tumour-specific antigens are present. This is useful in the field of oncology as indicated by the Goldie-Coldman hypothesis: sole targeting of a single antigen may result in tumour escape by modulation of said antigen due to the high mutation rate inherent in most cancers. By simultaneously targeting two different antigens, the probability of such escape is reduced.

The target antigen may be an antigen present on a cancer cell, for example a tumour-associated antigen. It may, for example, be one of the antigens listed in Table 1.

## TARGET ANTIGEN DENSITY

The target antigen for the first and/or second CAR may be expressed at relatively low density on the target cell. The target antigen for the first and/or second CAR may be of a size or configuration that causes it to be excluded from a T-cell:target cell synapse.

The target antigen may be expressed at a low density on the cell surface. Thus the cells of the present invention may be capable of killing target cells, such as cancer cells, which express a low density of the TAA. Examples of TAAs which are known to be expressed at low densities in certain cancers include, but are not limited to, ROR1 in CLL, Typr-1 in melanoma, BCMA and TACI in myeloma, CD22 in B-cell malignancies and ALK in Neuroblastoma.

Example 4 describes a study investigating the expression of BCMA on myeloma cells. It was found that the range of BCMA copy number on a myeloma cell surface is low: at 348.7-4268.4 BCMA copies per cell with a mean of 1181 and a median of 1084.9 (Figure 8).

Example 5 demonstrates that the inclusion of a CAR expressing a Pa domain increases killing and T-cell persistence with target cells expressing low densities of the target antigen CD22. The effect was observed for target cells expressing approximately 1090, 634, 441 and 255 copies of target antigen per cell on average.

The mean copy number of the target antigen, for example the target antigen for the second CAR, may be fewer than about 10,000; 5,000; 3,000; 2,000; 1,000; 500 copies or 250 per target cell.

The copy number of an antigen on a cell, such as a cancer cell may be measured using standard techniques, such as using PE Quantibrite beads as described in Example 4.

The copy number of the target antigen for the first CAR may be different from the copy number of the target antigen for the second CAR.

For example, the target antigen for the second CAR (which comprises an activating endodomain) may be expressed by the target cell at a lower antigen density that the target antigen for the first CAR (which comprises an endodomain comprising the tyrosine kinase domain of a Src family kinase or the intracellular domain of CD4 or CD8 coreceptor).

The target antigen for the second CAR (which comprises an activating endodomain) may be expressed by the target cell at an average copy number of 1000, 500 or 250 copies per cell or fewer. The target antigen for the first CAR (which comprises an endodomain comprising the tyrosine kinase domain of a Src family kinase or the intracellular domain of CD4 or CD8 coreceptor) may be expressed by the target cell at an average copy number of at least 1000; 2,000; 3,000; 5,000 or 10,000 copies per cell.

The following table summarises examples of high density tissue-specific antigens and low-density tumour-specific target antigens for various diseases. The high density tissue specific antigens are suitable for targeting wih the first CAR which comprises an endodomain and optionally a co-stimulatory domain. The low density tumour specific antigens are suitable for targeting with the second CAR which comprises an activating endodomain.

| Disease | Tissue specific antigen | Tumour specific low density antigen |
|---|---|---|
| Multiple Myeloma | CD38, CD56, CD138 | BCMA |
| B-cell Acute Lymphoblastic Leukaemia | CD10 | CD22 |
| Chronic Lymphocytic Leukaemia | ROR1 | CD19 |
| Neuroblastoma | | ALK |
| T-cell acute Lymphoblastic Leukaema | CD2, CD5, CD7 | CD21 |

The second CAR may bind to one of the following target antigens: B cell maturation antigen (BCMA), trans-membrane activator and calcium modulator and cyclophilin ligand interactor (TACI), CD22, CD19, CD21 and Anaplastic Lymphoma Kinase (ALK).

The target antigen for the second CAR may be BCMA and/or TACI and the target antigen for the first CAR may be CD38, CD56 or CD138.

The target antigen for the second CAR may be CD22 and the target antigen for the first CAR may be CD10.

The target antigen for the second CAR may be CD19 and the target antigen for the first CAR may be ROR1.

The target antigen for the second CAR may be CD21 and the target antigen for the first CAR may be CD2, CD5 or CD7.

An antigen binding domain against CD38 may be derived from daratumumab. A suitable daratumumab scFv sequence is shown as SEQ ID No. 13 below.

SEQ ID No. 13 - Daratumumab scFv

EIVLTQSPATLSLSPGERATLSCRASQSVSSYLAWYQQKPGQAPRLLIYDASNRATGIPARFSG
SGSGTDFTLTISSLEPEDFAVYYCQQRSNWPPTFGQGTKVEIKRSGGGGSGGGGSGGGGSE
VQLLESGGGLVQPGGSLRLSCAVSGFTFNSFAMSWVRQAPGKGLEWVSAISGSGGGTYYAD
SVKGRFTISRDNSKNTLYLQMNSLRAEDTAVYFCAKDKILWFGEPVFDYWGQGTLVTVSS

SEQ ID No. 14 shows an anti-CD10 scFv sequence.

SEQ ID No. 14 - anti-CD10 scFv

DIVMTQSPDSLAVSLGERATINCSVSSSISSSNLHWYQQKPGQPPKLLIYGTSNLASGVPDRFS
GSGSGTDFTLTISSLQAEDVAVYYCQQWSSYPLTFGQGTKVEIKRSGGGGSGGGGSGGGGS
EVQLVESGGGVVQPGRSLRLSCAASGFTFSSFGMHWVRQAPGKGLEWVAYISGGSYTIYYAD
TVKGRFTISRDNSKNTLYLQMNSLRAEDTAVYYCARSYGNFWYFDVWGQGTTVTVSS

An antigen binding domain based on the ligand APRIL is capable of binding TACI and BCMA. A truncated APRIL sequence is shown as SEQ ID No. 15. Alternatively an anti-BCMA or anti-TACI scFv may be used. Suitable sequences are shown as SEQ ID No. 16 and 17 respectively.

SEQ ID No.15 - truncated APRIL

SVLHLVPINATSKDDSDVTEVMWQPALRRGRGLQAQGYGVRIQDAGVYLLYSQVLFQDVTFT

MGQVVSREGQGRQETLFRCIRSMPSHPDRAYNSCYSAGVFHLHQGDILSVIIPRARAKLNLSP

HGTFLGFVKL

SEQ ID No.16 - anti-BCMA scFv

DIVLTQSPPSLAMSLGKRATISCRASESVTILGSHLIHWYQQKPGQPPTLLIQLASNVQTGVPAR

FSGSGSRTDFTLTIDPVEEDDVAVYYCLQSRTIPRTFGGGTKLEIKGSTSGSGKPGSGEGSTKG

QIQLVQSGPELKKPGETVKISCKASGYTFTDYSINWVKRAPGKGLKWMGWINTETREPAYAYD

FRGRFAFSLETSASTAYLQINNLKYEDTATYFCALDYSYAMDYWGQGTSVTVSS

SEQ ID No. 17 - anti-TACI scFv

DIVMTQSQKFMSTTVGDRVSITCKASQNVGTAVAWYQQKPGQSPKLLIYSASNRYTGVPDRFT

GSGSGTDFTLTISNMQSEDLADYFCQQYSSYRTFGGGTKLEIKRSGGGGSGGGGSGGGGSQ

VTLKESGPGMLQPSQTLSLTCSFSGFSLSTFGMGVGWIRQPSGKGLEWLAHIWWDDAQYSNP

ALRSRLTISKDTSKNQVFLKIANVDTADTATYYCSRIHSYYSYDEGFAYWGQGTLVTVSS

SEQ ID No. 18 shows an anti-CD22 scFv sequence suitable for use as an antigen-binding domain.
SEQ ID No. 18 - anti-CD22 scFv

DVQVTQSPSSLSASVGDRVTITCRSSQSLANSYGNTFLSWYLHKPGKAPQLLIYGISNRFSGVP

DRFSGSGSGTDFTLTISSLQPEDFATYYCLQGTHQPYTFGQGTKVEIKRSGGGGSGGGGSGG

GGSEVQLVQSGAEVKKPGASVKVSCKASGYRFTNYWIHWVRQAPGQGLEWIGGINPGNNYA

TYRRKFQGRVTMTADTSTSTVYMELSSLRSEDTAVYYCTREGYGNYGAWFAYWGQGTLVTV

SS

Further anti-CD22 sequences for use as antigen-binding domains are shown below.

ANTIGEN SIZE/CONFIGURATION

The target antigen may comprise a long and/or bulky extracellular domain. Antigens with long and/or bulky extracellular domains are difficult to target with CAR T cells because they may inhibit a close-contact zone forming at the synapse, and permit entry of phosphorylase molecules to dephosphorylate key molecules in T cell signalling, thus inhibiting T cell activation.
Examples of antigens with long and/or bulky extracellular domains include MUC-1, MUC-16, CEACAM-15, CD21 and CD22 (see next section).

All of these antigens are tumour-associated antigens (TAAs). In this respect, MUC-1 is associated with multiple myeloma, ovarian cancer, colon cancer and prostate cancer; MUC-16 is associated with ovarian cancer; CEACAM-15 is associated with colon cancer; and CD22 is associated with lymphoid and myeloid hematological malignancies.

The relative distances at a T-cell:target cell synapse are illustrated schematically in Figure 9, in which:

X is the length of the extracellular domain of the antigen;
X1 is the length of the CAR; and
X' is the combined length of X and X1.

The ideal length for X' for CAR T-cell signalling is about 15nm.

A "long" antigen in the context of the present invention may be one having an extracellular domain which is greater than about 15, 20, 25 or 30nm in length.

CD22

In one embodiment of the invention, the first CAR and/or the second CAR bind to the antigen CD22. CD22 is a sugar binding transmembrane protein belonging to the SIGLEC family of lectins. It is found on the surface of mature B cells and on some immature B cells. CD22 is a regulatory molecule that prevents the overactivation of the immune system and the development of autoimmune diseases. The presence of Ig domains makes CD22 a member of the immunoglobulin superfamily. CD22 functions as an inhibitory receptor for B cell receptor (BCR) signaling.

Like CD19, CD22 is widely considered to be a pan-B antigen, although expression on some non-lymphoid tissue has been described. Targeting of CD22 with therapeutic monoclonal antibodies and immunoconjugates has entered clinical testing.

Examples of anti-CD22 CARs are described by Haso et al. (Blood; 2013; 121(7)). Specifically, anti-CD22 CARs with antigen-binding domains derived from m971, HA22 and BL22 scFvs are described. The first and/or second CAR of the cell of the present invention may comprise a CD22 binding domain based on one of these scFvs

CD22 has seven extracellular IgG-like domains, which are commonly identified as Ig domain 1 to Ig domain 7, with Ig domain 7 being most proximal to the B cell membrane and Ig domain 7 being the most distal from the Ig cell membrane.

The positions of the Ig domains in terms of the amino acid sequence of CD22 (http://www.uniprot.org/uniprot/P20273) are summarised in the following table:

| Ig domain | Amino acids |
|---|---|
| 7 | 20-138 |
| 6 | 143-235 |
| 5 | 242-326 |
| 4 | 331-416 |
| 3 | 419-500 |
| 2 | 505-582 |
| 1 | 593-676 |

Where the first and/or second CAR of the present invention bind CD22, it may bind to an epitope on a membrane-proximal Ig domain of CD22, such as Ig domain 5, 6 or 7. The anti-CD22 antibodies HA22 and BL22 (Haso et al 2013 as above), bind to an epitope on Ig domain 5 of CD22.

Alternatively it may bind to an epitope on a membrane-distal Ig domain of CD22, such as Ig domain 1, 2 or 3.

*CD22-antigen binding domain: derived from Inotuzumab (INO)*

Where the first and/or second bind CD22, the antigen binding domain which may be derived from Inotuzumab, which has a heavy chain variable region (VH) having complementarity determining regions (CDRs) with the following sequences:

CDR1 - NYWIH (SEQ ID No. 19);
CDR2 - GINPGNNYATYRRKFQG (SEQ ID No. 20)

CDR3 - EGYGNYGAWFAY (SEQ ID No. 21); and

a light chain variable region (VL) having CDRs with the following sequences:

CDR1 - RSSQSLANSYGNTFLS (SEQ ID No. 22);
CDR2 - GISNRFS (SEQ ID No. 23)
CDR3 - LQGTHQPYT (SEQ ID No. 24).

Inotuzumab, binds to an epitope on Ig domain 7 of CD22.

The antigen-binding domain may have one or more mutations (substitutions, additions or deletions) in one or more of the CDR sequences provided that the resultant molecule retains the capacity to bind CD22. For example, each CDR may comprise one, two or three mutations compared to the sequences given above. The mutations may be in CDR1 or 2, or the light chain CDRs, which are often less critical for antigen binding. The antigen binding domain may comprise the VH and/or VL from Inotuzumab, which are given above as SEQ ID Nos. 25 and 26 respectively or a variant thereof with, for example 80%, 90% or 95% identity which retains the capacity to bind CD22.

SEQ ID No. 25: VH sequence

EVQLVQSGAEVKKPGASVKVSCKASGYRFTNYWIHWVRQAPGQGLEWIGGINPGN 35
NYATYRRKFQGRVTMTADTSTSTVYMELSSLRSEDTAVYYCTREGYGNYGAWFAY
WGQGTLVTVSS

SEQ ID No. 26: VL sequence

DVQVTQSPSSLSASVGDRVTITCRSSQSLANSYGNTFLSWYLHKPGKAPQLLIYGIS
NRFSGVPDRFSGSGSGTDFTLTISSLQPEDFATYYCLQGTHQPYTFGQGTKVEIK

*CD22-antigen binding domain: CD22ALAb*

An anti-CD22 CAR which based on the CD22 binder CD22ALAb has improved properties compared to a known anti-CD22 CAR which comprises the binder m971 (WO2016/102965). The CD22 binder CD22ALAb has the CDRs and VH/VL regions identified below.

Where the first and/or second bind CD22, the antigen binding domain which may be derived from CD22ALAb, which has a heavy chain variable region (VH) having complementarity determining regions (CDRs) with the following sequences:

CDR1 - NYWIN (SEQ ID No. 27);
CDR2 - NIYPSDSFTNYNQKFKD (SEQ ID No. 28)
CDR3 - DTQERSWYFDV (SEQ ID No. 29); and

a light chain variable region (VL) having CDRs with the following sequences:

CDR1 - RSSQSLVHSNGNTYLH (SEQ ID No. 30);
CDR2 - KVSNRFS (SEQ ID No. 31)
CDR3 - SQSTHVPWT (SEQ ID No. 32).

It may be possible to introduce one or more mutations (substitutions, additions or deletions) into the or each CDR without negatively affecting CD22-binding activity. Each CDR may, for example, have one, two or three amino acid mutations.

The first and/or second CAR of the cell of the present invention may comprise one of the following amino acid sequences:

SEQ ID No. 33 (Murine CD22ALAb scFv sequence)

QVQLQQPGAELVRPGASVKLSCKASGYTFTNYWINWVKQRPGQGLEWIGNIYPSDSFTNYNQ
KFKDKATLTVDKSSSTAYMQLSSPTSEDSAVYYCTRDTQERSWYFDVWGAGTTVTVSS
DVVMTQTPLSLPVSLGDQASISCRSSQSLVHSNGNTYLHWYLQKPGQSPKLLIYKVSNRFSGV
PDRFSGSGSGTDFTLKISRVEAEDLGLYFCSQSTHVPWTFGGGTKLEIK

SEQ ID No. 34 (Humanised CD22ALAb scFv sequence)

EVQLVESGAEVKKPGSSVKVSCKASGYTFTNYWINWVRQAPGQGLEWIGNIYPSDSFTNYNQ
KFKDRATLTVDKSTSTAYLELRNLRSDDTAVYYCTRDTQERSWYFDVWGQGTLVTVSSDIVMT
QSPATLSVSPGERATLSCRSSQSLVHSNGNTYLHWYQQKPGQAPRLLIYKVSNRFSGVPARF
SGSGSGVEFTLTISSLQSEDFAVYYCSQSTHVPWTFGQGTRLEIK

The scFv may be in a VH-VL orientation (as shown in SEQ ID Nos 33 and 34) or a VL-VH orientation.
The first and/or second CAR of the cell of the present invention may comprise one of the following VH sequences:

SEQ ID No. 35 (Murine CD22ALAb VH sequence)

QVQLQQPGAELVRPGASVKLSCKASGYTFTNYWINWVKQRPGQGLEWIGNIYPSDSFTNYNQ
KFKDKATLTVDKSSSTAYMQLSSPTSEDSAVYYCTRDTQERSWYFDVWGAGTTVTVSS

SEQ ID No. 36 (Humanised CD22ALAb VH sequence)

EVQLVESGAEVKKPGSSVKVSCKASGYTFTNYWINWVRQAPGQGLEWIGNIYPSDSFTNYNQ
KFKDRATLTVDKSTSTAYLELRNLRSDDTAVYYCTRDTQERSWYFDVWGQGTLVTVSS

The first and/or second CAR of the cell of the present invention may comprise one of the following VL sequences:

SEQ ID No. 37 (Murine CD22ALAb VL sequence)

DVVMTQTPLSLPVSLGDQASISCRSSQSLVHSNGNTYLHWYLQKPGQSPKLLIYKVSNRFSGV
PDRFSGSGSGTDFTLKISRVEAEDLGLYFCSQSTHVPWTFGGGTKLEIK

SEQ ID No. 38 (Humanised CD22ALAb VL sequence)

DIVMTQSPATLSVSPGERATLSCRSSQSLVHSNGNTYLHWYQQKPGQAPRLLIYKVSNRFSGV
PARFSGSGSGVEFTLTISSLQSEDFAVYYCSQSTHVPWTFGQGTRLEIK

The first CAR and a second CAR of the cell of the present invention may comprise a variant of a sequence shown as SEQ ID No. 19, 20, 21, 22, 23, 24, 25, 26, 27, 28, 29, 30, 31, 32, 33, 34, 35, 36, 37, or 38, having at least 80, 85, 90, 95, 98 or 99% sequence identity, provided that the variant sequence of the first CAR and the second CAR retain the capacity to bind CD22 (when in conjunction with a complementary VL or VH domain, if appropriate) with either CD22 antigen binding domains derived from Inotuzumab or CD22ALAb.
The percentage identity between two polypeptide sequences may be readily determined by programs such as BLAST, which is freely available at http://blast.ncbi.nlm.nih.gov.
In one embodiment of the invention, the first CAR of the cell comprises the antigen binding domain derived from Inotuzumab, and the second CAR of the cell comprises an antigen binding domain CD22ALab.
In another embodiment, the first CAR of the cell comprises the antigen binding domain derived from CD22ALab and the second CAR of the cell comprises the antigen binding domain derived from Inotuzumab.
In an alternative embodiment, both the first CAR and the second CAR comprise an antigen binding domain derived from either Inomuzumab or the antigen binding domain CD22ALab.

Other anti-CD22 antibodies are known, such as the mouse anti-human CD22 antibodies 1D9-3, 3B4-13, 7G6-6, 6C4-6, 4D9-12, 5H4-9, 10C1-D9, 15G7-2, 2B12-8, 2C4-4 and 3E10-7. Table 2 summarises the, VH, VL and CDR sequences (in bold and underlined) and the position of the target epitope on CD22 for each antibody.

Table 2

| Antibody | VH | VL | Position of epitope on CD22 |
|---|---|---|---|
| 1D9-3 | EVQLVESGGGLVQPKGSLKLS CAASGFTFN**TYAMH**WVRQAP GKGLEWVA**RIRSKSSNYATYY ADSVKD**RFTISRDDSQSMLYL QMNNLKTEDTAMYYCVV**DYLY AMDY**WGQGTSVTVSS (SEQ ID No. 58) | DIVMTQSQKFMSTSVGDRV SITC**KASQNVRTAVA**WYQ QKPGQSPKALIY**LASNRHT** GVPDRFTGSGSGTDFTLTI SNVQSEDLADYFC**LQHWN YPFT**FGSGTKLEIK (SEQ ID No. 59) | Domain 1 and 2 |
| 3B4-13 | QVQLQQSGAELVRPGASVTLS CKASGYTFT**DYEMH**WVKQTP VHGLEWIG**AIDPETGATAYNQ KFKG**KAILTADKSSSTAYMDLR SLTSEDSAVYYCTR**YDYGSSP WFAY**WGQGTLVTVSA (SEQ ID No. 60) | QAVVTQESALTTSPGETVT LTC**RSSAGAVTTSNYAN**W VQEKPDHLFTGLIG**GTNNR AP**GVPARFSGSLIGDKAAL TITGAQTEDEAIYFC**ALWNS NHWV**FGGGTKLTVL (SEQ ID No. 61) | Domain 1 and 2 |
| 7G6-6 | QVQLQQPGAELVMPGASVKLS CKASGYTFT**SYWMH**WVKQRP GQGLEWIG**EIDPSDSYTNYNQ KFKG**KATLTVDKSSSTAYMQL SSLTSEDSAVYYCAR**GYYGSS SFDY**WGQGTTLTVSS (SEQ ID No. 62) | DIVMSQSPSSLAVSVGEKV TMSC**KSSQSLLYSSNQKN YLA**WYQQKPGQSPKLLIY**W ASTRES**GVPDRFTGSGSG TDFTLTISSVKAEDLAVYYC **QQYYSYT**FGGGTKLEIK (SEQ ID No. 63) | Domain 1 and 2 |
| 6C4-6 | QVQLKESGPGLVAPSQSLSITC TVSGFSLT**SYGVH**WVRQPPG KGLEWLV**VIWSDGSTTYNSAL KS**RLSISKDNSKSQVFLKMNSL QTDDTAMYYCAR**HADDYGFA WFAY**WGQGTLVTVSA (SEQ ID No. 64) | DIQMTQSPASLSASVGETV TITC**RASENIYSYLA**WYQQ KQGKSPQLLVY**NAKTLAE**G VPSRFSGSGSGTQFSLKIN SLQPEDFGSYYC**QHHYGT PPT**FGGGTKLEIK (SEQ ID No. 65) | Domain 3 |
| 4D9-12 | EFQLQQSGPELVKPGASVKIS CKASGYSFT**DYNMN**WVKQSN GKSLEWIG**VINPNYGTTSYNQ KFKG**KATLTVDQSSSTAYMQL NSLTSEDSAVYYCAR**SSTTVV DWYFDV**WGTGTTVTVSS (SEQ ID No. 66) | DIQMTQSPSSLSASLGERV SLTC**RASQEISGYLS**WLQQ KPDGTIKRLIY**AASTLDS**GV PKRFSGSRSGSDYSLTISSL ESEDFADYYC**LQYASYPFT** FGSGTKLEIK (SEQ ID No. 67) | Domain 4 |
| 5H4-9 | QVQVQQPGAELVRPGTSVKLS CKASGYTFT**RYWMY**WVKQRP GQGLEWIG**VIDPSDNFTYYNQ KFKG**KATLTVDTSSSTAYMQL SSLTSEDSAVYYCAR**GYGSSY VGY**WGQGTTLTVSS (SEQ ID No. 68) | DVVMTQTPLSLPVSLGDQA SISC**RSSQSLVHSNGNTYL H**WYLQKPGQSPKLLIY**KVS NRFS**GVPDRFSGSGSGTD FTLKISRVEAEDLGVYFC**SQ STHVPP**WTFGGGTKLEIK (SEQ ID No. 69) | Domain 4 |

(continued)

| Antibody | VH | VL | Position of epitope on CD22 |
|---|---|---|---|
| 10C1-D9 | QVTLKESGPGILQSSQTLSLTC SFSGFSLS**TSDMGVS**WIRQPS GKGLEWLA**HIYWDDDKRYNP SLKS**RLTISKDASRNQVFLKIAT VDTADTATYYCAR**SPWIYYGH YWCFDV**WGTGTTVTVSS (SEQ ID No. 70) | DIQMTQTTSSLSASLGDRV TISC**RASQDISNYLN**WYQQ KPDGTVKLLIY**YTSRLHS**GV PSRFSGSGSGTDYSLTISNL EQEDIATYFC**QQGNTLPFT** FGSGTKLEIK (SEQ ID No. 71) | Domain 4 |
| 15G7-2 | QVQLQQSGAELVKPGASVKLS CKASGYTFT**EYTIH**WVKQRSG QGLEWIG**WFYPGSGSIKYNEK FKD**KATLTADKSSSTVYMELS RLTSEDSAVYFCA**RHGDGYYL PPYYFDY**WGQGTTLTVSS (SEQ ID No. 72) | QIVLTQSPAIMSASPGEKVT MTC**SASSSVSYMY**WYQQK PGSSPRLLIY**DTSNLAS**GVP VRFSGSGSGTSYSLTISRM EAEDAATYYC**QQWSSYPL TF**GAGTKLELK (SEQ ID No. 73) | Domain 4 |
| 2B12-8 | QVQLQQSGAELARPGASVKLS CKASGYIFT**SYGIS**WVKQRTG QGLEWIG**EIYPRSGNTYYNEK FKG**KATLTADKSSSTAYMELR SLTSEDSAVYFCAR**PIYYGSRE GFDY**WGQGTTLTVSS (SEQ ID No. 74) | DIVLTQSPATLSVTPGDSVS LSC**RASQSISTNLH**WYQQK SHASPRLLIK**YASQSVS**GIP SRFSGSGSGTDFTLSINSV ETEDFGIFFC**QQSYSWPYT** FGGGTKLEIK (SEQ ID No. 75) | Domain 4 |
| 2C4-4 | QVQLQQPGAELVMPGASVKLS CKASGYTFT**SYWMH**WVKQRP GQGLEWIG**EIDPSDSYTNYNQ KFKG**KSTLTVDKSSSTAYIQLS SLTSEDSAVYYCAR**WASYRGY AMDY**WGQGTSVTVSS (SEQ ID No. 76) | DVLMTQTPLSLPVSLGDQA SISC**RSSQSIVHSNGNTYLE** WYLQKPGQSPKLLIY**KVSN RFS**GVPDRFSGSESGTDFT LKISRVEAEDLGVYYC**FQG SHVPWT**FGGGTKLEIK (SEQ ID No. 77) | Domain 5-7 |
| 3E10-7 | EFQLQQSGPELVKPGASVKIS CKASGYSFT**DYNMN**WVKQSN GKSLEWIG**VINPNYGTTSYNQ RFKG**KATLTVDQSSSTAYMQL NSLTSEDSAVYYCAR**SGLRYW YFDV**WGTGTTVTVSS (SEQ ID No. 78) | DIQMTQSPSSLSASLGERV SLTC**RASQEISGYLS**WLQQ KPDGTIKRLIY**AASTLDS**GV PKRFSGSRSGSDYSLTISSL ESEDFADYYC**LQYASYPFT** FGSGTKLEIK (SEQ ID No. 79) | Domain 5-7 |

An antigen binding domain of a chimeric receptor which binds to CD22 may comprise the VH and/or VL sequence from any of the CD22 antibodies listed in table 2, or a variant thereof which has at least 70, 80, 90 or 90% sequence identity, which variant retains the capacity to bind CD22.

SPACER DOMAIN

CARs comprise a spacer sequence to connect the antigen-binding domain with the transmembrane domain and spatially separate the antigen-binding domain from the endodomain. A flexible spacer allows the antigen-binding domain to orient in different directions to facilitate binding.

Where the cell of the present invention comprises two or more chimeric receptors, the spacers may be the same or different.

The spacer sequence may, for example, comprise an IgG1 Fc region, an IgG1 hinge or a CD8 stalk. The linker may alternatively comprise an alternative linker sequence which has similar length and/or domain spacing properties as an IgG1 Fc region, an IgG1 hinge or a CD8 stalk.

A human IgG1 spacer may be altered to remove Fc binding motifs.

Examples of amino acid sequences for these spacers are given below:

SEQ ID NO. 39 (hinge-CH2CH3 of human IgG1)

AEPKSPDKTHTCPPCPAPPVAGPSVFLFPPKPKDTLMIARTPEVTCVVVDVSHEDPEVKFNWY

VDGVEVHNAKTKPREEQYNSTYRVVSVLTVLHQDWLNGKEYKCKVSNKALPAPIEKTISKAKG

QPREPQVYTLPPSRDELTKNQVSLTCLVKGFYPSDIAVEWESNGQPENNYKTTPPVLDSDGSF

FLYSKLTVDKSRWQQGNVFSCSVMHEALHNHYTQKSLSLSPGKKD

SEQ ID NO. 40 (human CD8 stalk):
TTTPAPRPPTPAPTIASQPLSLRPEACRPAAGGAVHTRGLDFACDI

SEQ ID NO. 41 (human IgG1 hinge):
AEPKSPDKTHTCPPCPKDPK

The spacer may be monomeric or dimeric. Monomeric spacers may be generated, for example, by mutation of the cysteine residue(s) responsible for disulphide bond formation (Bridgeman et al., 2010 J. Immunol. 184:6938-6949).

Where the first CAR and second CAR bind the same antigen or similar sized antigens, the spacer of the first CAR may be sufficiently different from the spacer of the second CAR in order to avoid cross-pairing but sufficiently similar to co-localise. Pairs of orthologous spacer sequences may be employed. Examples are murine and human CD8 stalks, or alternatively spacer domains which are monomeric - for instance the ectodomain of CD2.

Examples of spacer pairs which co-localise are shown in the following Table 2:

Table 2

| Stimulatory CAR spacer | Inhibitory CAR spacer |
| --- | --- |
| Human-CD8aSTK | Mouse CD8aSTK |
| Human-CD28STK | Mouse CD8aSTK |
| Human-IgG-Hinge | Human-CD3z ectodomain |
| Human-CD8aSTK | Mouse CD28STK |
| Human-CD28STK | Mouse CD28STK |
| Human-IgG-Hinge-CH2CH3 | Human-IgM-Hinge-CH2CH3CD4 |

Where the first and second CAR bind to different antigens, spacers may be chosen which give an approximately equal synapse distance between the target cell and the T-cell in the synapse.

The length of the spacers of the two CARs may be chosen such that upon binding the target cell, the intermolecular distance of:

$$(X+X1) \approx (Y+Y1)$$

where:

X is the distance from the target cell membrane to the epitope on the first antigen;
X1 is the length of the first CAR;
Y is the distance from the target cell membrane to the epitope on the second antigen;
Y1 is the length of the second CAR.

TRANSMEMBRANE DOMAIN

The transmembrane domain is the sequence of the CAR that spans the membrane. A transmembrane domain may be any protein structure which is thermodynamically stable in a membrane. This is typically an alpha helix comprising of several hydrophobic residues. The transmembrane domain of any transmembrane protein can be used to supply the transmembrane portion of the invention. The presence and span of a transmembrane domain of a protein can be determined by those skilled in the art using the TMHMM algorithm (http://www.cbs.dtu.dk/services/TMHMM-2.0/). Further, given that the transmembrane domain of a protein is a relatively simple structure, i.e a polypeptide sequence predicted to form a hydrophobic alpha helix of sufficient length to span the membrane, an artificially designed TM domain may also be used (US 7052906 B1 describes synthetic transmembrane components).

The transmembrane domain may, for example, be derived from CD28, which gives good receptor stability.

ACTIVATING ENDODOMAIN

The activating endodomain is the signal-transmission portion of a classical CAR. After antigen recognition by the antigen binding domain, individual CAR molecules cluster, native CD45 and CD148 are excluded from the synapse and a signal is transmitted to the cell.

The activating endodomain of the second CAR of the cell of the first aspect of the invention may comprise intracellular signalling domain. In an alternative embodiment, the activating endodomain of the present CAR may be capable of interacting with an intracellular signalling molecule which is present in the cytoplasm, leading to signalling.

The intracellular signalling domain or separate intracellular signalling molecule may be or comprise a T cell signalling domain.

The activating endodomain comprises one or more immunoreceptor tyrosine-based activation motifs (ITAMs). An ITAM is a conserved sequence of four amino acids that is repeated twice in the cytoplasmic tails of certain cell surface proteins of the immune system. The motif contains a tyrosine separated from a leucine or isoleucine by any two other amino acids, giving the signature YxxL/I. Two of these signatures are typically separated by between 6 and 8 amino acids in the tail of the molecule (YxxL/Ix$_{(6-8)}$YxxL/I).

ITAMs are important for signal transduction in immune cells. Hence, they are found in the tails of important cell signaling molecules such as the CD3 and ζ-chains of the T cell receptor complex, the CD79 alpha and beta chains of the B cell receptor complex, and certain Fc receptors. The tyrosine residues within these motifs become phosphorylated following interaction of the receptor molecules with their ligands and form docking sites for other proteins involved in the signaling pathways of the cell.

The most commonly used signalling domain component is that of CD3-ζ endodomain, which contains three ITAMs. This transmits an activation signal to the T cell after antigen is bound. CD3-ζ may not provide a fully competent activation signal and additional co-stimulatory signalling may be needed. For example, 4-1BB (also known as CD137) can be used with CD3-ζ, or CD28 and OX40 can be used with CD3-ζ to transmit a proliferative / survival signal.

The activating endodomain of the second CAR of the cell of the first aspect of the invention may comprise the CD3-ζ endodomain alone, the CD3-ζ endodomain in combination with one or more co-stimulatory domains selected from 4-1BB, CD28 or OX40 endodomain, and/or a combination of some or all of 4-1BB, CD28 or OX40.

The endodomain may comprise one or more of the following: an ICOS endodomain, a CD27 endodomain, a BTLA endodomain, a CD30 endodomain, a GITR endodomain and an HVEM endodomain.

The endodomain may comprise the sequence shown as SEQ ID NO 42 to 45 or a variant thereof having at least 80% sequence identity.

SEQ ID NO 42 - CD3-ζ endodomain

RVKFSRSADAPAYQQGQNQLYNELNLGRREEYDVLDKRRGRDPEMGGKPRRKNPQEGLYNE
LQKDKMAEAYSEIGMKGERRRGKGHDGLYQGLSTATKDTYDALHMQALPPR

SEQ ID NO 43 - 4-1BB and CD3-ζ endodomains

MGNSCYNIVATLLLVLNFERTRSLQDPCSNCPAGTFCDNNRNQICSPCPPNSFSSAGGQRTCD
ICRQCKGVFRTRKECSSTSNAECDCTPGFHCLGAGCSMCEQDCKQGQELTKKGCKDCCFGT
FNDQKRGICRPWTNCSLDGKSVLVNGTKERDVVCGPSPADLSPGASSVTPPAPAREPGHSPQ
IISFFLALTSTALLFLLFFLTLRFSVVKRGRKKLLYIFKQPFMRPVQTTQEEDGCSCRFPEEEEGG
CELRVKFSRSADAPAYQQGQNQLYNELNLGRREEYDVLDKRRGRDPEMGGKPQRRKNPQE
GLYNELQKDKMA EAYSEIGMKGERRRGKGHDGLYQGLSTATKDTYDALHMQALPPR

SEQ ID NO 44 - CD28 and CD3-ζ endodomains

SKRSRLLHSDYMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAYSEIGMKGERRR
GKGHDGLYQGLSTATKDTYDALHMQALPPR

SEQ ID NO 45 - CD28, OX40 and CD3-ζ endodomains

SKRSRLLHSDYMNMTPRRPGPTRKHYQPYAPPRDFAAYRSRDQRLPPDAHKPPGGGSFRTPI
QEEQADAHSTLAKIRVKFSRSADAPAYQQGQNQLYNELNLGRREEYDVLDKRRGRDPEMGG
KPRRKNPQEGLYNELQKDKMAEAYSEIGMKGERRRGKGHDGLYQGLSTATKDTYDALHMQA
LPPR

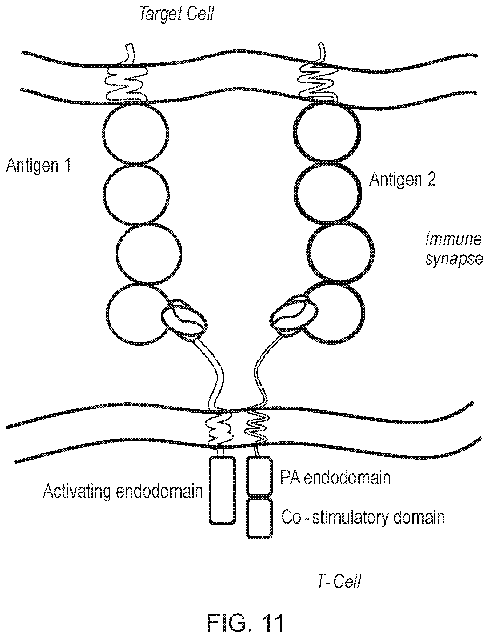

The endodomain of the first CAR of the present invention may comprise the tyrosine kinase domain of a Src family kinase or the intracellular domain of CD4 or CD8 coreceptor and a co-stimulatory domain, for example as illustrated in Figure 11. The co-stimulatory domain may, for example, be selected from one of the following endodomains: CD28, 4-1BB, OX40, ICOS, CD27, BTLA, CD30, GITR and HVEM.

NUCLEIC ACID CONSTRUCT

In one aspect, the present invention provides a nucleic acid construct which comprises two or more nucleic acid sequences. The nucleic acid construct comprises a first nucleic acid sequence encoding a first CAR and a second nucleic acid sequence encoding a second CAR according to the first aspect of the invention.
For example, the nucleic acid construct may comprise:

(a) a first nucleic acid sequence encoding an antigen-binding domain, a spacer domain, a transmembrane domain and an endodomain of the first CAR and
(b) a second nucleic acid sequence encoding an antigen-binding domain, a spacer domain, a transmembrane domain, and an activating endodomain of the second CAR.

The nucleic acid construct may have the following structure:
AgB1-spacer1-TM1-endo1-coexpr-AbB2-spacer2-TM2-endo2

in which, AgB1 is a nucleic acid sequence encoding an antigen-binding domain of the first CAR;
spacer 1 is a nucleic acid sequence encoding a spacer of the first CAR;
TM1 is a nucleic acid sequence encoding a transmembrane domain of the first CAR;
endo1 is a nucleic acid sequence encoding the endodomain of the first CAR;
coexpr is a nucleic acid sequence enabling co-expression of both CAR;
AgB2 is a nucleic acid sequence encoding an antigen-binding domain of the second CAR;
spacer 2 is a nucleic acid sequence encoding a spacer of the second CAR;
TM2 is a nucleic acid sequence encoding a transmembrane domain of the second CAR;
Endo2 is a nucleic acid sequence encoding the activating endodomain of the second CAR;

in which nucleic acid construct, when expressed in a T cell, encodes a polypeptide which is cleaved at the cleavage site such that the first and second CARs are co-expressed at the T cell surface.

Sequences encoding the first and second CARs may be in either order on the nucleic acid construct.

The activating endodomain may comprise an intracellular cell signalling domain or may associate intracellularly with a separate cell signalling domain.

The first CAR may comprise a co-stimulatory domain in addition to the endodomain according to the first aspect of the invention.

In the structure above, "coexpr" is a nucleic acid sequence enabling co-expression of both first and second CARs. It may be a sequence encoding a cleavage site, such that the nucleic acid construct produces comprises two or more CARs joined by a cleavage site(s). The cleavage site may be self-cleaving, such that when the polypeptide is produced, it is immediately cleaved into individual peptides without the need for any external cleavage activity.

The cleavage site may be any sequence which enables the first and second CARs to become separated.

The term "cleavage" is used herein for convenience, but the cleavage site may cause the peptides to separate into individual entities by a mechanism other than classical cleavage. For example, for the Foot-and-Mouth disease virus (FMDV) 2A self-cleaving peptide (see below), various models have been proposed for to account for the "cleavage" activity: proteolysis by a host-cell proteinase, autoproteolysis or a translational effect (Donnelly et al (2001) J. Gen. Virol. 82:1027-1041). The exact mechanism of such "cleavage" is not important for the purposes of the present invention, as long as the cleavage site, when positioned between nucleic acid sequences which encode proteins, causes the proteins to be expressed as separate entities.

The cleavage site may be a furin cleavage site.

Furin is an enzyme which belongs to the subtilisin-like proprotein convertase family. The members of this family are proprotein convertases that process latent precursor proteins into their biologically active products. Furin is a calcium-dependent serine endoprotease that can efficiently cleave precursor proteins at their paired basic amino acid processing sites. Examples of furin substrates include proparathyroid hormone, transforming growth factor beta 1 precursor, proalbumin, pro-beta-secretase, membrane type-1 matrix metalloproteinase, beta subunit of pro-nerve growth factor and von Willebrand factor. Furin cleaves proteins just downstream of a basic amino acid target sequence (canonically, Arg-X-(Arg/Lys)-Arg') and is enriched in the Golgi apparatus.

The cleavage site may be a Tobacco Etch Virus (TEV) cleavage site.

TEV protease is a highly sequence-specific cysteine protease which is chymotrypsin-like proteases. It is very specific for its target cleavage site and is therefore frequently used for the controlled cleavage of fusion proteins both *in vitro* and *in vivo*. The consensus TEV cleavage site is ENLYFQ\S (where '\' denotes the cleaved peptide bond). Mammalian cells, such as human cells, do not express TEV protease. Thus in embodiments in which the present nucleic acid construct comprises a TEV cleavage site and is expressed in a mammalian cell - exogenous TEV protease must also expressed in the mammalian cell.

The cleavage site may encode a self-cleaving peptide.

A 'self-cleaving peptide' refers to a peptide which functions such that when the polypeptide comprising the proteins and the self-cleaving peptide is produced, it is immediately "cleaved" or separated into distinct and discrete first and second polypeptides without the need for any external cleavage activity.

The self-cleaving peptide may be a 2A self-cleaving peptide from an aphtho- or a cardiovirus. The primary 2A/2B cleavage of the aptho- and cardioviruses is mediated by 2A "cleaving" at its own C-terminus. In apthoviruses, such as foot-and-mouth disease viruses (FMDV) and equine rhinitis A virus, the 2A region is a short section of about 18 amino acids, which, together with the N-terminal residue of protein 2B (a conserved proline residue) represents an autonomous element capable of mediating "cleavage" at its own C-terminus (Donely et al (2001) as above).

"2A-like" sequences have been found in picornaviruses other than aptho- or cardioviruses, 'picornavirus-like' insect viruses, type C rotaviruses and repeated sequences within Trypanosoma spp and a bacterial sequence (Donnelly et al., 2001) as above. The cleavage site may comprise one of these 2A-like sequences, such as:

    YHADYYKQRLIHDVEMNPGP (SEQ ID NO 46)
    HYAGYFADLLIHDIETNPGP (SEQ ID NO 47)
    QCTNYALLKLAGDVESNPGP (SEQ ID NO 48)
    ATNFSLLKQAGDVEENPGP (SEQ ID NO 49)
    AARQMLLLLSGDVETNPGP (SEQ ID NO 50)
    RAEGRGSLLTCGDVEENPGP (SEQ ID NO 51)
    TRAEIEDELIRAGIESNPGP (SEQ ID NO 52)
    TRAEIEDELIRADIESNPGP (SEQ ID NO 53)
    AKFQIDKILISGDVELNPGP (SEQ ID NO 54)
    SSIIRTKMLVSGDVEENPGP (SEQ ID NO 55)
    CDAQRQKLLLSGDIEQNPGP (SEQ ID NO 56)

YPIDFGGFLVKADSEFNPGP (SEQ ID NO 57)

The cleavage site may comprise the 2A-like sequence shown as SEQ ID NO 51 (RAEGRGSLLTCGDVEENPGP).

The co-expressing sequence may be an internal ribosome entry sequence (IRES). The co-expressing sequence may be an internal promoter.

The present invention also provides a kit comprising one or more nucleic acid sequence(s) encoding first and second CARs as defined above.

VECTOR

The present invention also provides a vector, or kit of vectors, which comprises one or more nucleic acid sequence(s) encoding a first and a second CAR as defined above. Such a vector may be used to introduce the nucleic acid sequence(s) or construct into a host cell so that it expresses a first and a second CAR.

The vector may, for example, be a plasmid or a viral vector, such as a retroviral vector or a lentiviral vector, or a transposon based vector or synthetic mRNA.

The vector may be capable of transfecting or transducing a T cell or a NK cell.

CELL

The present invention provides a cell which comprises a first chimeric antigen receptor (CAR) and a second CAR according to the first aspect of the invention.

The cell may comprise a nucleic acid sequence or construct or a vector of the present invention.

The cell may be a cytolytic immune cell such as a T cell or an NK cell.

T cells or T lymphocytes are a type of lymphocyte that play a central role in cell-mediated immunity. They can be distinguished from other lymphocytes, such as B cells and natural killer cells (NK cells), by the presence of a T-cell receptor (TCR) on the cell surface. There are various types of T cell, as summarised below.

Helper T helper cells (TH cells) assist other white blood cells in immunologic processes, including maturation of B cells into plasma cells and memory B cells, and activation of cytotoxic T cells and macrophages. TH cells express CD4 on their surface. TH cells become activated when they are presented with peptide antigens by MHC class II molecules on the surface of antigen presenting cells (APCs). These cells can differentiate into one of several subtypes, including TH1, TH2, TH3, TH17, Th9, or TFH, which secrete different cytokines to facilitate different types of immune responses.

Cytolytic T cells (TC cells, or CTLs) destroy virally infected cells and tumor cells, and are also implicated in transplant rejection. CTLs express the CD8 at their surface. These cells recognize their targets by binding to antigen associated with MHC class I, which is present on the surface of all nucleated cells. Through IL-10, adenosine and other molecules secreted by regulatory T cells, the CD8+ cells can be inactivated to an anergic state, which prevent autoimmune diseases such as experimental autoimmune encephalomyelitis.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory T cells comprise three subtypes: central memory T cells (TCM cells) and two types of effector memory T cells (TEM cells and TEMRA cells). Memory cells may be either CD4+ or CD8+. Memory T cells typically express the cell surface protein CD45RO.

Regulatory T cells (Treg cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Two major classes of CD4+ Treg cells have been described - naturally occurring Treg cells and adaptive Treg cells.

Naturally occurring Treg cells (also known as CD4+CD25+FoxP3+ Treg cells) arise in the thymus and have been linked to interactions between developing T cells with both myeloid (CD11c+) and plasmacytoid (CD123+) dendritic cells that have been activated with TSLP. Naturally occurring Treg cells can be distinguished from other T cells by the presence of an intracellular molecule called FoxP3. Mutations of the FOXP3 gene can prevent regulatory T cell development, causing the fatal autoimmune disease IPEX.

Adaptive Treg cells (also known as Tr1 cells or Th3 cells) may originate during a normal immune response.

The cell may be a Natural Killer cell (or NK cell). NK cells form part of the innate immune system. NK cells provide rapid responses to innate signals from virally infected cells in an MHC independent manner

NK cells (belonging to the group of innate lymphoid cells) are defined as large granular lymphocytes (LGL) and constitute the third kind of cells differentiated from the common lymphoid progenitor generating B and T lymphocytes.

NK cells are known to differentiate and mature in the bone marrow, lymph node, spleen, tonsils and thymus where they then enter into the circulation.

The CAR cells of the invention may be any of the cell types mentioned above.

T or NK cells according to the first aspect of the invention may either be created *ex vivo* either from a patient's own peripheral blood (1st party), or in the setting of a haematopoietic stem cell transplant from donor peripheral blood (2nd party), or peripheral blood from an unconnected donor (3rd party).

Alternatively, T or NK cells according to the first aspect of the invention may be derived from *ex vivo* differentiation of inducible progenitor cells or embryonic progenitor cells to T or NK cells. Alternatively, an immortalized T-cell line which retains its lytic function and could act as a therapeutic may be used.

In all these embodiments, CAR cells are generated by introducing DNA or RNA coding for first and second CARs by one of many means including transduction with a viral vector, transfection with DNA or RNA.

The cell of the present invention may be an *ex vivo* cell from a subject. The T (or NK) cell may be from a peripheral blood mononuclear cell (PBMC) sample. T or NK cells may be activated and/or expanded prior to being transduced with nucleic acid encoding the molecules providing the first or second CAR according to the first aspect of the invention.

The cell of the invention may be made by: (i) isolation of a cell-containing sample from a subject or other sources listed above; and (ii) transduction or transfection of the cells with one or more a nucleic acid sequence(s) encoding first and second CARs.

The cells may then by purified, for example, selected on the basis of expression of the antigen-binding domain of the antigen-binding polypeptide.

PHARMACEUTICAL COMPOSITION

The present invention also relates to a pharmaceutical composition containing a plurality of cells of the invention.

The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

METHOD OF TREATMENT

Described herein is a method for treating and/or preventing a disease which comprises the step of administering the cells of the present invention (for example in a pharmaceutical composition as described above) to a subject.

A method for treating a disease relates to the therapeutic use of the cells of the present invention. The cells may be administered to a subject having an existing disease or condition in order to lessen, reduce or improve at least one symptom associated with the disease and/or to slow down, reduce or block the progression of the disease.

The method for preventing a disease relates to the prophylactic use of the cells of the present invention. In this respect, the cells may be administered to a subject who has not yet contracted the disease and/or who is not showing any symptoms of the disease to prevent or impair the cause of the disease or to reduce or prevent development of at least one symptom associated with the disease. The subject may have a predisposition for, or be thought to be at risk of developing, the disease.

The method may involve the steps of:

(i) isolating a cell-containing sample from a subject;
(ii) transducing or transfecting such cells with a nucleic acid construct or first and second nucleic acid sequence or first and second vector, or vector provided by the present invention;
(iii) administering the cells from (ii) to a subject.

The cell-containing sample may be isolated from a subject or from other sources, for example as described above. The T or NK cells may be isolated from a subject's own peripheral blood (1st party), or in the setting of a haematopoietic stem cell transplant from donor peripheral blood (2nd party), or peripheral blood from an unconnected donor (3rd party).

The present invention provides a cell coexpressing a first CAR and a second CAR of the present invention for use in treating a disease.

Described herein is the use of a cell coexpressing a first CAR and a second CAR of the present invention in the manufacture of a medicament for the treatment and/or prevention of a disease.

The disease to be treated may be a cancerous disease, such as bladder cancer, breast cancer, colon cancer, endometrial cancer, kidney cancer (renal cell), leukaemia, lung cancer, melanoma, non-Hodgkin lymphoma, pancreatic cancer, prostate cancer and thyroid cancer.

The cells of the present invention may be capable of killing target cells, such as cancer cells. The target cell may be a characterised by the presence of a cell-surface antigen (or cell-surface ligand) present on the surface of the cell. The target cell may express a high level of one or more inhibitory receptors, such as PDL1. Where a target cell expresses a high level of an inhibitory receptor such as PDL1, recognition by a T-cell would recruit inhibitory phosphatases such as PD1 to the T-cell:target-cell synapse. In these circumstances it is beneficial to increase the amount of kinase (i.e. amplify phosphorylation) in order to allow T-cell activation to occur.

An inhibitory receptor may contain one or more immunoreceptor tyrosine-based inhibitory motifs (ITIMs) and, when phosphorylated, may recruit SHP1 and/or SHP2. In NK cells, inhibitory receptors include member of the immunoglobulin superfamily and the c-type lectin superfamily. In T cells, inhibitory receptors include programed cell death 1 (PD1) and cytotoxic T lymphocyte-associated Antigen 4 (CTLA-4), IM3, LAG3, CD160, BTLA, and 2B4.

Where the target cell is a malignant cell, it may express the inhibitory receptor at a level greater than the mean level of expression from an equivalent non-malignant cell.

The target cell may express the inhibitory receptor at a level which would mean that a cell expressing a classical CAR is not significantly activated upon recognition of the target cell, but a cell expressing a CAR system of the cell of the present invention (which comprises a CAR with an endodomain of the present invention) is activated upon recognition of the target cell.

The cells and pharmaceutical compositions of present invention may be for use in the treatment of the diseases described above.

The cells and pharmaceutical compositions of present invention may be for use in any of the methods described above.

FURTHER ASPECTS OF THE DISCLOSURE

Described herein is a chimeric antigen receptor (CAR) comprising:

    a. an antigen binding domain,
    b. a transmembrane domain,
    c. a phosphorylation amplifying domain,
    d. and an activating endodomain,

wherein the phosphorylation amplifying domain comprises a tyrosine kinase domain of Fyn.

The activating endodomain of the CAR described herein may comprise CD3-$\zeta$.

The activating endodomain of the CAR described herein may comprise CD3-$\zeta$ and one or more co-stimulatory domain(s) 4-1BB, CD28 and/ or OX40.

The activating endodomain of the CAR described herein may comprise CD3-$\zeta$ and the co-stimulatory domain 4-1 BB.

The antigen binding domain of the CAR described herein may bind to any antigen associated with a disease phenotype, preferably selected from the tumour-associated antigens disclosed in table 1.

The antigen binding domain of the CAR described herein may bind to CD22.

The antigen binding domain of the CAR described herein may bind to ROR1.

Described herein is a nucleic acid sequence encoding the CAR as described herein. The nucleic acid sequence may have the following structure:

    AgB-spacer-TM-endo-Pa
    or
    AgB-spacer-TM-Pa-endo

in which

    AgB is a nucleic acid sequence encoding an antigen-binding domain of the CAR;
    Spacer is a nucleic acid sequence encoding a spacer of the CAR;
    TM is a nucleic acid sequence encoding a transmembrane domain of the CAR;
    Endo is a nucleic acid sequence encoding the activating endodomain of the CAR;
    Pa is a nucleic acid sequence encoding the phosphorylation amplifying domain of the CAR;

wherein the Pa comprises a tyrosine kinase domain of Fyn; and
which nucleic acid sequence encodes a polypeptide which is then expressed at a cell surface.

Described here is a vector comprising the nucleic acid sequence as described herein.

The vector may, for example, be a retroviral vector or a lentiviral vector or a transposon.

Described here is a cell comprising a CAR as described herein.

Described herein is a pharmaceutical composition comprising a plurality of cells as described herein.

Described herein is a method for treating and/or preventing a disease, which comprises the step of administering a pharmaceutical composition as described herein to a subject.

The method may comprise the following steps:

(i) isolation of a cell-containing sample from a subject;

(ii) transduction or transfection of the cells with the nucleic acid sequence encoding the CAR as described herein or the vector described herein; and

(iii) administering the cells from (ii) a/ to the subject.

The disease may be cancer. The cancer may be a B cell malignancy.

Described herein is a pharmaceutical composition as described herein for use in treating and/or preventing a disease.

Described herein is the use of a CAR as described herein in the manufacture of a medicament for treating and/or preventing a disease.

The above paragraphs relating to the cells, nucleic acids, vectors, kits, compositions and methods of the first to eighth aspects of the invention, and components thereof, also apply to the corresponding components as described herein.

The invention will now be described by way of Examples, which are meant to serve to assist one of ordinary skill in the art in carrying out the invention and are not intended in any way to limit the scope of the invention.

EXAMPLES

A series of read-outs were measured to evaluate the sensitivity of various CAR constructs to a target antigen. In particular, cytotoxicity, proliferation and cytokine production were measured.

Example 1 - FACs-based killing (FBK)

A panel of CARs was created with and without phosphorylation amplifying endodomains and their cytotoxic capability was compared. The CAR system tested comprised a first CAR comprising an CD22 antigen binding domain derived Inotuzumab (INO) and a second CAR with an CD22ALAb antigen binding domain CAR (Figure 4). The Inotuzumab scFv tested was the clone g5/44. The phosphorylation amplifying CARs tested comprised the Inotuzumab scFv, a CD8stalk spacer, a transmembrane domain, and the intracellular domain comprising a phosphorylating amplifying endodomain comprising the tyrosine kinase domain of Fyn, Src, Lck or Lck mutant Y505F or an intracellular domain of CD4 or CD8 coreceptor. The activating CARs tested, as shown in Figure 4, comprised a hinge spacer, a transmembrane domain and an activating endodomain comprising CD3ζ and 4-1BB.

Seven days after the thawing of PBMCs, the culture was depleted of CD56 NK cells to reduce background cytotoxicity. On the eighth day, the T-cells were co-cultured with the target cells at a ratio 1:1. The assay was carried out in a 96-well plate in 0.2 ml total volume using $5 \times 10^4$ transduced T-cells per well and an equal number of target cells. The co-cultures are set up after being normalised for the transduction efficiency. The FBK was carried out after 72h of incubation.

The results of the FBK are shown in Figure 5. It is clear the cells co-expressing a first CAR comprising a phosphorylating amplifying (Pa) endodomain with a second CAR comprising an activating endodomain are superior. For example, the non-transduced cells, the EGFRvIII or the CD22ALAb-Hinge CAR, which lack a phosphorylating amplifying (Pa) endodomain, show significantly higher overall cell survival than those CAR constructs comprising a phosphorylating amplifying (Pa) endodomain.

The results show that a cell coexpressing a first CAR comprising a Fyn or Lck (Y505F mutant) phosphorylating amplifying domain achieved the best FACs based killing where on average the least percentage of Raji cells survived.

Example 2 - Proliferation assay (PA)

Proliferation is a key feature of CAR-mediated responses which is measured to test the efficacy of a CAR alongside cytotoxicity and cytokine secretion. Although 1st generation CARs display good levels of cytotoxicity, they do not display good proliferative responses *in vitro* and fail to persist well *in vivo*. Proliferation is enhanced by the inclusion of co-stimulatory domains such as CD28, OX40 or 4-1BB into the CAR endodomain.

In order to measure proliferation, CD56-depleted, the same panel of CAR-expressing T cells described in Example 1 were labelled with the dye Cell Trace Violet (CTV), a fluorescent dye which is hydrolysed and retained within the cell. It is excited by the 405nm (violet) laser and fluorescence can be detected in the pacific blue channel. The CTV dye was reconstituted to 5mM in DMSO. The T-cells were resuspended at $2x10^6$ cells per ml in PBS, and 1ul/ml of CTV was added. The T-cells were incubated the CTV for 20 minutes at 37°C. Subsequently, the cells were quenched by adding 5V of complete media. After a 5 minutes incubation, the T-cells were washed and resuspended in 2ml of complete media. An additional 10 minute incubation at room temperature allowed the occurrence of acetate hydrolysis and retention of the dye.

Labelled T-cells were co-cultured with antigen-expressing or antigen-negative target cells for seven days. The assay was carried out in a 96-well plate in 0.2 ml total volume using $5x10^4$ transduced T-cells per well and an equal number of target cells (ratio 1:1). At the day seven time point, the T-cells were analysed by flow cytometry to measure the dilution of the CTV which occurs as the T-cells divide. The number of T-cells present at the end of the co-culture was calculated, and expressed as a fold of proliferation compared to the input number of T cells.

Figure 6 shows that CAR constructs comprising a phosphorylating amplifying endodomain demonstrate increased proliferation compared to constructs lacking the phosphorylating amplifying endodomain. In particular, the CAR construct comprising the CD4 or the Fyn phosphorylating amplifying domain show the largest number of T-cells present at the end of the co-culture: the area under the curve in both CD4 and Fyn constructs have shifted furthest along the X-axis compared to the other constructs.

Figure 6 shows that there was less persistence of the CAR T cell construct without a phosphorylation amplifying domain where the area under the curve is less elongated across the cell trace count line than the results for CAR T cell constructs comprising the phosphorylating amplifying domain.

Example 3 - Cytokine bead array (CBA)

Typically, immune cells detect major histocompatibility complex (MHC) presented on infected cell surfaces, triggering cytokine release, causing lysis or apoptosis. Cytokine production by CAR T cells can activate host immunity and represent a key element as to why these effector cells are successful. Cytokines such as IFN-$\gamma$ and IL-2 from CAR cells also recruit and activate a variety of host immune cells to modulate the tumour microenvironment and disrupt tumour growth. Therefore to test the effectivity of the CAR constructs the inventors also chose to compare cytokine production.

The panel of CAR constructs described in Example 1 were compared for IFN-$\gamma$ secretion (Figure 7a) and IL-2 secretion (Figure 7b) after 72 hours co-culture with Raji target cells. Increased cytokine production was observed in the CAR constructs comprising a phosphorylating amplifying domain compared to constructs lacking a phosphorylating amplification domain. It was observed that constructs comprising CD4, CD8 coreceptors phosphorylating amplifying domains or constructs comprising Fyn phosphorylating amplifying domains presented greater cytokine production (pg/ml) than constructs comprising Lck or Lck mutant phosphorylating amplifying domains in this system.

Example 4 - Expression of BCMA on surface of myeloma cells

Primary myeloma cells were isolated by performing a CD138 immunomagnetic selection on fresh bone marrow samples from Multiple myeloma patients that were known to have frank disease. These cells were stained with the BCMA specific J6MO mAb (GSK) which was conjugated to PE. At the same time, a standard of beads with known numbers of binding sites was generated using the PE Quantibrite bead kit (Becton Dickenson) as per the manufacturer's instructions. The BCMA copy number on myeloma cells was derived by correlating the mean-fluorescent intensity from the myeloma cells with the standard curve derived from the beads. It was found that the range of BCMA copy number on a myeloma cell surface is low: at 348.7-4268.4 BCMA copies per cell with a mean of 1181 and a median of 1084.9 (Figure 8). This is considerably lower than e.g. CD19 and GD2, classic targets for CARs.

Example 5 - Investigating killing and T-cell proliferation in response to target cells expressing low density target antigen

SupT1 target cells were created expressing varying levels of the target antigen CD22 at the following average antigen densities: 0, 255, 441, 634, 1090 and 78,916 copies per cell.

A panel of CARs was created and their cytotoxic capability was compared. The CAR system tested comprised; and a first CAR with an CD22ALAb antigen binding domain. The first CAR comprised a CD8 or a coiled-coil (COMP) spacer and optionally the intracellular domain of the CD4 coreceptor as phosphorylation amplifying domain. The CAR system also comprised a second CAR (activating CAR) comprising an CD22 antigen binding domain derived from the binder 10C1 and a compound 4-1BBzeta intracellular signalling domain.

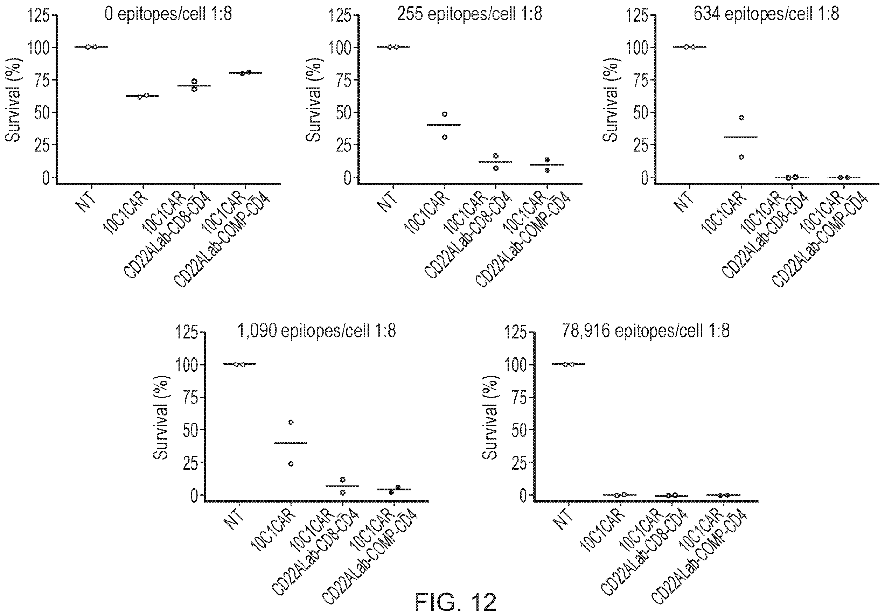

T-cells expressing these CARs were co-cultured with target cells at a 1:8 ratio for 72 hours and killing of target cells was investigated as described above. The results are shown in Figure 12. T cells expressing either of the two constructs in which the first CAR had a CD4 endodomain (10C1CAR-CD22ALAb-CD8-CD4 and 10C1CAR-CD22ALAb-COMP-CD4) gave better killing that T cells expressing the activating CAR alone (10C1CAR) at low densities of target antigen (1090 copies per cell or below).

After 4 days co-culture, proliferation of T cells was investigated as described above. T cells expressing either of the two constructs in which the first CAR had a CD4 endodomain (10C1CAR-CD22ALAb-CD8-CD4 and 10C1CAR-CD22ALAb-COMP-CD4) showed increased proliferation compared with T-cells T cells expressing the activating CAR alone (10C1CAR) at both low (441 copies per cell) and high (78,916) target antigen densities.

**Claims**

1.  A cell which co-expresses a first chimeric antigen receptor (CAR) and a second CAR, each CAR comprising an antigen-binding domain and an endodomain, wherein the endodomain of first CAR comprises the tyrosine kinase domain of a Src family kinase or the intracellular domain of CD4 or CD8 coreceptor; and the endodomain of the second CAR is an activating endodomain comprising one or more immunoreceptor tyrosine-based activation motif(s) (ITAM(s)).

2.  A cell according to claim 1, wherein the endodomain of first CAR comprises the tyrosine kinase domain of Fyn, Src, Lck or a mutated Lck (Y505F).

3.  A cell according to claim 2, wherein the endodomain of first CAR comprises the tyrosine kinase domain of Fyn.

4.  A cell according to any preceding claim, wherein antigen-binding domain of the first CAR and/or the second CAR binds to the antigen CD22.

5.  A nucleic acid construct comprising a first nucleic acid sequence encoding a first chimeric antigen receptor (CAR) and a second nucleic acid sequence encoding a second CAR, as defined in any of claims 1 to 4.

6.  A nucleic acid construct according to claim 5, which has the following structure:
    AgB1-spacer1-TM1- endo1-coexpr-AbB2-spacer2-TM2-endo2

    in which AgB1 is a nucleic acid sequence encoding an antigen-binding domain of the first CAR;
    spacer 1 is a nucleic acid sequence encoding a spacer of the first CAR;
    TM1 is a nucleic acid sequence encoding a transmembrane domain of the first CAR;
    endo1 is a nucleic acid sequence encoding the endodomain of the first CAR;
    coexpr is a nucleic acid sequence enabling co-expression of both CARs;
    AgB2 is a nucleic acid sequence encoding an antigen-binding domain of the second CAR;
    spacer 2 is a nucleic acid sequence encoding a spacer of the second CAR;
    TM2 is a nucleic acid sequence encoding a transmembrane domain of the second CAR;
    Endo2 is a nucleic acid sequence encoding the activating endodomain of the second CAR;

    which nucleic acid sequence, when expressed in a T cell, encodes a polypeptide which is cleaved such that the first and second CARs are co-expressed at the T cell surface.

7.  A kit which comprises:

    (i) a first nucleic acid sequence encoding the first chimeric antigen receptor (CAR) as defined in any of claims 1 to 4, which nucleic acid sequence has the following structure:
    AgB1-spacer1-TM1-endo1
    in which:

      AgB1 is a nucleic acid sequence encoding an antigen-binding domain of the first CAR;
      spacer 1 is a nucleic acid sequence encoding a spacer of the first CAR;
      TM1 is a nucleic acid sequence encoding a transmembrane domain of the first CAR; and
      endo1 is a nucleic acid sequence encoding the endodomain of the first CAR; and

    (ii) a second nucleic acid sequence encoding the second chimeric antigen receptor (CAR) as defined in any of claims 1 to 6, which nucleic acid sequence has the following structure:
    AgB2-spacer2- TM2-endo2
    in which:

      AgB2 is a nucleic acid sequence encoding an antigen-binding domain of the second CAR;
      spacer 2 is a nucleic acid sequence encoding a spacer of the second CAR;
      TM2 is a nucleic acid sequence encoding a transmembrane domain of the second CAR; and
      endo2 is a nucleic acid sequence encoding the activating endodomain of the second CAR.

8.  A kit comprising a first vector which comprises the first nucleic acid sequence as defined in claim 7; and a second vector which comprises the second nucleic acid sequence as defined in claim 7.

9. A vector comprising a nucleic acid construct according to claim 5 or 6.

10. A method for making a cell according to any of claims 1 to 4, which comprises the step of introducing: a nucleic acid construct according to claim 5 or 6; a first nucleic acid sequence and a second nucleic acid sequence as defined in claim 7; a first vector and a second vector as defined in claim 8 or a vector according to claim 9, into a cell *ex vivo*.

11. A pharmaceutical composition comprising a plurality of cells according to any of claims 1 to 4.

12. A pharmaceutical composition according to claim 11 for use in treating a disease.

13. A pharmaceutical composition according to claim 11 for use in treating cancer.

**Patentansprüche**

1. Zelle, die einen ersten CAR (Chimeric Antigen Receptor) und einen zweiten CAR coexprimiert, wobei die CAR jeweils eine antigenbindende Domäne und eine Endodomäne umfassen, wobei die Endodomäne des ersten CAR die Tyrosinkinase-Domäne einer Kinase der Src-Familie oder die intrazelluläre Domäne von CD4- oder CD8-Corezeptor umfasst; und es sich bei der Endodomäne des zweiten CAR um eine aktivierende Endodomäne handelt, die ein oder mehrere ITAM (Immunoreceptor Tyrosine-based Activation Motif(s)) umfasst.

2. Zelle nach Anspruch 1, wobei die Endodomäne des ersten CAR die Tyrosinkinase-Domäne von Fyn, Src, Lck oder eines mutierten Lck (Y505F) umfasst.

3. Zelle nach Anspruch 2, wobei die Endodomäne des ersten CAR die Tyrosinkinase-Domäne von Fyn umfasst.

4. Zelle nach einem vorhergehenden Anspruch, wobei die antigenbindende Domäne des ersten CAR und/oder des zweiten CAR an das Antigen CD22 bindet.

5. Nukleinsäurekonstrukt, umfassend eine erste Nukleinsäuresequenz, die einen ersten CAR (Chimeric Antigen Receptor) codiert, und eine zweite Nukleinsäuresequenz, die einen zweiten CAR codiert, gemäß einem der Ansprüche 1 bis 4.

6. Nukleinsäurekonstrukt nach Anspruch 5, das die folgende Struktur aufweist:

   AgB1-spacer1-TM1-endo1-coexpr-AbB2-spacer2-TM2-endo2
   in der AgB1 für eine Nukleinsäuresequenz steht, die eine antigenbindende Domäne des ersten CAR codiert;
   spacer 1 für eine Nukleinsäuresequenz steht, die einen Spacer des ersten CAR codiert;
   TM1 für eine Nukleinsäuresequenz steht, die eine Transmembrandomäne des ersten CAR codiert;
   endo1 für eine Nukleinsäuresequenz steht, die die Endodomäne des ersten CAR codiert;
   coexpr für eine Nukleinsäuresequenz steht, die eine Coexpression beider CAR ermöglicht;
   AgB2 für eine Nukleinsäuresequenz steht, die eine antigenbindende Domäne des zweiten CAR codiert;
   spacer 2 für eine Nukleinsäuresequenz steht, die einen Spacer des zweiten CAR codiert;
   TM2 für eine Nukleinsäuresequenz steht, die eine Transmembrandomäne des zweiten CAR codiert;
   Endo2 für eine Nukleinsäuresequenz steht, die die aktivierende Endodomäne des zweiten CAR codiert;

   wobei die Nukleinsäuresequenz bei Expression in einer T-Zelle ein Polypeptid codiert, das gespalten wird, so dass der erste und der zweite CAR an der T-Zelloberfläche coexprimiert werden.

7. Kit, das Folgendes umfasst:

   (i) eine erste Nukleinsäuresequenz, die den ersten CAR (Chimeric Antigen Receptor) gemäß einem der Ansprüche 1 bis 4 codiert, wobei die Nukleinsäuresequenz die folgende Struktur aufweist:
   AgB1-spacer1-TM1-endo1
   in der:

AgB1 für eine Nukleinsäuresequenz steht, die eine antigenbindende Domäne des ersten CAR codiert;

spacer 1 für eine Nukleinsäuresequenz steht, die einen Spacer des ersten CAR codiert;

TM1 für eine Nukleinsäuresequenz steht, die eine Transmembrandomäne des ersten CAR codiert; und

endo1 für eine Nukleinsäuresequenz steht, die die Endodomäne des ersten CAR codiert; und

(ii) eine zweite Nukleinsäuresequenz, die den zweiten CAR (Chimeric Antigen Receptor) gemäß einem der Ansprüche 1 bis 6 codiert, wobei die Nukleinsäuresequenz die folgende Struktur aufweist:

AgB2-spacer2-TM2-endo2

in der:

AgB2 für eine Nukleinsäuresequenz steht, die eine antigenbindende Domäne des zweiten CAR codiert;

spacer 2 für eine Nukleinsäuresequenz steht, die einen Spacer des zweiten CAR codiert;

TM2 für eine Nukleinsäuresequenz steht, die eine Transmembrandomäne des zweiten CAR codiert; und

endo2 für eine Nukleinsäuresequenz steht, die die aktivierende Endodomäne des zweiten CAR codiert.

8. Kit, umfassend einen ersten Vektor, der die erste Nukleinsäuresequenz gemäß Anspruch 7 umfasst; und einen zweiten Vektor, der die zweite Nukleinsäuresequenz gemäß Anspruch 7 umfasst.

9. Vektor, umfassend ein Nukleinsäurekonstrukt nach Anspruch 5 oder 6.

10. Verfahren zur Erzeugung einer Zelle nach einem der Ansprüche 1 bis 4, das den Schritt umfasst, bei dem:

ein Nukleinsäurekonstrukt nach Anspruch 5 oder 6;

eine erste Nukleinsäuresequenz und eine zweite Nukleinsäuresequenz gemäß Anspruch 7; ein erster Vektor und ein zweiter Vektor gemäß Anspruch 8 oder ein Vektor nach Anspruch 9 in eine Zelle *ex vivo* eingeführt wird bzw. werden.

11. Pharmazeutische Zusammensetzung, umfassend mehrere Zellen nach einem der Ansprüche 1 bis 4.

12. Pharmazeutische Zusammensetzung nach Anspruch 11 zur Verwendung bei der Behandlung einer Krankheit.

13. Pharmazeutische Zusammensetzung nach Anspruch 11 zur Verwendung bei der Behandlung von Krebs.

## Revendications

1. Cellule qui coexprime un premier récepteur d'antigène chimérique (CAR) et un deuxième CAR, chaque CAR comprenant un domaine de liaison d'antigène et un endodomaine, dans laquelle l'endodomaine du premier CAR comprend le domaine de tyrosine kinase d'une kinase de la famille Src ou le domaine intracellulaire du corécepteur de CD4 ou CD8 ; et l'endodomaine du deuxième CAR est un endodomaine d'activation comprenant un ou plusieurs motifs d'activation à base de tyrosine d'immunorécepteur (ITAM).

2. Cellule selon la revendication 1, dans laquelle l'endodomaine du premier CAR comprend le domaine de tyrosine kinase de Fyn, Src, Lck ou d'un Lck muté (Y505F).

3. Cellule selon la revendication 2, dans laquelle l'endodomaine du premier CAR comprend le domaine de tyrosine kinase de Fyn.

4. Cellule selon l'une quelconque des revendications précédentes, dans laquelle le domaine de liaison d'antigène du premier CAR et/ou du deuxième CAR se lie à l'antigène CD22.

5. Construction d'acide nucléique comprenant une première séquence d'acide nucléique codant pour un premier récepteur d'antigène chimérique (CAR) et une deuxième séquence d'acide nucléique codant pour un deuxième CAR, selon l'une quelconque des revendications 1 à 4.

6. Construction d'acide nucléique selon la revendication 5, qui a la structure suivante :

AgB1-spacer1-TM1-endo1-coexpr-AbB2-spacer2-TM2-endo2

dans laquelle AgB1 est une séquence d'acide nucléique codant pour un domaine de liaison d'antigène du premier CAR ;

spacer1 est une séquence d'acide nucléique codant pour un espaceur du premier CAR ;

TM1 est une séquence d'acide nucléique codant pour un domaine transmembranaire du premier CAR ;

endo1 est une séquence d'acide nucléique codant pour l'endodomaine du premier CAR ;

coexpr est une séquence d'acide nucléique permettant la coexpression des deux CAR ;

AgB2 est une séquence d'acide nucléique codant pour un domaine de liaison d'antigène du deuxième CAR ;

spacer2 est une séquence d'acide nucléique codant pour un espaceur du deuxième CAR ;

TM2 est une séquence d'acide nucléique codant pour un domaine transmembranaire du deuxième CAR ;

Endo2 est une séquence d'acide nucléique codant pour l'endodomaine d'activation du deuxième CAR ;

laquelle séquence d'acide nucléique, lorsqu'elle est exprimée dans un lymphocyte T, code pour un polypeptide qui est clivé de sorte que les premier et deuxième CAR sont coexprimés à la surface du lymphocyte T.

7. Kit qui comprend :

(i) une première séquence d'acide nucléique codant pour le premier récepteur d'antigène chimérique (CAR) tel que défini dans l'une quelconque des revendications 1 à 4, laquelle séquence d'acide nucléique a la structure suivante :

AgB1-spacer1-TM1-endo1

dans laquelle :

AgB1 est une séquence d'acide nucléique codant pour un domaine de liaison d'antigène du premier CAR ;

Spacer 1 est une séquence d'acide nucléique codant pour un espaceur du premier CAR ;

TM1 est une séquence d'acide nucléique codant pour un domaine transmembranaire du premier CAR ; et

endo1 est une séquence d'acide nucléique codant pour l'endodomaine du premier CAR ; et

(ii) une deuxième séquence d'acide nucléique codant pour le deuxième récepteur d'antigène chimérique (CAR) tel que défini dans l'une quelconque des revendications 1 à 6, laquelle séquence d'acide nucléique a la structure suivante :

AgB2-spacer2-TM2-endo2

dans laquelle :

AgB2 est une séquence d'acide nucléique codant pour un domaine de liaison d'antigène du deuxième CAR ;

spacer 2 est une séquence d'acide nucléique codant pour un espaceur du deuxième CAR ;

TM2 est une séquence d'acide nucléique codant pour un domaine transmembranaire du deuxième CAR ; et

endo2 est une séquence d'acide nucléique codant pour l'endodomaine d'activation du deuxième CAR.

8. Kit comprenant un premier vecteur qui comprend la première séquence d'acide nucléique telle que définie dans la revendication 7 ; et un deuxième vecteur qui comprend la deuxième séquence d'acide nucléique telle que définie dans la revendication 7.

9. Vecteur comprenant une construction d'acide nucléique selon la revendication 5 ou 6.

10. Procédé de fabrication d'une cellule selon l'une quelconque des revendications 1 à 4, qui comprend l'étape d'introduction de : une construction d'acide nucléique selon la revendication 5 ou 6 ; une première séquence d'acide nucléique et une deuxième séquence d'acide nucléique telles que définies dans la revendication 7 ; un premier vecteur et un deuxième vecteur tels que définis dans la revendication 8 ou un vecteur selon la revendication 9, dans une cellule ex *vivo.*

11. Composition pharmaceutique comprenant une pluralité de cellules selon l'une quelconque des revendications 1 à 4.

12. Composition pharmaceutique selon la revendication 11 pour utilisation dans le traitement d'une maladie.

13. Composition pharmaceutique selon la revendication 11 pour utilisation dans le traitement du cancer.

FIG. 1

*Target Cell*

antigen

binder 1

*Immune synapse*

2

1

binder 2

spacer 1

spacer 2

Activating endodomain

PA endodomain

*T-Cell*

FIG. 2

FIG. 3

FIG. 4

FIG. 5

FIG. 6

FIG. 7(a)

FIG. 7(b)

FIG. 8

FIG. 9

Target Cell

Antigen 1

Antigen 2

Immune synapse

Activating endodomain

PA endodomain

T-Cell

FIG. 10

FIG. 11

FIG. 12

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016102965 A
- US 7052906 B1

- GB 1706123 A

**Non-patent literature cited in the description**

- **WOOLDRIDGE et al.** *J Biol Chem,* 2005, vol. 280 (30), 27491-501
- **HASO et al.** *Blood,* 2013, vol. 121 (7

- **BRIDGEMAN et al.** *J. Immunol.,* 2010, vol. 184, 6938-6949
- **DONNELLY et al.** *J. Gen. Virol.,* 2001, vol. 82, 1027-1041

SEQUENCE LISTING

    <110> Autolus Ltd

    <120> CELL

    <130> P112129PCT

    <150> GB 1706123.5
    <151> 2017-04-18

    <160> 82

    <170> PatentIn version 3.5

    <210> 1
    <211> 254
    <212> PRT
    <213> Artificial Sequence

    <220>
    <223> Tyrosine kinase domain of Fyn

    <400> 1

Leu Gln Leu Ile Lys Arg Leu Gly Asn Gly Gln Phe Gly Glu Val Trp
1               5               10                      15

Met Gly Thr Trp Asn Gly Asn Thr Lys Val Ala Ile Lys Thr Leu Lys
            20              25                  30

Pro Gly Thr Met Ser Pro Glu Ser Phe Leu Glu Glu Ala Gln Ile Met
            35              40                  45

Lys Lys Leu Lys His Asp Lys Leu Val Gln Leu Tyr Ala Val Val Ser
    50              55                  60

Glu Glu Pro Ile Tyr Ile Val Thr Glu Tyr Met Asn Lys Gly Ser Leu
65              70                  75                      80

Leu Asp Phe Leu Lys Asp Gly Glu Gly Arg Ala Leu Lys Leu Pro Asn
            85                  90                  95

Leu Val Asp Met Ala Ala Gln Val Ala Ala Gly Met Ala Tyr Ile Glu
            100             105                 110

Arg Met Asn Tyr Ile His Arg Asp Leu Arg Ser Ala Asn Ile Leu Val
        115                 120                 125

Gly Asn Gly Leu Ile Cys Lys Ile Ala Asp Phe Gly Leu Ala Arg Leu
    130             135                 140

Ile Glu Asp Asn Glu Tyr Thr Ala Arg Gln Gly Ala Lys Phe Pro Ile
145                 150                 155                 160

Lys Trp Thr Ala Pro Glu Arg Ala Leu Tyr Gly Arg Phe Thr Ile Lys
            165                 170                 175

Ser Asp Val Trp Ser Phe Gly Ile Leu Leu Thr Glu Leu Val Thr Lys
            180             185                 190

Gly Arg Val Pro Tyr Pro Gly Met Asn Asn Arg Glu Val Leu Glu Gln
    195             200                 205

Val Glu Arg Gly Tyr Arg Met Pro Cys Pro Gln Asp Cys Pro Ile Ser
    210             215                 220

Leu His Glu Leu Met Ile His Cys Trp Lys Lys Asp Pro Glu Glu Arg
225             230                 235                     240

Pro Thr Phe Glu Tyr Leu Gln Ser Phe Leu Glu Asp Tyr Phe
            245                 250

31

<210> 2
<211> 536
<212> PRT
<213> Homo sapiens

<400> 2

```
Gly Cys Val Gln Cys Lys Asp Lys Glu Ala Thr Lys Leu Thr Glu Glu
1               5               10              15

Arg Asp Gly Ser Leu Asn Gln Ser Ser Gly Tyr Arg Tyr Gly Thr Asp
            20              25              30

Pro Thr Pro Gln His Tyr Pro Ser Phe Gly Val Thr Ser Ile Pro Asn
            35              40              45

Tyr Asn Asn Phe His Ala Ala Gly Gly Gln Gly Leu Thr Val Phe Gly
    50              55              60

Gly Val Asn Ser Ser Ser His Thr Gly Thr Leu Arg Thr Arg Gly Gly
65              70              75              80

Thr Gly Val Thr Leu Phe Val Ala Leu Tyr Asp Tyr Glu Ala Arg Thr
            85              90              95

Glu Asp Asp Leu Ser Phe His Lys Gly Glu Lys Phe Gln Ile Leu Asn
            100             105             110

Ser Ser Glu Gly Asp Trp Trp Glu Ala Arg Ser Leu Thr Thr Gly Glu
        115             120             125
```

```
Thr Gly Tyr Ile Pro Ser Asn Tyr Val Ala Pro Val Asp Ser Ile Gln
    130             135             140

Ala Glu Glu Trp Tyr Phe Gly Lys Leu Gly Arg Lys Asp Ala Glu Arg
145             150             155                 160

Gln Leu Leu Ser Phe Gly Asn Pro Arg Gly Thr Phe Leu Ile Arg Glu
                165             170                 175

Ser Glu Thr Thr Lys Gly Ser Tyr Ser Leu Ser Ile Arg Asp Trp Asp
            180             185             190

Asp Met Lys Gly Asp His Val Lys His Tyr Lys Ile Arg Lys Leu Asp
        195             200             205

Asn Gly Gly Tyr Tyr Ile Thr Thr Arg Ala Gln Phe Glu Thr Leu Gln
    210             215             220

Gln Leu Val Gln His Tyr Ser Glu Arg Ala Ala Gly Leu Cys Cys Arg
225             230             235                 240

Leu Val Val Pro Cys His Lys Gly Met Pro Arg Leu Thr Asp Leu Ser
            245             250                 255

Val Lys Thr Lys Asp Val Trp Glu Ile Pro Arg Glu Ser Leu Gln Leu
        260             265             270

Ile Lys Arg Leu Gly Asn Gly Gln Phe Gly Glu Val Trp Met Gly Thr
        275             280             285

Trp Asn Gly Asn Thr Lys Val Ala Ile Lys Thr Leu Lys Pro Gly Thr
    290             295             300

Met Ser Pro Glu Ser Phe Leu Glu Glu Ala Gln Ile Met Lys Lys Leu
305             310             315                 320

Lys His Asp Lys Leu Val Gln Leu Tyr Ala Val Val Ser Glu Glu Pro
            325             330                 335

Ile Tyr Ile Val Thr Glu Tyr Met Asn Lys Gly Ser Leu Leu Asp Phe
            340             345             350

Leu Lys Asp Gly Glu Gly Arg Ala Leu Lys Leu Pro Asn Leu Val Asp
        355             360             365

Met Ala Ala Gln Val Ala Ala Gly Met Ala Tyr Ile Glu Arg Met Asn
```

```
                  370                     375                         380

        Tyr Ile His Arg Asp Leu Arg Ser Ala Asn Ile Leu Val Gly Asn Gly
        385                 390                 395                 400


        Leu Ile Cys Lys Ile Ala Asp Phe Gly Leu Ala Arg Leu Ile Glu Asp
                        405                 410                 415


        Asn Glu Tyr Thr Ala Arg Gln Gly Ala Lys Phe Pro Ile Lys Trp Thr
                    420                 425                 430


        Ala Pro Glu Arg Ala Leu Tyr Gly Arg Phe Thr Ile Lys Ser Asp Val
                    435                 440                 445


        Trp Ser Phe Gly Ile Leu Leu Thr Glu Leu Val Thr Lys Gly Arg Val
            450                 455                 460


        Pro Tyr Pro Gly Met Asn Asn Arg Glu Val Leu Glu Gln Val Glu Arg
        465                 470                 475                 480


        Gly Tyr Arg Met Pro Cys Pro Gln Asp Cys Pro Ile Ser Leu His Glu
                        485                 490                 495


        Leu Met Ile His Cys Trp Lys Lys Asp Pro Glu Glu Arg Pro Thr Phe
                    500                 505                 510


        Glu Tyr Leu Gln Ser Phe Leu Glu Asp Tyr Phe Thr Ala Thr Glu Pro
                    515                 520                 525


        Gln Tyr Gln Pro Gly Glu Asn Leu
            530                 535
```

<210> 3
<211> 254
<212> PRT
<213> Artificial Sequence

<220>
<223> Tyrosine kinase domain of Src

<400> 3

34

Leu Arg Leu Glu Val Lys Leu Gly Gln Gly Cys Phe Gly Glu Val Trp
1           5           10              15

Met Gly Thr Trp Asn Gly Thr Thr Arg Val Ala Ile Lys Thr Leu Lys
        20              25              30

Pro Gly Thr Met Ser Pro Glu Ala Phe Leu Gln Glu Ala Gln Val Met
        35              40              45

Lys Lys Leu Arg His Glu Lys Leu Val Gln Leu Tyr Ala Val Val Ser
    50              55              60

Glu Glu Pro Ile Tyr Ile Val Thr Glu Tyr Met Ser Lys Gly Ser Leu
65              70              75              80

Leu Asp Phe Leu Lys Gly Glu Thr Gly Lys Tyr Leu Arg Leu Pro Gln
            85              90              95

Leu Val Asp Met Ala Ala Gln Ile Ala Ser Gly Met Ala Tyr Val Glu
        100             105             110

Arg Met Asn Tyr Val His Arg Asp Leu Arg Ala Ala Asn Ile Leu Val
        115             120             125

Gly Glu Asn Leu Val Cys Lys Val Ala Asp Phe Gly Leu Ala Arg Leu
    130             135             140

Ile Glu Asp Asn Glu Tyr Thr Ala Arg Gln Gly Ala Lys Phe Pro Ile
145             150             155             160

Lys Trp Thr Ala Pro Glu Ala Ala Leu Tyr Gly Arg Phe Thr Ile Lys
            165             170             175

Ser Asp Val Trp Ser Phe Gly Ile Leu Leu Thr Glu Leu Thr Thr Lys
        180             185             190

Gly Arg Val Pro Tyr Pro Gly Met Val Asn Arg Glu Val Leu Asp Gln
    195             200             205

Val Glu Arg Gly Tyr Arg Met Pro Cys Pro Pro Glu Cys Pro Glu Ser
    210             215             220

Leu His Asp Leu Met Cys Gln Cys Trp Arg Lys Glu Pro Glu Glu Arg
225             230             235             240

Pro Thr Phe Glu Tyr Leu Gln Ala Phe Leu Glu Asp Tyr Phe
            245             250

<210> 4
<211> 536
<212> PRT
<213> Homo sapiens

<400> 4

```
Met Gly Ser Asn Lys Ser Lys Pro Lys Asp Ala Ser Gln Arg Arg Arg
1               5                   10                  15
```

Ser Leu Glu Pro Ala Glu Asn Val His Gly Ala Gly Gly Gly Ala Phe
20                      25                      30

Pro Ala Ser Gln Thr Pro Ser Lys Pro Ala Ser Ala Asp Gly His Arg
35                      40                      45

Gly Pro Ser Ala Ala Phe Ala Pro Ala Ala Ala Glu Pro Lys Leu Phe
50                      55                      60

Gly Gly Phe Asn Ser Ser Asp Thr Val Thr Ser Pro Gln Arg Ala Gly
65                      70                      75                      80

Pro Leu Ala Gly Gly Val Thr Thr Phe Val Ala Leu Tyr Asp Tyr Glu
85                      90                      95

Ser Arg Thr Glu Thr Asp Leu Ser Phe Lys Lys Gly Glu Arg Leu Gln
100                     105                     110

Ile Val Asn Asn Thr Glu Gly Asp Trp Trp Leu Ala His Ser Leu Ser
115                     120                     125

Thr Gly Gln Thr Gly Tyr Ile Pro Ser Asn Tyr Val Ala Pro Ser Asp
130                     135                     140

Ser Ile Gln Ala Glu Glu Trp Tyr Phe Gly Lys Ile Thr Arg Arg Glu
145                     150                     155                     160

Ser Glu Arg Leu Leu Leu Asn Ala Glu Asn Pro Arg Gly Thr Phe Leu
165                     170                     175

Val Arg Glu Ser Glu Thr Thr Lys Gly Ala Tyr Cys Leu Ser Val Ser
180                     185                     190

Asp Phe Asp Asn Ala Lys Gly Leu Asn Val Lys His Tyr Lys Ile Arg
195                     200                     205

Lys Leu Asp Ser Gly Gly Phe Tyr Ile Thr Ser Arg Thr Gln Phe Asn
210                     215                     220

Ser Leu Gln Gln Leu Val Ala Tyr Tyr Ser Lys His Ala Asp Gly Leu
225                     230                     235                     240

Cys His Arg Leu Thr Thr Val Cys Pro Thr Ser Lys Pro Gln Thr Gln
245                     250                     255

Gly Leu Ala Lys Asp Ala Trp Glu Ile Pro Arg Glu Ser Leu Arg Leu

260          265          270

Glu Val Lys Leu Gly Gln Gly Cys Phe Gly Glu Val Trp Met Gly Thr
      275            280              285

Trp Asn Gly Thr Thr Arg Val Ala Ile Lys Thr Leu Lys Pro Gly Thr
290           295            300

Met Ser Pro Glu Ala Phe Leu Gln Glu Ala Gln Val Met Lys Lys Leu
305           310           315            320

Arg His Glu Lys Leu Val Gln Leu Tyr Ala Val Val Ser Glu Glu Pro
      325           330             335

Ile Tyr Ile Val Thr Glu Tyr Met Ser Lys Gly Ser Leu Leu Asp Phe
      340           345             350

Leu Lys Gly Glu Thr Gly Lys Tyr Leu Arg Leu Pro Gln Leu Val Asp
      355           360             365

Met Ala Ala Gln Ile Ala Ser Gly Met Ala Tyr Val Glu Arg Met Asn
      370           375            380

Tyr Val His Arg Asp Leu Arg Ala Ala Asn Ile Leu Val Gly Glu Asn
385           390           395           400

Leu Val Cys Lys Val Ala Asp Phe Gly Leu Ala Arg Leu Ile Glu Asp
      405           410             415

Asn Glu Tyr Thr Ala Arg Gln Gly Ala Lys Phe Pro Ile Lys Trp Thr
      420           425             430

Ala Pro Glu Ala Ala Leu Tyr Gly Arg Phe Thr Ile Lys Ser Asp Val
      435           440             445

Trp Ser Phe Gly Ile Leu Leu Thr Glu Leu Thr Thr Lys Gly Arg Val
450           455           460

Pro Tyr Pro Gly Met Val Asn Arg Glu Val Leu Asp Gln Val Glu Arg
465           470           475           480

Gly Tyr Arg Met Pro Cys Pro Pro Glu Cys Pro Glu Ser Leu His Asp
      485           490             495

Leu Met Cys Gln Cys Trp Arg Lys Glu Pro Glu Glu Arg Pro Thr Phe
      500           505             510

```
Glu Tyr Leu Gln Ala Phe Leu Glu Asp Tyr Phe Thr Ser Thr Glu Pro
        515                 520                 525


Gln Tyr Gln Pro Gly Glu Asn Leu
    530                 535
```

<210> 5
<211> 254
<212> PRT
<213> Artificial Sequence

<220>
<223> Tyrosine kinase domain of Lck

<400> 5

Leu Lys Leu Val Glu Arg Leu Gly Ala Gly Gln Phe Gly Glu Val Trp
1               5               10                  15

Met Gly Tyr Tyr Asn Gly His Thr Lys Val Ala Val Lys Ser Leu Lys
            20                  25                  30

Gln Gly Ser Met Ser Pro Asp Ala Phe Leu Ala Glu Ala Asn Leu Met
            35                  40                  45

Lys Gln Leu Gln His Gln Arg Leu Val Arg Leu Tyr Ala Val Val Thr
    50                  55                  60

Gln Glu Pro Ile Tyr Ile Ile Thr Glu Tyr Met Glu Asn Gly Ser Leu
65                  70                  75                  80

Val Asp Phe Leu Lys Thr Pro Ser Gly Ile Lys Leu Thr Ile Asn Lys
                85                  90                  95

Leu Leu Asp Met Ala Ala Gln Ile Ala Glu Gly Met Ala Phe Ile Glu
            100                 105                 110

Glu Arg Asn Tyr Ile His Arg Asp Leu Arg Ala Ala Asn Ile Leu Val
        115                 120                 125

Ser Asp Thr Leu Ser Cys Lys Ile Ala Asp Phe Gly Leu Ala Arg Leu
    130                 135                 140

Ile Glu Asp Asn Glu Tyr Thr Ala Arg Glu Gly Ala Lys Phe Pro Ile
145                 150                 155                 160

Lys Trp Thr Ala Pro Glu Ala Ile Asn Tyr Gly Thr Phe Thr Ile Lys
                165                 170                 175

Ser Asp Val Trp Ser Phe Gly Ile Leu Leu Thr Glu Ile Val Thr His

Gly Arg Ile Pro Tyr Pro Gly Met Thr Asn Pro Glu Val Ile Gln Asn
       195            200            205

Leu Glu Arg Gly Tyr Arg Met Val Arg Pro Asp Asn Cys Pro Glu Glu
       210            215            220

Leu Tyr Gln Leu Met Arg Leu Cys Trp Lys Glu Arg Pro Glu Asp Arg
225            230          235          240

Pro Thr Phe Asp Tyr Leu Arg Ser Val Leu Glu Asp Phe Phe
          245          250

<210> 6
<211> 509
<212> PRT
<213> Homo sapiens

<400> 6

```
Met Gly Cys Gly Cys Ser Ser His Pro Glu Asp Asp Trp Met Glu Asn
1               5               10              15

Ile Asp Val Cys Glu Asn Cys His Tyr Pro Ile Val Pro Leu Asp Gly
        20              25              30

Lys Gly Thr Leu Leu Ile Arg Asn Gly Ser Glu Val Arg Asp Pro Leu
        35              40              45

Val Thr Tyr Glu Gly Ser Asn Pro Pro Ala Ser Pro Leu Gln Asp Asn
        50              55              60

Leu Val Ile Ala Leu His Ser Tyr Glu Pro Ser His Asp Gly Asp Leu
65              70              75              80

Gly Phe Glu Lys Gly Glu Gln Leu Arg Ile Leu Glu Gln Ser Gly Glu
                85              90              95

Trp Trp Lys Ala Gln Ser Leu Thr Thr Gly Gln Glu Gly Phe Ile Pro
        100             105             110

Phe Asn Phe Val Ala Lys Ala Asn Ser Leu Glu Pro Glu Pro Trp Phe
        115             120             125

Phe Lys Asn Leu Ser Arg Lys Asp Ala Glu Arg Gln Leu Leu Ala Pro
        130             135             140

Gly Asn Thr His Gly Ser Phe Leu Ile Arg Glu Ser Glu Ser Thr Ala
```

|       |       |       | 145   |       | 150   |       |       |       | 155   |       |       |       | 160   |
|-------|-------|-------|-------|-------|-------|-------|-------|-------|-------|-------|-------|-------|-------|

Gly Ser Phe Ser Leu Ser Val Arg Asp Phe Asp Gln Asn Gln Gly Glu
165                 170                 175

Val Val Lys His Tyr Lys Ile Arg Asn Leu Asp Asn Gly Gly Phe Tyr
180                 185                 190

Ile Ser Pro Arg Ile Thr Phe Pro Gly Leu His Glu Leu Val Arg His
195                 200                 205

Tyr Thr Asn Ala Ser Asp Gly Leu Cys Thr Arg Leu Ser Arg Pro Cys
210                 215                 220

Gln Thr Gln Lys Pro Gln Lys Pro Trp Trp Glu Asp Glu Trp Glu Val
225                 230                 235                 240

Pro Arg Glu Thr Leu Lys Leu Val Glu Arg Leu Gly Ala Gly Gln Phe
245                 250                 255

Gly Glu Val Trp Met Gly Tyr Tyr Asn Gly His Thr Lys Val Ala Val
260                 265                 270

Lys Ser Leu Lys Gln Gly Ser Met Ser Pro Asp Ala Phe Leu Ala Glu
275                 280                 285

Ala Asn Leu Met Lys Gln Leu Gln His Gln Arg Leu Val Arg Leu Tyr
290                 295                 300

Ala Val Val Thr Gln Glu Pro Ile Tyr Ile Ile Thr Glu Tyr Met Glu
305                 310                 315                 320

Asn Gly Ser Leu Val Asp Phe Leu Lys Thr Pro Ser Gly Ile Lys Leu
325                 330                 335

Thr Ile Asn Lys Leu Leu Asp Met Ala Ala Gln Ile Ala Glu Gly Met
340                 345                 350

Ala Phe Ile Glu Glu Arg Asn Tyr Ile His Arg Asp Leu Arg Ala Ala
355                 360                 365

Asn Ile Leu Val Ser Asp Thr Leu Ser Cys Lys Ile Ala Asp Phe Gly
370                 375                 380

Leu Ala Arg Leu Ile Glu Asp Asn Glu Tyr Thr Ala Arg Glu Gly Ala
385                 390                 395                 400

43

```
Lys Phe Pro Ile Lys Trp Thr Ala Pro Glu Ala Ile Asn Tyr Gly Thr
                405             410                 415

Phe Thr Ile Lys Ser Asp Val Trp Ser Phe Gly Ile Leu Leu Thr Glu
                420             425             430

Ile Val Thr His Gly Arg Ile Pro Tyr Pro Gly Met Thr Asn Pro Glu
                435             440             445

Val Ile Gln Asn Leu Glu Arg Gly Tyr Arg Met Val Arg Pro Asp Asn
    450             455             460

Cys Pro Glu Glu Leu Tyr Gln Leu Met Arg Leu Cys Trp Lys Glu Arg
465             470             475             480

Pro Glu Asp Arg Pro Thr Phe Asp Tyr Leu Arg Ser Val Leu Glu Asp
                485             490             495

Phe Phe Thr Ala Thr Glu Gly Gln Tyr Gln Pro Gln Pro
                500             505
```

<210> 7
<211> 254
<212> PRT
<213> Artificial Sequence

<220>
<223> Tyrosine kinase domain of Lck_Y505F

<400> 7

44

```
Leu Lys Leu Val Glu Arg Leu Gly Ala Gly Gln Phe Gly Glu Val Trp
1               5               10              15

Met Gly Tyr Tyr Asn Gly His Thr Lys Val Ala Val Arg Ser Leu Lys
            20              25              30

Gln Gly Ser Met Ser Pro Asp Ala Phe Leu Ala Glu Ala Asn Leu Met
            35              40              45

Lys Gln Leu Gln His Gln Arg Leu Val Arg Leu Tyr Ala Val Val Thr
    50              55              60

Gln Glu Pro Ile Tyr Ile Ile Thr Glu Tyr Met Glu Asn Gly Ser Leu
65              70              75              80

Val Asp Phe Leu Lys Thr Pro Ser Gly Ile Lys Leu Thr Ile Asn Lys
            85              90              95

Leu Leu Asp Met Ala Ala Gln Ile Ala Glu Gly Met Ala Phe Ile Glu
```

                    100                        105                        110

        Glu Arg Asn Tyr Ile His Arg Asp Leu Arg Ala Ala Asn Ile Leu Val
                115                    120                    125


        Ser Asp Thr Leu Ser Cys Lys Ile Ala Asp Phe Gly Leu Ala Arg Leu
                130                    135                    140


        Ile Glu Asp Asn Glu Tyr Thr Ala Arg Glu Gly Ala Lys Phe Pro Ile
        145                    150                    155                    160


        Lys Trp Thr Ala Pro Glu Ala Ile Asn Tyr Gly Thr Phe Thr Ile Lys
                        165                    170                    175


        Ser Asp Val Trp Ser Phe Gly Ile Leu Leu Thr Glu Ile Val Thr His
                        180                    185                    190


        Gly Arg Ile Pro Tyr Pro Gly Met Thr Asn Pro Glu Val Ile Gln Asn
                195                    200                    205


        Leu Glu Arg Gly Tyr Arg Met Val Arg Pro Asp Asn Cys Pro Glu Glu
                210                    215                    220


        Leu Tyr Gln Leu Met Arg Leu Cys Trp Lys Glu Arg Pro Glu Asp Arg
        225                    230                    235                    240


        Pro Thr Phe Asp Tyr Leu Arg Ser Val Leu Glu Asp Phe Phe
                        245                    250

<210> 8
<211> 509
<212> PRT
<213> Artificial Sequence

<220>
<223> Full length Lck_Y505F

<400> 8

```
Met Gly Cys Gly Cys Ser Ser His Pro Glu Asp Asp Trp Met Glu Asn
1               5               10              15

Ile Asp Val Cys Glu Asn Cys His Tyr Pro Ile Val Pro Leu Asp Gly
        20              25              30

Lys Gly Thr Leu Leu Ile Arg Asn Gly Ser Glu Val Arg Asp Pro Leu
        35              40              45

Val Thr Tyr Glu Gly Ser Asn Pro Pro Ala Ser Pro Leu Gln Asp Asn
    50              55              60
```

```
Leu Val Ile Ala Leu His Ser Tyr Glu Pro Ser His Asp Gly Asp Leu
65                  70              75                  80

Gly Phe Glu Lys Gly Glu Gln Leu Arg Ile Leu Glu Gln Ser Gly Glu
                85              90              95

Trp Trp Lys Ala Gln Ser Leu Thr Thr Gly Gln Glu Gly Phe Ile Pro
            100             105             110

Phe Asn Phe Val Ala Lys Ala Asn Ser Leu Glu Pro Glu Pro Trp Phe
        115             120             125

Phe Lys Asn Leu Ser Arg Lys Asp Ala Glu Arg Gln Leu Leu Ala Pro
    130             135             140

Gly Asn Thr His Gly Ser Phe Leu Ile Arg Glu Ser Glu Ser Thr Ala
145             150             155             160

Gly Ser Phe Ser Leu Ser Val Arg Asp Phe Asp Gln Asn Gln Gly Glu
            165             170             175

Val Val Lys His Tyr Lys Ile Arg Asn Leu Asp Asn Gly Gly Phe Tyr
        180             185             190

Ile Ser Pro Arg Ile Thr Phe Pro Gly Leu His Glu Leu Val Arg His
        195             200             205

Tyr Thr Asn Ala Ser Asp Gly Leu Cys Thr Arg Leu Ser Arg Pro Cys
    210             215             220

Gln Thr Gln Lys Pro Gln Lys Pro Trp Trp Glu Asp Glu Trp Glu Val
225             230             235             240

Pro Arg Glu Thr Leu Lys Leu Val Glu Arg Leu Gly Ala Gly Gln Phe
            245             250             255

Gly Glu Val Trp Met Gly Tyr Tyr Asn Gly His Thr Lys Val Ala Val
            260             265             270

Arg Ser Leu Lys Gln Gly Ser Met Ser Pro Asp Ala Phe Leu Ala Glu
    275             280             285

Ala Asn Leu Met Lys Gln Leu Gln His Gln Arg Leu Val Arg Leu Tyr
    290             295             300

Ala Val Val Thr Gln Glu Pro Ile Tyr Ile Ile Thr Glu Tyr Met Glu
```

```
                  305                    310                    315                    320


         Asn Gly Ser Leu Val Asp Phe Leu Lys Thr Pro Ser Gly Ile Lys Leu
                         325                    330                    335

         Thr Ile Asn Lys Leu Leu Asp Met Ala Ala Gln Ile Ala Glu Gly Met
                 340                    345                    350

         Ala Phe Ile Glu Glu Arg Asn Tyr Ile His Arg Asp Leu Arg Ala Ala
                 355                    360                    365

         Asn Ile Leu Val Ser Asp Thr Leu Ser Cys Lys Ile Ala Asp Phe Gly
                 370                    375                    380

         Leu Ala Arg Leu Ile Glu Asp Asn Glu Tyr Thr Ala Arg Glu Gly Ala
         385                    390                    395                    400

         Lys Phe Pro Ile Lys Trp Thr Ala Pro Glu Ala Ile Asn Tyr Gly Thr
                         405                    410                    415

         Phe Thr Ile Lys Ser Asp Val Trp Ser Phe Gly Ile Leu Leu Thr Glu
                 420                    425                    430

         Ile Val Thr His Gly Arg Ile Pro Tyr Pro Gly Met Thr Asn Pro Glu
                 435                    440                    445

         Val Ile Gln Asn Leu Glu Arg Gly Tyr Arg Met Val Arg Pro Asp Asn
                 450                    455                    460

         Cys Pro Glu Glu Leu Tyr Gln Leu Met Arg Leu Cys Trp Lys Glu Arg
         465                    470                    475                    480

         Pro Glu Asp Arg Pro Thr Phe Asp Tyr Leu Arg Ser Val Leu Glu Asp
                         485                    490                    495

         Phe Phe Thr Ala Thr Glu Gly Gln Phe Gln Pro Gln Pro
                         500                    505
```

<210> 9
<211> 40
<212> PRT
<213> Artificial Sequence

<220>
<223> Cytoplasmic tail of CD4

<400> 9

```
Cys Val Arg Cys Arg His Arg Arg Arg Gln Ala Glu Arg Met Ser Gln
1               5               10              15

Ile Lys Arg Leu Leu Ser Glu Lys Lys Thr Cys Gln Cys Pro His Arg
            20              25              30

Phe Gln Lys Thr Cys Ser Pro Ile
            35              40
```

<210> 10
<211> 458
<212> PRT
<213> Homo sapiens

<400> 10

```
Met Asn Arg Gly Val Pro Phe Arg His Leu Leu Leu Val Leu Gln Leu
1               5               10              15

Ala Leu Leu Pro Ala Ala Thr Gln Gly Lys Lys Val Val Leu Gly Lys
        20              25              30

Lys Gly Asp Thr Val Glu Leu Thr Cys Thr Ala Ser Gln Lys Lys Ser
        35              40              45

Ile Gln Phe His Trp Lys Asn Ser Asn Gln Ile Lys Ile Leu Gly Asn
    50              55              60

Gln Gly Ser Phe Leu Thr Lys Gly Pro Ser Lys Leu Asn Asp Arg Ala
65              70              75              80

Asp Ser Arg Arg Ser Leu Trp Asp Gln Gly Asn Phe Pro Leu Ile Ile
            85              90              95

Lys Asn Leu Lys Ile Glu Asp Ser Asp Thr Tyr Ile Cys Glu Val Glu
        100             105             110

Asp Gln Lys Glu Glu Val Gln Leu Leu Val Phe Gly Leu Thr Ala Asn
        115             120             125

Ser Asp Thr His Leu Leu Gln Gly Gln Ser Leu Thr Leu Thr Leu Glu
        130             135             140

Ser Pro Pro Gly Ser Ser Pro Ser Val Gln Cys Arg Ser Pro Arg Gly
145             150             155             160

Lys Asn Ile Gln Gly Gly Lys Thr Leu Ser Val Ser Gln Leu Glu Leu
            165             170             175

Gln Asp Ser Gly Thr Trp Thr Cys Thr Val Leu Gln Asn Gln Lys Lys
            180             185             190
```

Val Glu Phe Lys Ile Asp Ile Val Val Leu Ala Phe Gln Lys Ala Ser
        195             200             205

Ser Ile Val Tyr Lys Lys Glu Gly Glu Gln Val Glu Phe Ser Phe Pro
        210             215             220

Leu Ala Phe Thr Val Glu Lys Leu Thr Gly Ser Gly Glu Leu Trp Trp
225             230             235             240

Gln Ala Glu Arg Ala Ser Ser Ser Lys Ser Trp Ile Thr Phe Asp Leu
            245             250             255

Lys Asn Lys Glu Val Ser Val Lys Arg Val Thr Gln Asp Pro Lys Leu
        260             265             270

Gln Met Gly Lys Lys Leu Pro Leu His Leu Thr Leu Pro Gln Ala Leu
        275             280             285

Pro Gln Tyr Ala Gly Ser Gly Asn Leu Thr Leu Ala Leu Glu Ala Lys
        290             295             300

Thr Gly Lys Leu His Gln Glu Val Asn Leu Val Val Met Arg Ala Thr
305             310             315             320

Gln Leu Gln Lys Asn Leu Thr Cys Glu Val Trp Gly Pro Thr Ser Pro
            325             330             335

Lys Leu Met Leu Ser Leu Lys Leu Glu Asn Lys Glu Ala Lys Val Ser
            340             345             350

Lys Arg Glu Lys Ala Val Trp Val Leu Asn Pro Glu Ala Gly Met Trp
        355             360             365

Gln Cys Leu Leu Ser Asp Ser Gly Gln Val Leu Leu Glu Ser Asn Ile
        370             375             380

Lys Val Leu Pro Thr Trp Ser Thr Pro Val Gln Pro Met Ala Leu Ile
385             390             395             400

Val Leu Gly Gly Val Ala Gly Leu Leu Leu Phe Ile Gly Leu Gly Ile
            405             410             415

Phe Phe Cys Val Arg Cys Arg His Arg Arg Arg Gln Ala Glu Arg Met
        420             425             430

Ser Gln Ile Lys Arg Leu Leu Ser Glu Lys Lys Thr Cys Gln Cys Pro

52

435 440 445

His Arg Phe Gln Lys Thr Cys Ser Pro Ile
450 455

<210> 11
<211> 32
<212> PRT
<213> Artificial Sequence

<220>
<223> Cytoplasmic tail of CD8

<400> 11

Leu Tyr Cys Asn His Arg Asn Arg Arg Val Cys Lys Cys Pro Arg
1               5                   10                  15

Pro Val Val Lys Ser Gly Asp Lys Pro Ser Leu Ser Ala Arg Tyr Val
20                  25                  30

<210> 12
<211> 235
<212> PRT
<213> Homo sapiens

<400> 12

```
Met Ala Leu Pro Val Thr Ala Leu Leu Leu Pro Leu Ala Leu Leu Leu
1               5                   10                  15

His Ala Ala Arg Pro Ser Gln Phe Arg Val Ser Pro Leu Asp Arg Thr
            20                  25                  30

Trp Asn Leu Gly Glu Thr Val Glu Leu Lys Cys Gln Val Leu Leu Ser
            35                  40                  45

Asn Pro Thr Ser Gly Cys Ser Trp Leu Phe Gln Pro Arg Gly Ala Ala
        50                  55                  60

Ala Ser Pro Thr Phe Leu Leu Tyr Leu Ser Gln Asn Lys Pro Lys Ala
65                  70                  75                  80

Ala Glu Gly Leu Asp Thr Gln Arg Phe Ser Gly Lys Arg Leu Gly Asp
                85                  90                  95

Thr Phe Val Leu Thr Leu Ser Asp Phe Arg Arg Glu Asn Glu Gly Tyr
            100                 105                 110

Tyr Phe Cys Ser Ala Leu Ser Asn Ser Ile Met Tyr Phe Ser His Phe
            115                 120                 125

Val Pro Val Phe Leu Pro Ala Lys Pro Thr Thr Thr Pro Ala Pro Arg
    130                 135                 140

Pro Pro Thr Pro Ala Pro Thr Ile Ala Ser Gln Pro Leu Ser Leu Arg
145                 150                 155                 160

Pro Glu Ala Cys Arg Pro Ala Ala Gly Gly Ala Val His Thr Arg Gly
                165                 170                 175

Leu Asp Phe Ala Cys Asp Ile Tyr Ile Trp Ala Pro Leu Ala Gly Thr
            180                 185                 190

Cys Gly Val Leu Leu Leu Ser Leu Val Ile Thr Leu Tyr Cys Asn His
        195                 200                 205

Arg Asn Arg Arg Arg Val Cys Lys Cys Pro Arg Pro Val Val Lys Ser
    210                 215                 220

Gly Asp Lys Pro Ser Leu Ser Ala Arg Tyr Val
225                 230                 235
```

<210> 13
<211> 246

54

<212> PRT
<213> Artificial Sequence

<220>
<223> daratumumab scFv sequence

<400> 13

```
        Glu Ile Val Leu Thr Gln Ser Pro Ala Thr Leu Ser Leu Ser Pro Gly
        1               5                   10                  15

        Glu Arg Ala Thr Leu Ser Cys Arg Ala Ser Gln Ser Val Ser Ser Tyr
                    20                  25                  30

        Leu Ala Trp Tyr Gln Gln Lys Pro Gly Gln Ala Pro Arg Leu Leu Ile
                    35                  40                  45

        Tyr Asp Ala Ser Asn Arg Ala Thr Gly Ile Pro Ala Arg Phe Ser Gly
            50                  55                  60

        Ser Gly Ser Gly Thr Asp Phe Thr Leu Thr Ile Ser Ser Leu Glu Pro
        65                  70                  75                  80

        Glu Asp Phe Ala Val Tyr Tyr Cys Gln Gln Arg Ser Asn Trp Pro Pro
                        85                  90                  95
```

```
        Thr Phe Gly Gln Gly Thr Lys Val Glu Ile Lys Arg Ser Gly Gly Gly
                    100                 105             110


        Gly Ser Gly Gly Gly Gly Ser Gly Gly Gly Ser Glu Val Gln Leu
                    115             120             125


        Leu Glu Ser Gly Gly Gly Leu Val Gln Pro Gly Gly Ser Leu Arg Leu
                    130             135             140


        Ser Cys Ala Val Ser Gly Phe Thr Phe Asn Ser Phe Ala Met Ser Trp
        145             150             155             160


        Val Arg Gln Ala Pro Gly Lys Gly Leu Glu Trp Val Ser Ala Ile Ser
                    165             170             175


        Gly Ser Gly Gly Gly Thr Tyr Tyr Ala Asp Ser Val Lys Gly Arg Phe
                    180             185             190


        Thr Ile Ser Arg Asp Asn Ser Lys Asn Thr Leu Tyr Leu Gln Met Asn
                    195             200             205


        Ser Leu Arg Ala Glu Asp Thr Ala Val Tyr Phe Cys Ala Lys Asp Lys
                    210             215             220


        Ile Leu Trp Phe Gly Glu Pro Val Phe Asp Tyr Trp Gly Gln Gly Thr
        225             230             235             240


        Leu Val Thr Val Ser Ser
                        245
```

<210> 14
<211> 244
<212> PRT
<213> Artificial Sequence

<220>
<223> anti-CD10 scFv sequence

<400> 14

```
Asp Ile Val Met Thr Gln Ser Pro Asp Ser Leu Ala Val Ser Leu Gly
1               5               10              15

Glu Arg Ala Thr Ile Asn Cys Ser Val Ser Ser Ser Ile Ser Ser Ser
            20              25              30

Asn Leu His Trp Tyr Gln Gln Lys Pro Gly Gln Pro Pro Lys Leu Leu
            35              40              45

Ile Tyr Gly Thr Ser Asn Leu Ala Ser Gly Val Pro Asp Arg Phe Ser
```

<pre>
                50                      55                      60


        Gly Ser Gly Ser Gly Thr Asp Phe Thr Leu Thr Ile Ser Ser Leu Gln
        65                  70                  75                  80


        Ala Glu Asp Val Ala Val Tyr Tyr Cys Gln Gln Trp Ser Ser Tyr Pro
                        85                  90                  95


        Leu Thr Phe Gly Gln Gly Thr Lys Val Glu Ile Lys Arg Ser Gly Gly
                100                 105                 110


        Gly Gly Ser Gly Gly Gly Gly Ser Gly Gly Gly Gly Ser Glu Val Gln
                115                 120                 125


        Leu Val Glu Ser Gly Gly Gly Val Val Gln Pro Gly Arg Ser Leu Arg
                130                 135                 140


        Leu Ser Cys Ala Ala Ser Gly Phe Thr Phe Ser Ser Phe Gly Met His
        145                 150                 155                 160


        Trp Val Arg Gln Ala Pro Gly Lys Gly Leu Glu Trp Val Ala Tyr Ile
                        165                 170                 175


        Ser Gly Gly Ser Tyr Thr Ile Tyr Tyr Ala Asp Thr Val Lys Gly Arg
                        180                 185                 190


        Phe Thr Ile Ser Arg Asp Asn Ser Lys Asn Thr Leu Tyr Leu Gln Met
                195                 200                 205


        Asn Ser Leu Arg Ala Glu Asp Thr Ala Val Tyr Tyr Cys Ala Arg Ser
                210                 215                 220


        Tyr Gly Asn Phe Trp Tyr Phe Asp Val Trp Gly Gln Gly Thr Thr Val
        225                 230                 235                 240


        Thr Val Ser Ser
</pre>

<210> 15
<211> 135
<212> PRT
<213> Artificial Sequence

<220>
<223> truncated APRIL sequence

<400> 15

<pre>
        Ser Val Leu His Leu Val Pro Ile Asn Ala Thr Ser Lys Asp Asp Ser
        1                   5                   10                  15
</pre>

```
Asp Val Thr Glu Val Met Trp Gln Pro Ala Leu Arg Arg Gly Arg Gly
            20              25              30

Leu Gln Ala Gln Gly Tyr Gly Val Arg Ile Gln Asp Ala Gly Val Tyr
            35              40              45

Leu Leu Tyr Ser Gln Val Leu Phe Gln Asp Val Thr Phe Thr Met Gly
            50              55              60

Gln Val Val Ser Arg Glu Gly Gln Gly Arg Gln Glu Thr Leu Phe Arg
65              70              75              80

Cys Ile Arg Ser Met Pro Ser His Pro Asp Arg Ala Tyr Asn Ser Cys
                85              90              95

Tyr Ser Ala Gly Val Phe His Leu His Gln Gly Asp Ile Leu Ser Val
            100             105             110

Ile Ile Pro Arg Ala Arg Ala Lys Leu Asn Leu Ser Pro His Gly Thr
            115             120             125

Phe Leu Gly Phe Val Lys Leu
            130             135
```

<210> 16
<211> 246
<212> PRT
<213> Artificial Sequence

<220>
<223> anti-BCMA scFv sequence

<400> 16

```
Asp Ile Val Leu Thr Gln Ser Pro Pro Ser Leu Ala Met Ser Leu Gly
1               5               10              15

Lys Arg Ala Thr Ile Ser Cys Arg Ala Ser Glu Ser Val Thr Ile Leu
            20              25              30

Gly Ser His Leu Ile His Trp Tyr Gln Gln Lys Pro Gly Gln Pro Pro
            35              40              45

Thr Leu Leu Ile Gln Leu Ala Ser Asn Val Gln Thr Gly Val Pro Ala
            50              55              60

Arg Phe Ser Gly Ser Gly Ser Arg Thr Asp Phe Thr Leu Thr Ile Asp
65              70              75              80
```

Pro Val Glu Glu Asp Asp Val Ala Val Tyr Tyr Cys Leu Gln Ser Arg
                85                      90                      95

Thr Ile Pro Arg Thr Phe Gly Gly Gly Thr Lys Leu Glu Ile Lys Gly
            100                 105                 110

Ser Thr Ser Gly Ser Gly Lys Pro Gly Ser Gly Glu Gly Ser Thr Lys
            115                 120                 125

Gly Gln Ile Gln Leu Val Gln Ser Gly Pro Glu Leu Lys Lys Pro Gly
    130                 135                 140

Glu Thr Val Lys Ile Ser Cys Lys Ala Ser Gly Tyr Thr Phe Thr Asp
145                 150                 155                 160

Tyr Ser Ile Asn Trp Val Lys Arg Ala Pro Gly Lys Gly Leu Lys Trp
                165                 170                 175

Met Gly Trp Ile Asn Thr Glu Thr Arg Glu Pro Ala Tyr Ala Tyr Asp
            180                 185                 190

Phe Arg Gly Arg Phe Ala Phe Ser Leu Glu Thr Ser Ala Ser Thr Ala
        195                 200                 205

Tyr Leu Gln Ile Asn Asn Leu Lys Tyr Glu Asp Thr Ala Thr Tyr Phe
    210                 215                 220

Cys Ala Leu Asp Tyr Ser Tyr Ala Met Asp Tyr Trp Gly Gln Gly Thr
225                 230                 235                 240

Ser Val Thr Val Ser Ser
                245

<210> 17
<211> 246
<212> PRT
<213> Artificial Sequence

<220>
<223> anti-TACl scFv sequence

<400> 17

Asp Ile Val Met Thr Gln Ser Gln Lys Phe Met Ser Thr Thr Val Gly
1               5                   10                  15

Asp Arg Val Ser Ile Thr Cys Lys Ala Ser Gln Asn Val Gly Thr Ala
            20                  25                  30

```
Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
        50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Ser Tyr Arg Thr
                85                  90                  95

Phe Gly Gly Gly Thr Lys Leu Glu Ile Lys Arg Ser Gly Gly Gly Gly
            100                 105                 110

Ser Gly Gly Gly Gly Ser Gly Gly Gly Ser Gln Val Thr Leu Lys
            115                 120                 125

Glu Ser Gly Pro Gly Met Leu Gln Pro Ser Gln Thr Leu Ser Leu Thr
        130                 135                 140

Cys Ser Phe Ser Gly Phe Ser Leu Ser Thr Phe Gly Met Gly Val Gly
145                 150                 155                 160

Trp Ile Arg Gln Pro Ser Gly Lys Gly Leu Glu Trp Leu Ala His Ile
                165                 170                 175

Trp Trp Asp Asp Ala Gln Tyr Ser Asn Pro Ala Leu Arg Ser Arg Leu
            180                 185                 190

Thr Ile Ser Lys Asp Thr Ser Lys Asn Gln Val Phe Leu Lys Ile Ala
            195                 200                 205

Asn Val Asp Thr Ala Asp Thr Ala Thr Tyr Tyr Cys Ser Arg Ile His
    210                 215                 220

Ser Tyr Tyr Ser Tyr Asp Glu Gly Phe Ala Tyr Trp Gly Gln Gly Thr
225                 230                 235                 240

Leu Val Thr Val Ser Ser
                245
```

<210> 18
<211> 250
<212> PRT
<213> Artificial Sequence

<220>

<223> anti-CD22 scFv sequence

<400> 18

```
Asp Val Gln Val Thr Gln Ser Pro Ser Ser Leu Ser Ala Ser Val Gly
1               5                   10                  15

Asp Arg Val Thr Ile Thr Cys Arg Ser Ser Gln Ser Leu Ala Asn Ser
        20                  25                  30

Tyr Gly Asn Thr Phe Leu Ser Trp Tyr Leu His Lys Pro Gly Lys Ala
        35                  40                  45

Pro Gln Leu Leu Ile Tyr Gly Ile Ser Asn Arg Phe Ser Gly Val Pro
    50                  55                  60

Asp Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Tyr Cys Leu Gln Gly
            85                  90                  95

Thr His Gln Pro Tyr Thr Phe Gly Gln Gly Thr Lys Val Glu Ile Lys
            100                 105                 110

Arg Ser Gly Gly Gly Gly Ser Gly Gly Gly Gly Ser Gly Gly Gly Gly
        115                 120                 125

Ser Glu Val Gln Leu Val Gln Ser Gly Ala Glu Val Lys Lys Pro Gly
        130                 135                 140

Ala Ser Val Lys Val Ser Cys Lys Ala Ser Gly Tyr Arg Phe Thr Asn
145             150                 155                 160

Tyr Trp Ile His Trp Val Arg Gln Ala Pro Gly Gln Gly Leu Glu Trp
                165                 170                 175

Ile Gly Gly Ile Asn Pro Gly Asn Asn Tyr Ala Thr Tyr Arg Arg Lys
            180                 185                 190

Phe Gln Gly Arg Val Thr Met Thr Ala Asp Thr Ser Thr Ser Thr Val
        195                 200                 205

Tyr Met Glu Leu Ser Ser Leu Arg Ser Glu Asp Thr Ala Val Tyr Tyr
    210                 215                 220

Cys Thr Arg Glu Gly Tyr Gly Asn Tyr Gly Ala Trp Phe Ala Tyr Trp
225                 230                 235                 240

        Gly Gln Gly Thr Leu Val Thr Val Ser Ser
                    245                 250
```

<210> 19
<211> 5
<212> PRT
<213> Artificial Sequence

<220>
<223> heavy chain variable region (VH) complementarity determining region (CDR) CDR1

<400> 19

```
Asn Tyr Trp Ile His
1               5
```

<210> 20
<211> 17
<212> PRT
<213> Artificial Sequence

<220>
<223> heavy chain variable region (VH) CDR2

<400> 20

```
Gly Ile Asn Pro Gly Asn Asn Tyr Ala Thr Tyr Arg Arg Lys Phe Gln
1               5                   10                  15

Gly
```

<210> 21
<211> 12
<212> PRT
<213> Artificial Sequence

<220>
<223> heavy chain variable region (VH) CDR3

<400> 21

```
Glu Gly Tyr Gly Asn Tyr Gly Ala Trp Phe Ala Tyr
1               5                   10
```

<210> 22
<211> 16
<212> PRT
<213> Artificial Sequence

<220>
<223> light chain variable region (VL) CDR1

<400> 22

```
Arg Ser Ser Gln Ser Leu Ala Asn Ser Tyr Gly Asn Thr Phe Leu Ser
1               5                   10                  15
```

<210> 23
<211> 7
<212> PRT

<213> Artificial Sequence

<220>
<223> light chain variable region (VL) CDR2

<400> 23

```
Gly Ile Ser Asn Arg Phe Ser
1               5
```

<210> 24
<211> 9
<212> PRT
<213> Artificial Sequence

<220>
<223> light chain variable region (VL) CDR3

<400> 24

```
Leu Gln Gly Thr His Gln Pro Tyr Thr
1               5
```

<210> 25
<211> 121
<212> PRT
<213> Artificial Sequence

<220>
<223> VH sequence from Inotuzumab

<400> 25

```
Glu Val Gln Leu Val Gln Ser Gly Ala Glu Val Lys Lys Pro Gly Ala
1               5                   10                  15

Ser Val Lys Val Ser Cys Lys Ala Ser Gly Tyr Arg Phe Thr Asn Tyr
            20                  25                  30

Trp Ile His Trp Val Arg Gln Ala Pro Gly Gln Gly Leu Glu Trp Ile
            35                  40                  45

Gly Gly Ile Asn Pro Gly Asn Asn Tyr Ala Thr Tyr Arg Arg Lys Phe
    50                  55                  60

Gln Gly Arg Val Thr Met Thr Ala Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95
```

```
        Thr Arg Glu Gly Tyr Gly Asn Tyr Gly Ala Trp Phe Ala Tyr Trp Gly
                    100                 105                 110

        Gln Gly Thr Leu Val Thr Val Ser Ser
                    115                 120
```

<210> 26
<211> 112
<212> PRT
<213> Artificial Sequence

<220>
<223> VL sequence from Inotuzumab

<400> 26

```
        Asp Val Gln Val Thr Gln Ser Pro Ser Ser Leu Ser Ala Ser Val Gly
        1               5                   10                  15

        Asp Arg Val Thr Ile Thr Cys Arg Ser Ser Gln Ser Leu Ala Asn Ser
                    20                  25                  30

        Tyr Gly Asn Thr Phe Leu Ser Trp Tyr Leu His Lys Pro Gly Lys Ala
                    35                  40                  45

        Pro Gln Leu Leu Ile Tyr Gly Ile Ser Asn Arg Phe Ser Gly Val Pro
            50                  55                  60

        Asp Arg Phe Ser Gly Ser Gly Ser Gly Thr Asp Phe Thr Leu Thr Ile
        65                  70                  75                  80

        Ser Ser Leu Gln Pro Glu Asp Phe Ala Thr Tyr Tyr Cys Leu Gln Gly
                        85                  90                  95

        Thr His Gln Pro Tyr Thr Phe Gly Gln Gly Thr Lys Val Glu Ile Lys
                    100                 105                 110
```

<210> 27
<211> 5
<212> PRT
<213> Artificial Sequence

<220>
<223> heavy chain variable region (VH) complementarity determining region (CDR) CDR1

<400> 27

```
                        Asn Tyr Trp Ile Asn
                        1               5
```

<210> 28
<211> 17

<212> PRT
<213> Artificial Sequence

<220>
<223> heavy chain variable region (VH) CDR2

<400> 28

Asn Ile Tyr Pro Ser Asp Ser Phe Thr Asn Tyr Asn Gln Lys Phe Lys
1               5                   10                  15

Asp

<210> 29
<211> 11
<212> PRT
<213> Artificial Sequence

<220>
<223> heavy chain variable region (VH) CDR3

<400> 29

Asp Thr Gln Glu Arg Ser Trp Tyr Phe Asp Val
1               5                   10

<210> 30
<211> 16
<212> PRT
<213> Artificial Sequence

<220>
<223> light chain variable region (VL) CDR1

<400> 30

Arg Ser Ser Gln Ser Leu Val His Ser Asn Gly Asn Thr Tyr Leu His
1               5                   10                  15

<210> 31
<211> 7
<212> PRT
<213> Artificial Sequence

<220>
<223> light chain variable region (VL) CDR2

<400> 31

Lys Val Ser Asn Arg Phe Ser
1               5

<210> 32
<211> 9
<212> PRT
<213> Artificial Sequence

<220>
<223> light chain variable region (VL) CDR3

<400> 32

```
                    Ser Gln Ser Thr His Val Pro Trp Thr
                    1                   5
```

<210> 33
<211> 232
<212> PRT
<213> Artificial Sequence

<220>
<223> Murine CD22ALAb scFv sequence

<400> 33

```
        Gln Val Gln Leu Gln Gln Pro Gly Ala Glu Leu Val Arg Pro Gly Ala
        1               5                   10                  15


        Ser Val Lys Leu Ser Cys Lys Ala Ser Gly Tyr Thr Phe Thr Asn Tyr
                    20                  25                  30


        Trp Ile Asn Trp Val Lys Gln Arg Pro Gly Gln Gly Leu Glu Trp Ile
                    35                  40                  45


        Gly Asn Ile Tyr Pro Ser Asp Ser Phe Thr Asn Tyr Asn Gln Lys Phe
                    50                  55                  60


        Lys Asp Lys Ala Thr Leu Thr Val Asp Lys Ser Ser Ser Thr Ala Tyr
        65                  70                  75                  80


        Met Gln Leu Ser Ser Pro Thr Ser Glu Asp Ser Ala Val Tyr Tyr Cys
                        85                  90                  95


        Thr Arg Asp Thr Gln Glu Arg Ser Trp Tyr Phe Asp Val Trp Gly Ala
                    100                 105                 110


        Gly Thr Thr Val Thr Val Ser Ser Asp Val Val Met Thr Gln Thr Pro
                    115                 120                 125


        Leu Ser Leu Pro Val Ser Leu Gly Asp Gln Ala Ser Ile Ser Cys Arg
                    130                 135                 140


        Ser Ser Gln Ser Leu Val His Ser Asn Gly Asn Thr Tyr Leu His Trp
        145                 150                 155                 160


        Tyr Leu Gln Lys Pro Gly Gln Ser Pro Lys Leu Leu Ile Tyr Lys Val
                        165                 170                 175
```

```
Ser Asn Arg Phe Ser Gly Val Pro Asp Arg Phe Ser Gly Ser Gly Ser
        180                 185                 190

Gly Thr Asp Phe Thr Leu Lys Ile Ser Arg Val Glu Ala Glu Asp Leu
        195                 200                 205

Gly Leu Tyr Phe Cys Ser Gln Ser Thr His Val Pro Trp Thr Phe Gly
        210                 215                 220

Gly Gly Thr Lys Leu Glu Ile Lys
225                 230
```

<210> 34
<211> 232
<212> PRT
<213> Artificial Sequence

<220>
<223> Humanised CD22ALAb scFv sequence

<400> 34

```
Glu Val Gln Leu Val Glu Ser Gly Ala Glu Val Lys Lys Pro Gly Ser
1               5               10              15

Ser Val Lys Val Ser Cys Lys Ala Ser Gly Tyr Thr Phe Thr Asn Tyr
        20              25              30

Trp Ile Asn Trp Val Arg Gln Ala Pro Gly Gln Gly Leu Glu Trp Ile
        35              40              45

Gly Asn Ile Tyr Pro Ser Asp Ser Phe Thr Asn Tyr Asn Gln Lys Phe
    50              55              60

Lys Asp Arg Ala Thr Leu Thr Val Asp Lys Ser Thr Ser Thr Ala Tyr
65              70              75              80

Leu Glu Leu Arg Asn Leu Arg Ser Asp Asp Thr Ala Val Tyr Tyr Cys
            85              90              95

Thr Arg Asp Thr Gln Glu Arg Ser Trp Tyr Phe Asp Val Trp Gly Gln
        100             105             110

Gly Thr Leu Val Thr Val Ser Ser Asp Ile Val Met Thr Gln Ser Pro
        115             120             125

Ala Thr Leu Ser Val Ser Pro Gly Glu Arg Ala Thr Leu Ser Cys Arg
        130             135             140
```

```
Ser Ser Gln Ser Leu Val His Ser Asn Gly Asn Thr Tyr Leu His Trp
145             150             155             160

Tyr Gln Gln Lys Pro Gly Gln Ala Pro Arg Leu Leu Ile Tyr Lys Val
            165             170             175

Ser Asn Arg Phe Ser Gly Val Pro Ala Arg Phe Ser Gly Ser Gly Ser
            180             185             190

Gly Val Glu Phe Thr Leu Thr Ile Ser Ser Leu Gln Ser Glu Asp Phe
            195             200             205

Ala Val Tyr Tyr Cys Ser Gln Ser Thr His Val Pro Trp Thr Phe Gly
    210             215             220

Gln Gly Thr Arg Leu Glu Ile Lys
225             230
```

<210> 35
<211> 120
<212> PRT
<213> Artificial Sequence

<220>
<223> Murine CD22ALAb VH sequence

<400> 35

```
Gln Val Gln Leu Gln Gln Pro Gly Ala Glu Leu Val Arg Pro Gly Ala
1               5               10              15

Ser Val Lys Leu Ser Cys Lys Ala Ser Gly Tyr Thr Phe Thr Asn Tyr
            20              25              30

Trp Ile Asn Trp Val Lys Gln Arg Pro Gly Gln Gly Leu Glu Trp Ile
            35              40              45

Gly Asn Ile Tyr Pro Ser Asp Ser Phe Thr Asn Tyr Asn Gln Lys Phe
    50              55              60

Lys Asp Lys Ala Thr Leu Thr Val Asp Lys Ser Ser Ser Thr Ala Tyr
65              70              75              80

Met Gln Leu Ser Ser Pro Thr Ser Glu Asp Ser Ala Val Tyr Tyr Cys
            85              90              95

Thr Arg Asp Thr Gln Glu Arg Ser Trp Tyr Phe Asp Val Trp Gly Ala
            100             105             110

Gly Thr Thr Val Thr Val Ser Ser

                    115                 120
```

<210> 36
<211> 120
<212> PRT
<213> Artificial Sequence

<220>
<223> Humanised CD22ALAb VH sequence

<400> 36

```
Glu Val Gln Leu Val Glu Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Trp Ile Asn Trp Val Arg Gln Ala Pro Gly Gln Gly Leu Glu Trp Ile
            35                  40                  45

Gly Asn Ile Tyr Pro Ser Asp Ser Phe Thr Asn Tyr Asn Gln Lys Phe
    50                  55                  60

Lys Asp Arg Ala Thr Leu Thr Val Asp Lys Ser Thr Ser Thr Ala Tyr
65                  70                  75                  80

Leu Glu Leu Arg Asn Leu Arg Ser Asp Asp Thr Ala Val Tyr Tyr Cys
                85                  90                  95

Thr Arg Asp Thr Gln Glu Arg Ser Trp Tyr Phe Asp Val Trp Gly Gln
            100                 105                 110

Gly Thr Leu Val Thr Val Ser Ser
            115                 120
```

<210> 37
<211> 112
<212> PRT
<213> Artificial Sequence

<220>
<223> Murine CD22ALAb VL sequence

<400> 37

```
Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30
```

```
Asn Gly Asn Thr Tyr Leu His Trp Tyr Leu Gln Lys Pro Gly Gln Ser
        35              40              45

Pro Lys Leu Leu Ile Tyr Lys Val Ser Asn Arg Phe Ser Gly Val Pro
        50              55              60

Asp Arg Phe Ser Gly Ser Gly Ser Gly Thr Asp Phe Thr Leu Lys Ile
65              70              75              80

Ser Arg Val Glu Ala Glu Asp Leu Gly Leu Tyr Phe Cys Ser Gln Ser
                85              90              95

Thr His Val Pro Trp Thr Phe Gly Gly Gly Thr Lys Leu Glu Ile Lys
                100             105             110
```

<210> 38
<211> 112
<212> PRT
<213> Artificial Sequence

<220>
<223> Humanised CD22ALAb VL sequence

<400> 38

```
Asp Ile Val Met Thr Gln Ser Pro Ala Thr Leu Ser Val Ser Pro Gly
1               5               10              15

Glu Arg Ala Thr Leu Ser Cys Arg Ser Ser Gln Ser Leu Val His Ser
        20              25              30

Asn Gly Asn Thr Tyr Leu His Trp Tyr Gln Gln Lys Pro Gly Gln Ala
        35              40              45

Pro Arg Leu Leu Ile Tyr Lys Val Ser Asn Arg Phe Ser Gly Val Pro
        50              55              60

Ala Arg Phe Ser Gly Ser Gly Ser Gly Val Glu Phe Thr Leu Thr Ile
65              70              75              80

Ser Ser Leu Gln Ser Glu Asp Phe Ala Val Tyr Tyr Cys Ser Gln Ser
                85              90              95

Thr His Val Pro Trp Thr Phe Gly Gln Gly Thr Arg Leu Glu Ile Lys
                100             105             110
```

<210> 39
<211> 234
<212> PRT
<213> Artificial Sequence

<220>
<223> spacer sequence, hinge-CH2CH3 of human IgG1

<400> 39

```
Ala Glu Pro Lys Ser Pro Asp Lys Thr His Thr Cys Pro Pro Cys Pro
1               5               10              15

Ala Pro Pro Val Ala Gly Pro Ser Val Phe Leu Phe Pro Pro Lys Pro
            20              25              30

Lys Asp Thr Leu Met Ile Ala Arg Thr Pro Glu Val Thr Cys Val Val
            35              40              45

Val Asp Val Ser His Glu Asp Pro Glu Val Lys Phe Asn Trp Tyr Val
    50              55              60

Asp Gly Val Glu Val His Asn Ala Lys Thr Lys Pro Arg Glu Glu Gln
65              70              75              80

Tyr Asn Ser Thr Tyr Arg Val Val Ser Val Leu Thr Val Leu His Gln
            85              90              95

Asp Trp Leu Asn Gly Lys Glu Tyr Lys Cys Lys Val Ser Asn Lys Ala
            100             105             110

Leu Pro Ala Pro Ile Glu Lys Thr Ile Ser Lys Ala Lys Gly Gln Pro
    115             120             125

Arg Glu Pro Gln Val Tyr Thr Leu Pro Pro Ser Arg Asp Glu Leu Thr
    130             135             140

Lys Asn Gln Val Ser Leu Thr Cys Leu Val Lys Gly Phe Tyr Pro Ser
145             150             155             160

Asp Ile Ala Val Glu Trp Glu Ser Asn Gly Gln Pro Glu Asn Asn Tyr
            165             170             175

Lys Thr Thr Pro Pro Val Leu Asp Ser Asp Gly Ser Phe Phe Leu Tyr
            180             185             190

Ser Lys Leu Thr Val Asp Lys Ser Arg Trp Gln Gln Gly Asn Val Phe
    195             200             205

Ser Cys Ser Val Met His Glu Ala Leu His Asn His Tyr Thr Gln Lys
    210             215             220

Ser Leu Ser Leu Ser Pro Gly Lys Lys Asp
225             230
```

<210> 40
<211> 46

75

<212> PRT
<213> Artificial Sequence

<220>
<223> spacer sequence, human CD8 stalk

<400> 40

```
Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly
            20                  25                  30

Gly Ala Val His Thr Arg Gly Leu Asp Phe Ala Cys Asp Ile
            35                  40                  45
```

<210> 41
<211> 20
<212> PRT
<213> Artificial Sequence

<220>
<223> spacer sequence, human IgG1 hinge

<400> 41

```
Ala Glu Pro Lys Ser Pro Asp Lys Thr His Thr Cys Pro Pro Cys Pro
1               5                   10                  15

Lys Asp Pro Lys
            20
```

<210> 42
<211> 112
<212> PRT
<213> Artificial Sequence

<220>
<223> CD3-zeta endodomain

<400> 42

```
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
            35                  40                  45
```

Pro Arg Arg Lys Asn Pro Gln Glu Gly Leu Tyr Asn Glu Leu Gln Lys
50          55              60

Asp Lys Met Ala Glu Ala Tyr Ser Glu Ile Gly Met Lys Gly Glu Arg
65          70              75              80

Arg Arg Gly Lys Gly His Asp Gly Leu Tyr Gln Gly Leu Ser Thr Ala
85              90              95

Thr Lys Asp Thr Tyr Asp Ala Leu His Met Gln Ala Leu Pro Pro Arg
100             105             110

<210> 43
<211> 368
<212> PRT
<213> Artificial Sequence

<220>
<223> 4-1BB and CD3-zeta endodomains

<400> 43

Met Gly Asn Ser Cys Tyr Asn Ile Val Ala Thr Leu Leu Leu Val Leu
1           5               10              15

Asn Phe Glu Arg Thr Arg Ser Leu Gln Asp Pro Cys Ser Asn Cys Pro
20              25              30

Ala Gly Thr Phe Cys Asp Asn Asn Arg Asn Gln Ile Cys Ser Pro Cys
35              40              45

Pro Pro Asn Ser Phe Ser Ser Ala Gly Gly Gln Arg Thr Cys Asp Ile
50              55              60

Cys Arg Gln Cys Lys Gly Val Phe Arg Thr Arg Lys Glu Cys Ser Ser
65              70              75              80

Thr Ser Asn Ala Glu Cys Asp Cys Thr Pro Gly Phe His Cys Leu Gly
85              90              95

Ala Gly Cys Ser Met Cys Glu Gln Asp Cys Lys Gln Gly Gln Glu Leu
100             105             110

Thr Lys Lys Gly Cys Lys Asp Cys Cys Phe Gly Thr Phe Asn Asp Gln
115             120             125

Lys Arg Gly Ile Cys Arg Pro Trp Thr Asn Cys Ser Leu Asp Gly Lys
130             135             140

```
Ser Val Leu Val Asn Gly Thr Lys Glu Arg Asp Val Val Cys Gly Pro
145             150             155             160

Ser Pro Ala Asp Leu Ser Pro Gly Ala Ser Ser Val Thr Pro Pro Ala
            165             170             175

Pro Ala Arg Glu Pro Gly His Ser Pro Gln Ile Ile Ser Phe Phe Leu
            180             185             190

Ala Leu Thr Ser Thr Ala Leu Leu Phe Leu Leu Phe Phe Leu Thr Leu
            195             200             205

Arg Phe Ser Val Val Lys Arg Gly Arg Lys Lys Leu Leu Tyr Ile Phe
    210             215             220

Lys Gln Pro Phe Met Arg Pro Val Gln Thr Thr Gln Glu Glu Asp Gly
225             230             235             240

Cys Ser Cys Arg Phe Pro Glu Glu Glu Glu Gly Gly Cys Glu Leu Arg
            245             250             255

Val Lys Phe Ser Arg Ser Ala Asp Ala Pro Ala Tyr Gln Gln Gly Gln
            260             265             270

Asn Gln Leu Tyr Asn Glu Leu Asn Leu Gly Arg Arg Glu Glu Tyr Asp
            275             280             285

Val Leu Asp Lys Arg Arg Gly Arg Asp Pro Glu Met Gly Gly Lys Pro
    290             295             300

Gln Arg Arg Lys Asn Pro Gln Glu Gly Leu Tyr Asn Glu Leu Gln Lys
305             310             315             320

Asp Lys Met Ala Glu Ala Tyr Ser Glu Ile Gly Met Lys Gly Glu Arg
            325             330             335

Arg Arg Gly Lys Gly His Asp Gly Leu Tyr Gln Gly Leu Ser Thr Ala
            340             345             350

Thr Lys Asp Thr Tyr Asp Ala Leu His Met Gln Ala Leu Pro Pro Arg
            355             360             365
```

<210> 44
<211> 152
<212> PRT
<213> Artificial Sequence

<220>

<223> CD28 and CD3-zeta endodomains

<400> 44

```
      Ser Lys Arg Ser Arg Leu Leu His Ser Asp Tyr Met Asn Met Thr Pro
      1               5                   10                  15

      Arg Arg Pro Gly Pro Thr Arg Lys His Tyr Gln Pro Tyr Ala Pro Pro
                  20                  25                  30

      Arg Asp Phe Ala Ala Tyr Arg Ser Arg Val Lys Phe Ser Arg Ser Ala
                  35                  40                  45

      Asp Ala Pro Ala Tyr Gln Gln Gly Gln Asn Gln Leu Tyr Asn Glu Leu
                  50                  55                  60

      Asn Leu Gly Arg Arg Glu Glu Tyr Asp Val Leu Asp Lys Arg Arg Gly
      65                  70                  75                  80

      Arg Asp Pro Glu Met Gly Gly Lys Pro Arg Arg Lys Asn Pro Gln Glu
                      85                  90                  95

      Gly Leu Tyr Asn Glu Leu Gln Lys Asp Lys Met Ala Glu Ala Tyr Ser
                  100                 105                 110

      Glu Ile Gly Met Lys Gly Glu Arg Arg Arg Gly Lys Gly His Asp Gly
                  115                 120                 125

      Leu Tyr Gln Gly Leu Ser Thr Ala Thr Lys Asp Thr Tyr Asp Ala Leu
          130                 135                 140

      His Met Gln Ala Leu Pro Pro Arg
      145                 150
```

<210> 45
<211> 188
<212> PRT
<213> Artificial Sequence

<220>
<223> CD28, OX40 and CD3-zeta endodomains

<400> 45

```
Ser Lys Arg Ser Arg Leu Leu His Ser Asp Tyr Met Asn Met Thr Pro
1               5               10              15

Arg Arg Pro Gly Pro Thr Arg Lys His Tyr Gln Pro Tyr Ala Pro Pro
            20              25              30

Arg Asp Phe Ala Ala Tyr Arg Ser Arg Asp Gln Arg Leu Pro Pro Asp
            35              40              45

Ala His Lys Pro Pro Gly Gly Gly Ser Phe Arg Thr Pro Ile Gln Glu
    50              55              60

Glu Gln Ala Asp Ala His Ser Thr Leu Ala Lys Ile Arg Val Lys Phe
65              70              75              80

Ser Arg Ser Ala Asp Ala Pro Ala Tyr Gln Gln Gly Gln Asn Gln Leu
            85              90              95

Tyr Asn Glu Leu Asn Leu Gly Arg Arg Glu Glu Tyr Asp Val Leu Asp
            100             105             110

Lys Arg Arg Gly Arg Asp Pro Glu Met Gly Gly Lys Pro Arg Arg Lys
            115             120             125

Asn Pro Gln Glu Gly Leu Tyr Asn Glu Leu Gln Lys Asp Lys Met Ala
    130             135             140

Glu Ala Tyr Ser Glu Ile Gly Met Lys Gly Glu Arg Arg Arg Gly Lys
145             150             155             160

Gly His Asp Gly Leu Tyr Gln Gly Leu Ser Thr Ala Thr Lys Asp Thr
            165             170             175

Tyr Asp Ala Leu His Met Gln Ala Leu Pro Pro Arg
            180             185
```

<210> 46
<211> 20
<212> PRT
<213> Artificial Sequence

<220>
<223> cleavage site, 2A-like sequence

<400> 46

```
Tyr His Ala Asp Tyr Tyr Lys Gln Arg Leu Ile His Asp Val Glu Met
1               5                   10                  15
```

```
Asn Pro Gly Pro
            20
```

<210> 47
<211> 20
<212> PRT
<213> Artificial Sequence

<220>
<223> cleavage site, 2A-like sequence

<400> 47

```
His Tyr Ala Gly Tyr Phe Ala Asp Leu Leu Ile His Asp Ile Glu Thr
1               5                   10                  15
```

```
Asn Pro Gly Pro
            20
```

<210> 48
<211> 20
<212> PRT
<213> Artificial Sequence

<220>
<223> cleavage site, 2A-like sequence

<400> 48

```
Gln Cys Thr Asn Tyr Ala Leu Leu Lys Leu Ala Gly Asp Val Glu Ser
1               5                   10                  15
```

```
Asn Pro Gly Pro
            20
```

<210> 49
<211> 19
<212> PRT
<213> Artificial Sequence

<220>
<223> cleavage site, 2A-like sequence

<400> 49

```
Ala Thr Asn Phe Ser Leu Leu Lys Gln Ala Gly Asp Val Glu Glu Asn
1               5                   10                  15
```

```
Pro Gly Pro
```

<210> 50
<211> 19
<212> PRT
<213> Artificial Sequence

<220>
<223> cleavage site, 2A-like sequence

<400> 50

```
Ala Ala Arg Gln Met Leu Leu Leu Leu Ser Gly Asp Val Glu Thr Asn
1               5                   10                  15

Pro Gly Pro
```

<210> 51
<211> 20
<212> PRT
<213> Artificial Sequence

<220>
<223> cleavage site, 2A-like sequence

<400> 51

```
Arg Ala Glu Gly Arg Gly Ser Leu Leu Thr Cys Gly Asp Val Glu Glu
1               5                   10                  15

Asn Pro Gly Pro
            20
```

<210> 52
<211> 20
<212> PRT
<213> Artificial Sequence

<220>
<223> cleavage site, 2A-like sequence

<400> 52

```
Thr Arg Ala Glu Ile Glu Asp Glu Leu Ile Arg Ala Gly Ile Glu Ser
1               5                   10                  15

Asn Pro Gly Pro
            20
```

<210> 53
<211> 20
<212> PRT
<213> Artificial Sequence

<220>
<223> cleavage site, 2A-like sequence

<400> 53

```
        Thr Arg Ala Glu Ile Glu Asp Glu Leu Ile Arg Ala Asp Ile Glu Ser
        1               5                   10                  15

        Asn Pro Gly Pro
                    20
```

<210> 54
<211> 20
<212> PRT
<213> Artificial Sequence

<220>
<223> cleavage site, 2A-like sequence

<400> 54

```
        Ala Lys Phe Gln Ile Asp Lys Ile Leu Ile Ser Gly Asp Val Glu Leu
        1               5                   10                  15

        Asn Pro Gly Pro
                    20
```

<210> 55
<211> 20
<212> PRT
<213> Artificial Sequence

<220>
<223> cleavage site, 2A-like sequence

<400> 55

```
        Ser Ser Ile Ile Arg Thr Lys Met Leu Val Ser Gly Asp Val Glu Glu
        1               5                   10                  15

        Asn Pro Gly Pro
                    20
```

<210> 56
<211> 20
<212> PRT
<213> Artificial Sequence

<220>
<223> cleavage site, 2A-like sequence

<400> 56

```
        Cys Asp Ala Gln Arg Gln Lys Leu Leu Leu Ser Gly Asp Ile Glu Gln
        1               5                   10                  15


        Asn Pro Gly Pro
                    20
```

<210> 57
<211> 20
<212> PRT
<213> Artificial Sequence

<220>
<223> cleavage site, 2A-like sequence

<400> 57

```
        Tyr Pro Ile Asp Phe Gly Gly Phe Leu Val Lys Ala Asp Ser Glu Phe
        1               5                   10                  15


        Asn Pro Gly Pro
                    20
```

<210> 58
<211> 119
<212> PRT
<213> Artificial Sequence

<220>
<223> antibody 1D9-3, VH sequence

<400> 58

Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
                20                  25                  30

Ala Met His Trp Val Arg Gln Ala Pro Gly Lys Gly Leu Glu Trp Val
            35                  40                  45

Ala Arg Ile Arg Ser Lys Ser Ser Asn Tyr Ala Thr Tyr Tyr Ala Asp
        50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Val Asp Tyr Leu Tyr Ala Met Asp Tyr Trp Gly Gln Gly
            100                 105                 110

Thr Ser Val Thr Val Ser Ser
            115

<210> 59
<211> 107
<212> PRT
<213> Artificial Sequence

<220>
<223> antibody 1D9-3, VL sequence

<400> 59

Asp Ile Val Met Thr Gln Ser Gln Lys Phe Met Ser Thr Ser Val Gly
1               5                   10                  15

Asp Arg Val Ser Ile Thr Cys Lys Ala Ser Gln Asn Val Arg Thr Ala
                20                  25                  30

Val Ala Trp Tyr Gln Gln Lys Pro Gly Gln Ser Pro Lys Ala Leu Ile

                    35                        40                           45

Tyr Leu Ala Ser Asn Arg His Thr Gly Val Pro Asp Arg Phe Thr Gly
    50              55              60

Ser Gly Ser Gly Thr Asp Phe Thr Leu Thr Ile Ser Asn Val Gln Ser
65              70              75                      80

Glu Asp Leu Ala Asp Tyr Phe Cys Leu Gln His Trp Asn Tyr Pro Phe
                85              90                      95

Thr Phe Gly Ser Gly Thr Lys Leu Glu Ile Lys
            100             105

<210> 60
<211> 120
<212> PRT
<213> Artificial Sequence

<220>
<223> antibody 3B4-13, VH sequence

<400> 60

Gln Val Gln Leu Gln Gln Ser Gly Ala Glu Leu Val Arg Pro Gly Ala
1               5               10                      15

Ser Val Thr Leu Ser Cys Lys Ala Ser Gly Tyr Thr Phe Thr Asp Tyr
            20              25              30

Glu Met His Trp Val Lys Gln Thr Pro Val His Gly Leu Glu Trp Ile
        35              40              45

Gly Ala Ile Asp Pro Glu Thr Gly Ala Thr Ala Tyr Asn Gln Lys Phe
    50              55              60

Lys Gly Lys Ala Ile Leu Thr Ala Asp Lys Ser Ser Ser Thr Ala Tyr
65              70              75                      80

Met Asp Leu Arg Ser Leu Thr Ser Glu Asp Ser Ala Val Tyr Tyr Cys
                85              90                      95

Thr Arg Tyr Asp Tyr Gly Ser Ser Pro Trp Phe Ala Tyr Trp Gly Gln
            100             105                     110

Gly Thr Leu Val Thr Val Ser Ala
            115             120

<210> 61
<211> 109
<212> PRT
<213> Artificial Sequence

<220>
<223> antibody 3B4-13, VL sequence

<400> 61

```
        Gln Ala Val Val Thr Gln Glu Ser Ala Leu Thr Thr Ser Pro Gly Glu
        1               5                   10                  15

        Thr Val Thr Leu Thr Cys Arg Ser Ser Ala Gly Ala Val Thr Thr Ser
                        20                  25                  30

        Asn Tyr Ala Asn Trp Val Gln Glu Lys Pro Asp His Leu Phe Thr Gly
                    35                  40                  45

        Leu Ile Gly Gly Thr Asn Asn Arg Ala Pro Gly Val Pro Ala Arg Phe
                    50                  55                  60

        Ser Gly Ser Leu Ile Gly Asp Lys Ala Ala Leu Thr Ile Thr Gly Ala
        65                  70                  75                  80

        Gln Thr Glu Asp Glu Ala Ile Tyr Phe Cys Ala Leu Trp Asn Ser Asn
                        85                  90                  95

        His Trp Val Phe Gly Gly Gly Thr Lys Leu Thr Val Leu
                        100                 105
```

<210> 62
<211> 119
<212> PRT
<213> Artificial Sequence

<220>
<223> antibody 7G6-6, VH sequence

<400> 62

```
Gln Val Gln Leu Gln Gln Pro Gly Ala Glu Leu Val Met Pro Gly Ala
1               5                   10              15

Ser Val Lys Leu Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Glu Ile Asp Pro Ser Asp Ser Tyr Thr Asn Tyr Asn Gln Lys Phe
    50                  55                  60

Lys Gly Lys Ala Thr Leu Thr Val Asp Lys Ser Ser Ser Thr Ala Tyr

65                  70                  75                  80

Met Gln Leu Ser Ser Leu Thr Ser Glu Asp Ser Ala Val Tyr Tyr Cys
            85                  90                  95

Ala Arg Gly Tyr Tyr Gly Ser Ser Ser Phe Asp Tyr Trp Gly Gln Gly
        100                 105                 110

Thr Thr Leu Thr Val Ser Ser
        115
```

<210> 63
<211> 111
<212> PRT
<213> Artificial Sequence

<220>
<223> antibody 7G6-6, VL sequence

<400> 63

```
Asp Ile Val Met Ser Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
                20                  25                  30

Ser Asn Gln Lys Asn Tyr Leu Ala Trp Tyr Gln Gln Lys Pro Gly Gln
            35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Ser Tyr Thr Phe Gly Gly Gly Thr Lys Leu Glu Ile Lys
            100                 105                 110
```

<210> 64
<211> 120
<212> PRT
<213> Artificial Sequence

<220>
<223> antibody 6C4-6, VH sequence

<400> 64

```
Gln Val Gln Leu Lys Glu Ser Gly Pro Gly Leu Val Ala Pro Ser Gln
1               5               10                  15

Ser Leu Ser Ile Thr Cys Thr Val Ser Gly Phe Ser Leu Thr Ser Tyr
            20              25                  30

Gly Val His Trp Val Arg Gln Pro Pro Gly Lys Gly Leu Glu Trp Leu
        35              40                  45

Val Val Ile Trp Ser Asp Gly Ser Thr Thr Tyr Asn Ser Ala Leu Lys
    50              55                  60

Ser Arg Leu Ser Ile Ser Lys Asp Asn Ser Lys Ser Gln Val Phe Leu
65              70                  75                  80

Lys Met Asn Ser Leu Gln Thr Asp Asp Thr Ala Met Tyr Tyr Cys Ala
            85                  90                  95

Arg His Ala Asp Asp Tyr Gly Phe Ala Trp Phe Ala Tyr Trp Gly Gln
        100             105                 110

Gly Thr Leu Val Thr Val Ser Ala
        115             120
```

<210> 65
<211> 107
<212> PRT
<213> Artificial Sequence

<220>
<223> antibody 6C4-6, VL sequence

<400> 65

```
Asp Ile Gln Met Thr Gln Ser Pro Ala Ser Leu Ser Ala Ser Val Gly
1               5               10                  15

Glu Thr Val Thr Ile Thr Cys Arg Ala Ser Glu Asn Ile Tyr Ser Tyr
            20              25                  30

Leu Ala Trp Tyr Gln Gln Lys Gln Gly Lys Ser Pro Gln Leu Leu Val
            35              40              45

Tyr Asn Ala Lys Thr Leu Ala Glu Gly Val Pro Ser Arg Phe Ser Gly
        50              55              60

Ser Gly Ser Gly Thr Gln Phe Ser Leu Lys Ile Asn Ser Leu Gln Pro
65              70              75                  80

Glu Asp Phe Gly Ser Tyr Tyr Cys Gln His His Tyr Gly Thr Pro Pro
                85                  90                  95

    Thr Phe Gly Gly Gly Thr Lys Leu Glu Ile Lys
            100                 105
```

<210> 66
<211> 121
<212> PRT
<213> Artificial Sequence

<220>
<223> antibody 4D9-12, VH sequence

<400> 66

91

```
Glu Phe Gln Leu Gln Gln Ser Gly Pro Glu Leu Val Lys Pro Gly Ala
1               5               10              15

Ser Val Lys Ile Ser Cys Lys Ala Ser Gly Tyr Ser Phe Thr Asp Tyr
            20              25              30

Asn Met Asn Trp Val Lys Gln Ser Asn Gly Lys Ser Leu Glu Trp Ile
        35              40              45

Gly Val Ile Asn Pro Asn Tyr Gly Thr Thr Ser Tyr Asn Gln Lys Phe
    50              55              60

Lys Gly Lys Ala Thr Leu Thr Val Asp Gln Ser Ser Ser Thr Ala Tyr
65              70              75              80

Met Gln Leu Asn Ser Leu Thr Ser Glu Asp Ser Ala Val Tyr Tyr Cys
            85              90              95

Ala Arg Ser Ser Thr Thr Val Val Asp Trp Tyr Phe Asp Val Trp Gly
        100             105             110

Thr Gly Thr Thr Val Thr Val Ser Ser
        115             120
```

<210> 67
<211> 107
<212> PRT
<213> Artificial Sequence

<220>
<223> antibody 4D9-12, VL sequence

<400> 67

```
Asp Ile Gln Met Thr Gln Ser Pro Ser Ser Leu Ser Ala Ser Leu Gly
1               5               10              15
```

```
Glu Arg Val Ser Leu Thr Cys Arg Ala Ser Gln Glu Ile Ser Gly Tyr
            20                      25                  30

Leu Ser Trp Leu Gln Gln Lys Pro Asp Gly Thr Ile Lys Arg Leu Ile
            35                      40                  45

Tyr Ala Ala Ser Thr Leu Asp Ser Gly Val Pro Lys Arg Phe Ser Gly
        50                      55                  60

Ser Arg Ser Gly Ser Asp Tyr Ser Leu Thr Ile Ser Ser Leu Glu Ser
65                      70                  75                  80

Glu Asp Phe Ala Asp Tyr Tyr Cys Leu Gln Tyr Ala Ser Tyr Pro Phe
                85                      90                  95

Thr Phe Gly Ser Gly Thr Lys Leu Glu Ile Lys
            100                     105
```

<210> 68
<211> 118
<212> PRT
<213> Artificial Sequence

<220>
<223> antibody 5H4-9, VH sequence

<400> 68

```
Gln Val Gln Val Gln Gln Pro Gly Ala Glu Leu Val Arg Pro Gly Thr
1                   5                   10                  15

Ser Val Lys Leu Ser Cys Lys Ala Ser Gly Tyr Thr Phe Thr Arg Tyr
            20                  25                  30

Trp Met Tyr Trp Val Lys Gln Arg Pro Gly Gln Gly Leu Glu Trp Ile
        35                  40                  45

Gly Val Ile Asp Pro Ser Asp Asn Phe Thr Tyr Tyr Asn Gln Lys Phe
    50                  55                  60

Lys Gly Lys Ala Thr Leu Thr Val Asp Thr Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Gly Tyr Gly Ser Ser Tyr Val Gly Tyr Trp Gly Gln Gly Thr
            100                 105                 110

Thr Leu Thr Val Ser Ser


                                    115
```

<210> 69
<211> 113
<212> PRT
<213> Artificial Sequence

<220>
<223> antibody 5H4-9, VL sequence

<400> 69

```
Asp Val Val Met Thr Gln Thr Pro Leu Ser Leu Pro Val Ser Leu Gly
1               5               10              15

Asp Gln Ala Ser Ile Ser Cys Arg Ser Ser Gln Ser Leu Val His Ser
            20              25              30

Asn Gly Asn Thr Tyr Leu His Trp Tyr Leu Gln Lys Pro Gly Gln Ser
        35              40              45

Pro Lys Leu Leu Ile Tyr Lys Val Ser Asn Arg Phe Ser Gly Val Pro
    50              55              60

Asp Arg Phe Ser Gly Ser Gly Ser Gly Thr Asp Phe Thr Leu Lys Ile
65              70              75              80

Ser Arg Val Glu Ala Glu Asp Leu Gly Val Tyr Phe Cys Ser Gln Ser
            85              90              95

Thr His Val Pro Pro Trp Thr Phe Gly Gly Gly Thr Lys Leu Glu Ile
            100             105             110

Lys
```

<210> 70
<211> 124
<212> PRT
<213> Artificial Sequence

<220>
<223> antibody 10C1-D9, VH sequence

<400> 70

```
Gln Val Thr Leu Lys Glu Ser Gly Pro Gly Ile Leu Gln Ser Ser Gln
1               5               10              15

Thr Leu Ser Leu Thr Cys Ser Phe Ser Gly Phe Ser Leu Ser Thr Ser
            20              25              30
```

```
        Asp Met Gly Val Ser Trp Ile Arg Gln Pro Ser Gly Lys Gly Leu Glu
            35              40              45

        Trp Leu Ala His Ile Tyr Trp Asp Asp Asp Lys Arg Tyr Asn Pro Ser
            50              55              60

        Leu Lys Ser Arg Leu Thr Ile Ser Lys Asp Ala Ser Arg Asn Gln Val
            65              70              75              80

        Phe Leu Lys Ile Ala Thr Val Asp Thr Ala Asp Thr Ala Thr Tyr Tyr
                        85              90              95

        Cys Ala Arg Ser Pro Trp Ile Tyr Tyr Gly His Tyr Trp Cys Phe Asp
                100             105             110

        Val Trp Gly Thr Gly Thr Thr Val Thr Val Ser Ser
                115             120
```

<210> 71
<211> 107
<212> PRT
<213> Artificial Sequence

<220>
<223> antibody 10C1-D9, VH sequence

<400> 71

```
        Asp Ile Gln Met Thr Gln Thr Thr Ser Ser Leu Ser Ala Ser Leu Gly
        1           5               10              15

        Asp Arg Val Thr Ile Ser Cys Arg Ala Ser Gln Asp Ile Ser Asn Tyr
                20              25              30

        Leu Asn Trp Tyr Gln Gln Lys Pro Asp Gly Thr Val Lys Leu Leu Ile
            35              40              45

        Tyr Tyr Thr Ser Arg Leu His Ser Gly Val Pro Ser Arg Phe Ser Gly
            50              55              60

        Ser Gly Ser Gly Thr Asp Tyr Ser Leu Thr Ile Ser Asn Leu Glu Gln
        65              70              75              80

        Glu Asp Ile Ala Thr Tyr Phe Cys Gln Gln Gly Asn Thr Leu Pro Phe
                        85              90              95

        Thr Phe Gly Ser Gly Thr Lys Leu Glu Ile Lys
                100             105
```

<210> 72
<211> 123
<212> PRT
<213> Artificial Sequence

<220>
<223> antibody 15G7-2, VH sequence

<400> 72

```
Gln Val Gln Leu Gln Gln Ser Gly Ala Glu Leu Val Lys Pro Gly Ala
1               5                   10                  15

Ser Val Lys Leu Ser Cys Lys Ala Ser Gly Tyr Thr Phe Thr Glu Tyr
            20                  25                  30

Thr Ile His Trp Val Lys Gln Arg Ser Gly Gln Gly Leu Glu Trp Ile
            35                  40                  45

Gly Trp Phe Tyr Pro Gly Ser Gly Ser Ile Lys Tyr Asn Glu Lys Phe
        50                  55                  60

Lys Asp Lys Ala Thr Leu Thr Ala Asp Lys Ser Ser Ser Thr Val Tyr
65                  70                  75                  80

Met Glu Leu Ser Arg Leu Thr Ser Glu Asp Ser Ala Val Tyr Phe Cys
                85                  90                  95

Ala Arg His Gly Asp Gly Tyr Tyr Leu Pro Pro Tyr Tyr Phe Asp Tyr
            100                 105                 110

Trp Gly Gln Gly Thr Thr Leu Thr Val Ser Ser
            115                 120
```

<210> 73
<211> 106
<212> PRT
<213> Artificial Sequence

<220>
<223> antibody 15G7-2, VH sequence

<400> 73

```
Gln Ile Val Leu Thr Gln Ser Pro Ala Ile Met Ser Ala Ser Pro Gly
1               5               10                  15

Glu Lys Val Thr Met Thr Cys Ser Ala Ser Ser Ser Val Ser Tyr Met
            20              25                  30

Tyr Trp Tyr Gln Gln Lys Pro Gly Ser Ser Pro Arg Leu Leu Ile Tyr
        35                  40                  45

Asp Thr Ser Asn Leu Ala Ser Gly Val Pro Val Arg Phe Ser Gly Ser
        50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Ser Tyr Pro Leu Thr
                85                  90                  95

Phe Gly Ala Gly Thr Lys Leu Glu Leu Lys
            100                 105
```

<210> 74
<211> 121
<212> PRT
<213> Artificial Sequence

<220>
<223> antibody 2B12-8, VH sequence

<400> 74

Gln Val Gln Leu Gln Gln Ser Gly Ala Glu Leu Ala Arg Pro Gly Ala
1               5               10              15

Ser Val Lys Leu Ser Cys Lys Ala Ser Gly Tyr Ile Phe Thr Ser Tyr
            20              25              30

Gly Ile Ser Trp Val Lys Gln Arg Thr Gly Gln Gly Leu Glu Trp Ile
            35              40              45

Gly Glu Ile Tyr Pro Arg Ser Gly Asn Thr Tyr Tyr Asn Glu Lys Phe
    50              55              60

Lys Gly Lys Ala Thr Leu Thr Ala Asp Lys Ser Ser Ser Thr Ala Tyr
65              70              75              80

Met Glu Leu Arg Ser Leu Thr Ser Glu Asp Ser Ala Val Tyr Phe Cys
            85              90              95

Ala Arg Pro Ile Tyr Tyr Gly Ser Arg Glu Gly Phe Asp Tyr Trp Gly
        100             105             110

Gln Gly Thr Thr Leu Thr Val Ser Ser
        115             120

<210> 75
<211> 107
<212> PRT
<213> Artificial Sequence

<220>
<223> antibody 2B12-8, VH sequence

<400> 75

```
Asp Ile Val Leu Thr Gln Ser Pro Ala Thr Leu Ser Val Thr Pro Gly
1               5               10              15

Asp Ser Val Ser Leu Ser Cys Arg Ala Ser Gln Ser Ile Ser Thr Asn
        20              25              30

Leu His Trp Tyr Gln Gln Lys Ser His Ala Ser Pro Arg Leu Leu Ile
        35              40              45

Lys Tyr Ala Ser Gln Ser Val Ser Gly Ile Pro Ser Arg Phe Ser Gly
    50              55              60

Ser Gly Ser Gly Thr Asp Phe Thr Leu Ser Ile Asn Ser Val Glu Thr
65              70              75              80

Glu Asp Phe Gly Ile Phe Phe Cys Gln Gln Ser Tyr Ser Trp Pro Tyr
            85              90              95

Thr Phe Gly Gly Gly Thr Lys Leu Glu Ile Lys
        100             105
```

<210> 76
<211> 120
<212> PRT
<213> Artificial Sequence

<220>
<223> antibody 2C4-4, VH sequence

<400> 76

```
Gln Val Gln Leu Gln Gln Pro Gly Ala Glu Leu Val Met Pro Gly Ala
1               5               10              15

Ser Val Lys Leu Ser Cys Lys Ala Ser Gly Tyr Thr Phe Thr Ser Tyr
        20              25              30

Trp Met His Trp Val Lys Gln Arg Pro Gly Gln Gly Leu Glu Trp Ile
        35              40              45

Gly Glu Ile Asp Pro Ser Asp Ser Tyr Thr Asn Tyr Asn Gln Lys Phe
    50              55              60

Lys Gly Lys Ser Thr Leu Thr Val Asp Lys Ser Ser Ser Thr Ala Tyr
65              70              75              80
```

```
Ile Gln Leu Ser Ser Leu Thr Ser Glu Asp Ser Ala Val Tyr Tyr Cys
                85                  90                  95

Ala Arg Trp Ala Ser Tyr Arg Gly Tyr Ala Met Asp Tyr Trp Gly Gln
            100                 105                 110

Gly Thr Ser Val Thr Val Ser Ser
            115                 120
```

<210> 77
<211> 112
<212> PRT
<213> Artificial Sequence

<220>
<223> antibody 2C4-4, VL sequence

<400> 77

```
Asp Val Leu Met Thr Gln Thr Pro Leu Ser Leu Pro Val Ser Leu Gly
1               5                   10                  15

Asp Gln Ala Ser Ile Ser Cys Arg Ser Ser Gln Ser Ile Val His Ser
            20                  25                  30

Asn Gly Asn Thr Tyr Leu Glu Trp Tyr Leu Gln Lys Pro Gly Gln Ser
            35                  40                  45

Pro Lys Leu Leu Ile Tyr Lys Val Ser Asn Arg Phe Ser Gly Val Pro
        50                  55                  60

Asp Arg Phe Ser Gly Ser Glu Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Val Pro Trp Thr Phe Gly Gly Gly Thr Lys Leu Glu Ile Lys
            100                 105                 110
```

<210> 78
<211> 119
<212> PRT
<213> Artificial Sequence

<220>
<223> antibody 3E10-7, VH sequence

<400> 78

Glu Phe Gln Leu Gln Gln Ser Gly Pro Glu Leu Val Lys Pro Gly Ala
1                   5                   10                  15

Ser Val Lys Ile Ser Cys Lys Ala Ser Gly Tyr Ser Phe Thr Asp Tyr
                20                  25                  30

Asn Met Asn Trp Val Lys Gln Ser Asn Gly Lys Ser Leu Glu Trp Ile
        35                  40                  45

Gly Val Ile Asn Pro Asn Tyr Gly Thr Thr Ser Tyr Asn Gln Arg Phe
    50                  55                  60

Lys Gly Lys Ala Thr Leu Thr Val Asp Gln Ser Ser Ser Thr Ala Tyr
65                  70                  75                  80

Met Gln Leu Asn Ser Leu Thr Ser Glu Asp Ser Ala Val Tyr Tyr Cys
                85                  90                  95

Ala Arg Ser Gly Leu Arg Tyr Trp Tyr Phe Asp Val Trp Gly Thr Gly
            100                 105                 110

Thr Thr Val Thr Val Ser Ser
            115

<210> 79
<211> 107
<212> PRT
<213> Artificial Sequence

<220>
<223> antibody 3E10-7, VL sequence

<400> 79

102

```
        Asp Ile Gln Met Thr Gln Ser Pro Ser Ser Leu Ser Ala Ser Leu Gly
        1               5                   10                  15

        Glu Arg Val Ser Leu Thr Cys Arg Ala Ser Gln Glu Ile Ser Gly Tyr
                    20                  25                  30

        Leu Ser Trp Leu Gln Gln Lys Pro Asp Gly Thr Ile Lys Arg Leu Ile
                    35                  40                  45

        Tyr Ala Ala Ser Thr Leu Asp Ser Gly Val Pro Lys Arg Phe Ser Gly
            50                  55                  60

        Ser Arg Ser Gly Ser Asp Tyr Ser Leu Thr Ile Ser Ser Leu Glu Ser
        65                  70                  75                  80

        Glu Asp Phe Ala Asp Tyr Tyr Cys Leu Gln Tyr Ala Ser Tyr Pro Phe
                        85                  90                  95

            Thr Phe Gly Ser Gly Thr Lys Leu Glu Ile Lys
                        100                 105
```

<210> 80
<211> 4
<212> PRT
<213> Artificial Sequence

<220>
<223> ITAM motif

<220>
<221> misc_feature
<222> (2)..(3)
<223> Xaa can be any naturally occurring amino acid

<220>
<221> MISC_FEATURE
<222> (4)..(4)
<223> Xaa may be Leu or Ile

<400> 80

```
            Tyr Xaa Xaa Xaa
            1
```

<210> 81
<211> 4
<212> PRT
<213> Artificial Sequence

<220>
<223> basic amino acid furin target sequence

<220>
<221> misc_feature

<222> (2)..(2)
<223> Xaa can be any naturally occurring amino acid

<220>
<221> MISC_FEATURE
<222> (3)..(3)
<223> Xaa may be Arg or Lys

<400> 81

```
                              Arg Xaa Xaa Arg
                              1
```

<210> 82
<211> 7
<212> PRT
<213> Artificial Sequence

<220>
<223> consensus Tobacco Etch Virus (TEV) cleavage site

<400> 82

```
Glu Asn Leu Tyr Phe Gln Ser
1               5
```